# FASCICULE DE BREVET EUROPEEN

(11) **EP 3 024 833 B1**
(45) Date de publication et mention de la délivrance du brevet: **06.09.2017**
(21) Numéro de dépôt: 14758605.1
(22) Date de dépôt: 22.07.2014
(51) Int. Cl.: C07D 471/04, C07D 487/04, C07D 519/00, A61K 31/4709, A61K 31/4985, A61P 35/00, A61P 37/00

(54) **NOUVEAUX DÉRIVÉS D'INDOLIZINE, LEUR PROCÉDÉ DE PRÉPARATION ET LES COMPOSITIONS PHARMACEUTIQUES QUI LES CONTIENNENT**
NEUARTIGE INDOLIZINDERIVATE, VERFAHREN ZUR HERSTELLUNG DAVON UND PHARMAZEUTISCHE ZUSAMMENSETZUNGEN DAMIT
NOVEL INDOLIZINE DERIVATIVES, METHOD FOR THE PRODUCTION THEREOF AND PHARMACEUTICAL COMPOSITIONS CONTAINING SAME

(30) Priorité: 23.07.2013 FR 1357265
(43) Date de publication de la demande: 01.06.2016
(73) Titulaire: Les Laboratoires Servier, 92284 Suresnes (FR); Vernalis (R&D) Ltd., Berkshire RG41 5RD (GB)
(72) Inventeur: LE TIRAN, Arnaud, F-78290 Croissy sur Seine (FR); LE DIGUARHER, Thierry, F-45550 Saint Denis de l'Hôtel (FR); STARCK, Jérôme-Benoît, F-92500 Rueil-Malmaison (FR); HENLIN, Jean-Michel, F-92150 Suresnes (FR); DE NANTEUIL, Guillaume, F-92150 Suresnes (FR); GENESTE, Olivier, F-92500 Rueil Malmaison (FR); DAVIDSON, James Edward Paul, Cambridge Cambridgeshire CB22 5DJ (GB); MURRAY, James Brooke, Linton Cambridgeshire CB21 4YL (GB); CHEN, I-Jen, Cambridge, CB1 3JJ (GB)
(86) Numéro de dépôt international: PCT/FR2014/051885
(87) Numéro de publication internationale: WO 2015/011397

(56) Documents cités:
- WO-A1-2012/162365
- WO-A1-2013/110890
- WO-A2-2006/023778

## Description

La présente invention concerne de nouveaux dérivés d'indolizine, leur procédé de préparation et les compositions pharmaceutiques qui les contiennent.

Les composés de la présente invention sont nouveaux et présentent des caractéristiques pharmacologiques très intéressantes dans le domaine de l'apoptose et de la cancérologie.

L'apoptose, ou mort cellulaire programmée, est un processus physiologique crucial pour le développement embryonnaire et le maintien de l'homéostasie tissulaire.
La mort cellulaire de type apoptotique fait intervenir des changements morphologiques, tels que la condensation du noyau, la fragmentation de l'ADN, ainsi que des phénomènes biochimiques, tels que l'activation des caspases qui vont dégrader des composants structuraux clés de la cellule pour induire son désassemblage et sa mort. La régulation du processus d'apoptose est complexe et implique l'activation ou la répression de plusieurs voies de signalisation intracellulaire (Cory S. et al., Nature Review Cancer, 2002, 2, 647-656).
Des dérégulations de l'apoptose sont impliquées dans certaines pathologies. Une apoptose accrue est liée aux maladies neurodégénératives telles que la maladie de Parkinson, la maladie d'Alzheimer et l'ischémie. Inversement, des déficiences dans l'exécution de l'apoptose jouent un rôle important dans le développement des cancers et leur chimiorésistance, des maladies auto-immunes, des maladies inflammatoires et des infections virales. Ainsi, l'échappement à l'apoptose fait partie des signatures phénotypiques du cancer (Hanahan D. et al., Cell 2000, 100, 57-70).

Les protéines anti-apoptotiques de la famille Bcl-2 sont associées à de nombreuses pathologies. L'implication des protéines de la famille Bcl-2 est décrite dans de nombreux types de cancer, tel que le cancer colorectal, le cancer du sein, le cancer du poumon à petites cellules, le cancer du poumon non à petites cellules, le cancer de la vessie, le cancer de l'ovaire, le cancer de la prostate, la leucémie lymphoïde chronique, le lymphome un besoin thérapeutique de composés inhibant l'activité anti-apoptotique des protéines de la famille Bcl-2. Parmi les inhibiteurs de Bcl-2 déjà connus dans la littérature, on distingue les composés 2-(1,2,3,4-tétrahydroisoquinolin-2-carbonyl)-4-(sulfonylcarbamoyl)phenyl décrits dans WO2012/162365, les dérivés 1-(1,2,3,4-tétrahydroisoquinolin-2-carbonyl)-2,3,4-trihydroxyphényl décrits dans WO2006/023778, ainsi que les composés 3-[2-(1,2,3,4-tétrahydroisoquinolin-2-carbonyl)phenyl]indolizine décrits dans WO2013/110890. Tous présentent un intérêt potentiel dans le traitement du cancer.

Les composés de la présente invention outre leur nouveauté, présentent des propriétés pro-apoptotiques permettant de les utiliser dans les pathologies impliquant un défaut d'apoptose, comme par exemple dans le traitement du cancer, des maladies auto-immunes et du système immunitaire.

La présente invention concerne plus particulièrement les composés de formule (I) : 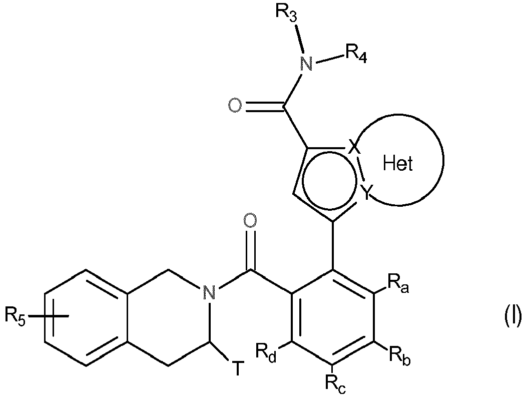 dans laquelle :
◆ X et Y représentent un atome de carbone ou un atome d'azote, étant entendu qu'ils ne peuvent représenter simultanément deux atomes de carbone ou deux atomes d'azote,
◆ la partie Het du groupe 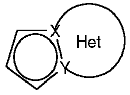 représente un cycle éventuellement substitué, aromatique ou non, constitué de 5, 6 ou 7 chaînons, et pouvant contenir, en plus de l'azote représenté par X ou par Y, un à 3 hétéroatomes choisis indépendamment parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié,
◆ T représente un atome d'hydrogène, un alkyle (C₁-C₆) linéaire ou ramifié éventuellement substitué par un à trois atomes d'halogène, un groupement alkyl(C₂-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
   ou bien R₁ et R₂ forment avec l'atome d'azote qui les porte un hétérocycloalkyle,
◆ R₃ représente un groupement alkyle (C₁-C₆) linéaire, aryle ou hétéroaryle, ces deux derniers groupements pouvant être substitués par un à trois groupements choisis parmi halogène, alkyle (C₁-C₆) linéaire ou ramifié, alkoxy (C₁-C₆) linéaire ou ramifié, cyano et hétérocycloalkyl-alkyle (C₁-C₆) linéaire ou ramifié, étant entendu qu'un ou plusieurs atomes de carbone des groupements précédents, ou de leurs éventuels substituants, peut(vent) être deutéré(s),
◆ R₄ représente un groupement 4-hydroxyphényle, étant entendu qu'un ou plusieurs atomes de carbone du groupement, ou de ses éventuels substituants, précédent peut(vent) être deutéré(s),
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ et R_{d} représentent chacun un atome d'hydrogène et (R_{b},R_{c}) forment ensemble avec les atomes de carbone qui les portent un groupe 1,3-dioxolane ou un groupe 1,4-dioxane, ou bien Rₐ, R_{c} et R_{d} représentent chacun un atome d'hydrogène et R_{b} représente un hydrogène, un halogène, un méthyle ou un méthoxy, ou encore Rₐ, R_{b} et R_{d} représentent chacun un atome d'hydrogène et R_{c} représente un groupement hydroxy ou méthoxy,
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié, spiro (C₃-C₆), alkoxy (C₁-C₆) linéaire ou ramifié, (C₁-C₆)alkyl-S-, hydroxy, oxo (ou N-oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle, hétéroaryle, aryloxy, arylthio, cycloalkyle, hétérocycloalkyle éventuellement substitué par un ou plusieurs atomes d'halogène ou groupements alkyles, étant entendu que R' et R" représentent, indépendamment l'un de l'autre, un atome d'hydrogène ou un groupement alkyle (C₁-C₆) linéaire ou ramifié,
la partie Het du groupe 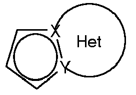 défini dans la formule (I) pouvant être substituée par un à trois groupements choisis parmi alkyle (C₁-C₆) linéaire ou ramifié, hydroxy, alkoxy (C₁-C₆) linéaire ou ramifié, NR₁'R₁", ou halogène, étant entendu que R₁' et R₁" ont les mêmes définitions que les groupes R' et R" mentionnés précédemment,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

De manière avantageuse, le groupe : 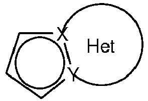 représente l'un des groupements suivants : 5,6,7,8-tétrahydroindolizine éventuellement substitué par un groupe amino ; indolizine ; 1,2,3,4-tétrahydropyrrolo[1,2-*a*]pyrazine éventuellement substitué par un méthyle ; pyrrolo[1,2-*a*]pyrimidine. Les groupements 5,6,7,8-tétrahydroindolizine et indolizine sont plus particulièrement préférés.

Dans les composés préférés de l'invention, T représente un atome d'hydrogène, un groupement méthyle (et plus particulièrement un *(R)*-méthyle), un groupement 2-(morpholin-4-yl)éthyle, 3-(morpholin-4-yl)propyle, -CH₂-OH, 2-aminoéthyle, 2-(3,3-difluoropipéridin-1-yl)éthyle, 2-[(2,2-difluoroéthyl)amino]éthyle ou 2-(3-méthoxyazétidin-1-yl)éthyle.

De manière préférentielle, Rₐ et R_{d} représentent chacun un atome d'hydrogène et (R_{b},R_{c}) forment ensemble avec les atomes de carbone qui les portent un groupe 1,3-dioxolane, ou bien Rₐ, R_{c} et R_{d} représentent chacun un atome d'hydrogène et R_{b} représente un halogène.

Dans les composés préférés de l'invention, R₃ représente un groupement hétéroaryle choisi parmi le groupe suivant : 1*H*-indole, 2,3-dihydro-1*H*-indole, 1*H*-indazole, pyridine, 1*H-*pyrrolo[2,3-*b*]pyridine, 1*H*-pyrazole, imidazo[1,2-*a*]pyridine, pyrazolo[1,5-*a*]pyrimidine, [1,2,4]triazolo[1,5-*a*]pyrimidine, et 1*H*-pyrazolo[3,4-*b*]pyridine, tous pouvant être substitués par un groupement alkyle (C₁-C₆) linéaire ou ramifié.

Les composés préférés selon l'invention sont compris dans le groupe suivant :
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-{1-[2-(morpholin-4-yl)éthyl]-1*H*-indol-5-yl}-5,6,7,8-tétrahydroindolizine-1-carboxamide,
- *N*-(4-hydroxyphényl)-3-(6-{[(3*S*)-3-[2-(morpholin-4-yl)éthyl]-3,4-dihydroisoquinolin-2(1*H*)-yl]carbonyl}-1,3-benzodioxol-5-yl)-*N*-phényl-5,6,7,8-tétrahydroindolizine-1-carboxamide,
- *N*-{3-fluoro-4-[2-(morpholin-4-yl)éthoxy]phényl}-*N*-(4-hydroxyphényl)-3-(6-{[(3*R*)-3-méthyl-3,4-dihydroisoquinolin-2(1*H*)-yl]carbonyl}-1,3-benzodioxol-5-yl)indolizine-1-carboxamide,
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-(pyridin-4-yl)indolizine-1-carboxamide,
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-(2-méthylpyridin-4-yl)indolizine-1-carboxamide,
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-(1-méthyl-1*H*-pyrrolo[2,3-*b*]pyridin-5-yl)indolizine-1-carboxamide,
- *N*-(4-hydroxyphényl)-3-(6-{[(3*R*)-3-[3-(morpholin-4-yl)propyl]-3,4-dihydroisoquinolin-2(1*H*)-yl]carbonyl}-1,3-benzodioxol-5-yl)-*N*-phényl-5,6,7,8-tétrahydroindolizine-1-carboxamide,
- *N*-(2,6-diméthylpyridin-4-yl)-*N*-(4-hydroxyphényl)-3-(6-{[(3*R*)-3-méthyl-3,4-dihydroisoquinolin-2(1*H*)-yl]carbonyl}-1,3-benzodioxol-5-yl)indolizine-1-carboxamide,
- *N-*(4-hydroxyphényl)-3-(6-{[(3R)-3-méthyl-3,4-dihydroisoquinolin-2(1*H*)-yl]carbonyl}-1,3-benzodioxol-5-yl)-*N-*(pyridin-4-yl)-5,6,7,8-tétrahydroindolizine-1-carboxamide,
- 3-(5-Chloro-2-{[(3*R*)-3-méthyl-3,4-dihydroisoquinolin-2(1*H*)-yl]carbonyl}phényl)-*N*-(4-hydroxyphényl)-*N*-(1-méthyl-1*H*-pyrrolo[2,3-*b*]pyridin-5-yl)indolizine-1-carboxamide,
- *N*-(4-Hydroxyphényl)-*N*-(2-méthoxypyridin-4-yl)-3-(6-{[(3*R*)-3-méthyl-3,4-dihydroisoquinolin-2(1*H*)-yl]carbonyl}-1,3-benzodioxol-5-yl)indolizine-1-carboxamide,
leurs énantiomères et diastéréoisomères, ainsi que leurs sels d'addition à un acide ou à une base pharmaceutiquement acceptable.

L'invention s'étend également au procédé de préparation des composés de formule (I) caractérisé en ce que l'on utilise comme produit de départ le composé de formule (II) : 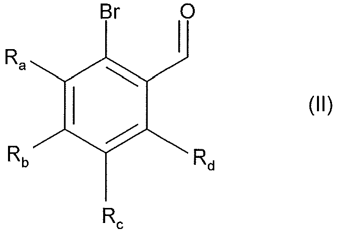 dans laquelle Rₐ, R_{b}, R_{c} et R_{d} sont tels que définis dans la formule (I), composé de formule (II) qui est soumis à une réaction de Heck, en milieu aqueux ou organique, en présence d'un catalyseur au palladium, d'une base, d'une phosphine et du composé de formule (III) : 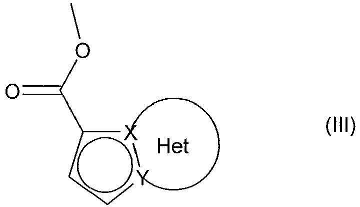 dans laquelle les groupements X, Y et Het sont tels que définis dans la formule (I),
pour obtenir le composé de formule (IV) : 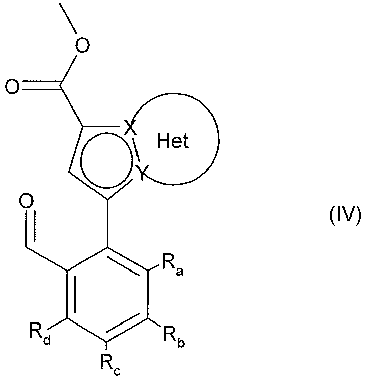 dans laquelle Rₐ, R_{b}, R_{c}, R_{d}, X, Y et Het sont tels que définis dans la formule (I),
composé de formule (IV) dont la fonction aldéhyde est oxydée en acide carboxylique pour former le composé de formule (V) : 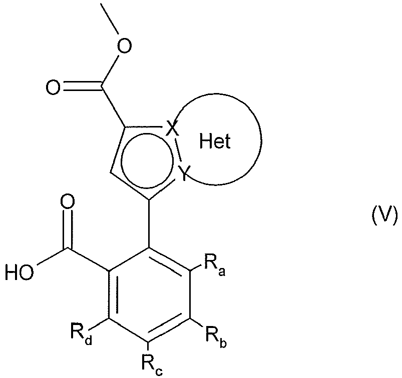 dans laquelle Rₐ, R_{b}, R_{c}, R_{d}, X, Y et Het sont tels que définis dans la formule (I),
composé de formule (V) qui subit ensuite un couplage peptidique avec un composé de formule (VI) : 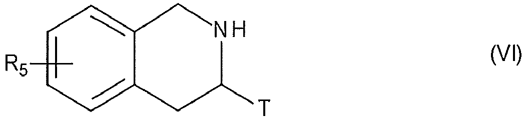 dans laquelle T et R₅ sont tels que définis dans la formule (I),
pour conduire au composé de formule (VII) : 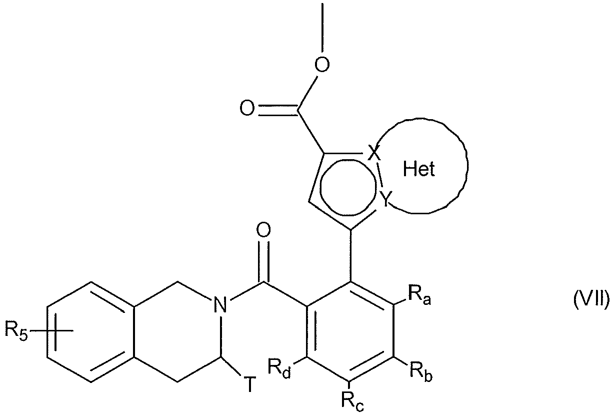 dans laquelle Rₐ, R_{b}, R_{c}, R_{d}, T, R₅, X, Y et Het sont tels que définis dans la formule (I), composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

Plus particulièrement, lorsque l'un des groupements R₃ ou R₄ de l'amine NHR₃R₄ est substitué par une fonction hydroxy, cette dernière peut être préalablement soumise à une réaction de protection avant tout couplage avec l'acide carboxylique du composé de formule (VII), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

Les composés de formules (II), (III), (VI), ainsi que l'amine NHR₃R₄, sont soit commerciaux, soit accessibles à l'homme du métier par des réactions chimiques classiques et décrites dans la littérature.

L'étude pharmacologique des dérivés de l'invention a montré qu'ils possédaient des propriétés pro-apoptotiques. La capacité à réactiver le processus apoptotique dans les cellules cancéreuses représente un intérêt thérapeutique majeur dans le traitement des cancers, des maladies auto-immunes et du système immunitaire.

Plus particulièrement, les composés selon l'invention seront utiles dans le traitement des cancers chimio ou radiorésistants, ainsi que dans les hémopathies malignes et le cancer du poumon à petites cellules.
Parmi les traitements des cancers envisagés on peut citer, sans s'y limiter, le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules. Parmi les lymphomes non hodgkiniens, on peut citer plus préférentiellement les lymphomes folliculaires, les lymphomes des cellules du manteau, les lymphomes diffus à grandes cellules B, les petits lymphomes lymphocytiques et les lymphomes à cellules B de la zone marginale.

La présente invention a également pour objet les compositions pharmaceutiques contenant au moins un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Par ailleurs, la présente invention concerne également l'association d'un composé de formule (I) avec un agent anticancéreux choisi parmi les agents génotoxiques, les poisons mitotiques, les anti-métabolites, les inhibiteurs du protéasome, les inhibiteurs de kinase, ou les anticorps, ainsi que les compositions pharmaceutiques contenant ce type d'association et leur utilisation pour la fabrication de médicaments utiles dans le traitement du cancer.

Les composés de l'invention peuvent également être utilisés en association avec une radiothérapie dans le traitement du cancer.

Enfin, les composés de l'invention peuvent être liés à des anticorps monoclonaux ou à des fragments de ceux-ci ou peuvent être liés à des protéines de charpente qui peuvent être apparentées, ou non, à des anticorps monoclonaux.
Par fragments d'anticorps, on doit entendre des fragments du type Fv, scFv, Fab, F(ab')2, F(ab'), scFv-Fc, ou des diabodies, qui en général ont la même spécificité de liaison que l'anticorps duquel ils sont issus. Selon la présente invention, les fragments d'anticorps de l'invention peuvent être obtenus à partir d'anticorps par des méthodes telles que digestion par enzymes telles que la pepsine ou la papaïne, et/ou par clivage des ponts disulfures par réduction chimique. D'autre manière, les fragments d'anticorps compris dans la présente invention peuvent être obtenus par des techniques de recombinaison génétique également bien connues à l'homme du métier ou bien par synthèse de peptides au moyen de synthétiseurs automatiques de peptides par exemple, tels que ceux fournis par la société Applied Biosystems etc...
Par protéines de charpente qui peuvent être apparentées, ou non, à des anticorps monoclonaux, on entend une protéine qui comprend, ou non, un repliement d'immunoglobuline et qui livre une capacité de liaison similaire à celle d'un anticorps monoclonal. L'homme du métier sait comment sélectionner la protéine de charpente. Plus particulièrement, il est connu que, pour être sélectionné, une telle charpente devrait présenter plusieurs caractéristiques comme suivant (Skerra A., J. Mol. Recogn. 13, 2000, 167-187): une bonne conservation phylogénétique, une architecture robuste avec une organisation moléculaire en trois dimensions bien connue (telle que la cristallographie ou la RMN, par exemple), une petite taille, aucune ou peu de modification(s) post-traductionnelle(s), la facilité de production, d'expression et de purification. Une telle protéine de charpente peut être, sans s'y limiter, une structure choisie parmi le groupe consistant en la fibronectine et de préférence le dixième domaine type III de la fibronectine (FNfn10), la lipocaline, l'anticaline (Skerra A., J. Biotechnol., 2001, 74(4):257-75), la protéine Z dérivée du domaine B de la protéine A des staphylocoques, la thiorédoxine A ou une protéine quelconque avec un domaine répété tel qu'une "répétition ankyrine" (Kohl et al., PNAS, 2003, vol.100, no.4, 1700-1705), une "répétition armadillo", une "répétition riche en leucine" ou une "répétition tétratricopeptide". On pourrait aussi mentionner une charpente dérivée de toxines (telles que des toxines de scorpions, d'insectes, de plantes ou des mollusques, par exemple) ou des protéines inhibitrices de l'oxyde nitrique synthase (PIN).

Les Préparations et Exemples suivants illustrent l'invention et ne la limitent en aucune façon.

### Procédures générales

Tous les réactifs et les solvants anhydres sont commerciaux et ont été utilisés sans purification ou séchage supplémentaire. La chromatographie éclair est réalisée sur un appareil ISCO CombiFlash Rf 200i avec des cartouches prépackées remplies de gel de silice (SiliaSep™ F60 (40-63µm), 60Å). Les chromatographies sur couche mince ont été réalisées sur des plaques de 5 x 10 cm recouvertes de gel de silice Merck Type 60 F₂₅₄. Le chauffage par microonde a été réalisé avec un appareil CEM Discover® SP.

### LC-MS analytique

Les composés de l'invention ont été caractérisés par chromatographie liquide à haute performance couplée à la spectrométrie de masse (HPLC-MS) soit sur un appareil Agilent HP 1200 à résolution rapide couplé à un détecteur de masse 6140 à source multimodale (intervalle m/z de 150 à 1000 unités de masse atomique ou uma), soit sur un appareil Agilent HP1100 couplé à un détecteur de masse 1946D à source d'ionisation par électrospray (intervalle m/z de 150 à 1000 uma). Les conditions et les méthodes listées ci-dessous sont identiques pour les deux machines.
Détection : Détection UV à 230, 254 et 270 nm.
Volume d'injection : 2µL
Phases Mobiles :
A - Eau + 10 mMol / formate d'ammonium + 0,08% (v/v) acide formique à pH environ 3,5.
B - 95% Acétonitrile + 5% A + 0,08% (v/v) acide formique

### Méthode A (3,75 min; soit ionisation positive (pos) soit ionisation positive et négative (pos / neg))

Colonne : Gemini 5µm, C18, 30 mm x 4,6mm (Phenomenex).
Température : 35° C.

**Gradient :**

| **Temps (min)** | **Solvent A (%)** | **Solvent B (%)** | **Débit (mL/min)** |
|---|---|---|---|
| 0 | 95 | 5 | 2 |
| 0,25 | 95 | 5 | 2 |
| 2,50 | 95 | 5 | 2 |
| 2,55 | 5 | 95 | 3 |
| 3,60 | 5 | 95 | 3 |
| 3,65 | 5 | 95 | 2 |
| 3,70 | 95 | 5 | 2 |
| 3,75 | 95 | 5 | 2 |

### Méthode B (1,9 min; soit ionisation positive (pos) soit ionisation positive et négative (pos / neg))

Colonne : Gemini 5µm, C18, 30 mm x 4,6mm (Phenomenex).
Température : 35° C.

**Gradient:**

| **Temps (min)** | **Solvent A (%)** | **Solvent B (%)** | **Débit (mL/min)** |
|---|---|---|---|
| 0 | 95 | 5 | 1,1 |
| 0,12 | 95 | 5 | 1,1 |
| 1,30 | 5 | 95 | 1,1 |
| 1,35 | 5 | 95 | 1,7 |
| 1,85 | 5 | 95 | 1,7 |
| 1,90 | 5 | 95 | 1,1 |
| 1,95 | 95 | 5 | 1,1 |

### Préparation 1 : Acide 6-[1-(méthoxycarbonyl)-5,6,7,8-tétrahydro-3-indolizinyl]-1,3-benzodioxole-5-carboxylique

### Stade A : Acide 1-formyl-2-pipéridine carboxylique

A une solution de 40 g d'un mélange racémique d'acide 2-pipéridine carboxylique (0,310 mmol) dans 300 mL d'acide formique placée à 0°C, on ajoute goutte à goutte 200 mL (2,15 mmol) d'anhydride acétique. L'ensemble est ensuite agité à température ambiante durant une nuit. Puis, le milieu réactionnel est refroidi à 0°C, hydrolysé par l'addition de 250 mL d'eau, et agité pendant une demi-heure à 0°C avant d'être concentré à sec. L'huile ainsi obtenue est reprise dans 200 mL de méthanol puis concentrée à sec. Le produit du titre est obtenu sous la forme d'une huile avec un rendement de 98%. Il est utilisé directement, sans autre purification, pour l'étape suivante.
**RMN ¹H :** δ (400 MHz; dmso-d6 ; 300K) : 13.0 (m, 1H OH); 8.0-8.05 (2s, 1H aldéhyde) ; 4.9-4.5 (2d, 1H α de N et COOH) ; 4.1-2.6 (m, 2H en α du N); 2.2-1.2 (m, 6H pipéridine)
**IR :** ν : -OH : 2000-3000 cm⁻¹ acide ; ν : >C=O 1703 cm⁻¹ bande large

### Stade B : 5,6,7,8-Tétrahydro-1-indolizine carboxylate de méthyle

A une solution de 10 g d'acide carboxylique obtenu au Stade A (63,6 mmol) dans 65 mL de dichlorométhane sont ajoutés successivement 13,4 g de chlorure de tosyle (70,4 mmol), 11,5 mL de 2-chloroacrylate de méthyle (113,5 mmol), puis, au goutte à goutte, 17,8 mL de *N,N,N*-triéthylamine (127,2 mmol). Le milieu réactionnel est ensuite porté au reflux pendant 1h30. On se place ensuite à température ambiante, puis on ajoute 5 mL de 2-chloroacrylate de méthyle (48,9 mmol) et, au goutte à goutte, 9 mL de *N,N,N*-triéthylamine (64 mmol). L'ensemble est chauffé au reflux pendant une nuit.
Le milieu réactionnel est ensuite dilué avec du chlorure de méthylène, lavé successivement avec une solution HCl 1N, une solution saturée de NaHCO₃, puis une solution saturée de NaCl jusqu'à obtenir un pH neutre. La phase organique est alors séchée sur MgSO₄, filtrée, concentrée à sec, et purifiée par chromatographie sur gel de silice (gradient heptane/ AcOEt). On obtient le produit du titre sous la forme d'une huile.
**RMN ¹H :** δ (400 MHz ; CDCl₃ ; 300K) : 6.55-6.40 (d, 2H, tétrahydroindolizine) ; 3.91 (t, 3H méthyl ester); 3.78 (s, 3H tétrahydroindolizine); 3.08 (t, 2H, tétrahydroindolizine); 1.95-1.85 (m, 4H, tétrahydroindolizine)
**IR :** ν :>C=O 1692 cm⁻¹ ester

### Stade C : 3-(6-Formyl-1,3-benzodioxol-5-yl)-5,6,7,8-tétrahydro-1-indolizine carboxylate de méthyle

A une solution de 6,4 g de l'ester obtenu au Stade B (35,7 mmol) dans 12 mL de *N*,*N-*diméthylacétamide, on ajoute successivement 12,3g de 6-bromo-1,3-benzodioxole-5-carbaldéhyde (53,6 mmol), 7 g d'acétate de potassium (71,4 mmol), puis l'ensemble est agité sous argon pendant 20 minutes. On ajoute alors 1,3 g de catalyseur au palladium PdCl₂(PPh₃)₂ (1,8 mmol). Le milieu réactionnel est ensuite chauffé à 130°C pendant une heure avant d'y ajouter 139 µL d'H₂O. Le chauffage est maintenu à cette même température pendant la nuit. On laisse le milieu revenir à température ambiante, puis on le dilue avec de l'AcOEt. On ajoute du noir animal (2 g par g de produit) et l'ensemble est agité à température ambiante pendant 1 heure, puis filtré. La phase organique est alors lavée avec de l'eau, séchée sur sulfate de magnésium et concentrée à sec. Le produit brut ainsi obtenu est purifié sur gel de silice (gradient heptane/ ACOEt). Le produit du titre est obtenu sous la forme d'une huile.
**RMN¹H :** δ :(400 MHz ; dmso-d6 ; 353°K) : 9.65 (s, 1H, H aldéhyde) ; 7.3-7.15 (2s, 2H, H aromatiques) ; 6.45 (s, 1H tétrahydroindolizine) ; 6.20 (s, 2H méthylènedioxy); 3.70 (s, 3H méthyl ester) ; 3.5-4.0 (m, 2H tétrahydroindolizine) ; 3.05 (m, 2H tétrahydroindolizine) ; 1.85 (m, 4H tétrahydroindolizine)
**IR :** ν : >C=O 1695 cm⁻¹ ester; ν : >C=O 1674 cm⁻¹

### Stade D: Acide 6-[1-(méthoxycarbonyl)-5,6,7,8-tétrahydro-3-indolizinyl]-1,3-benzodioxole-5-carboxylique

On prépare une solution contenant 3,37 g du composé obtenu au Stade C (10,3 mmol) dans 9,3 mL d'acétone et 8,8 mL (80,24 mmol) de 2-méthyl-2-butène, laquelle est placée à 0°C. On ajoute, goutte à goutte, 9,3 mL d'une solution aqueuse contenant un mélange de 3,3 g de NaClO₂ (36,05 mmol) et de 3,6 g de Na₂PO₄ (25,75 mmol). L'ensemble est ensuite agité à température ambiante durant 7 heures. Puis, le milieu réactionnel est concentré pour éliminer l'acétone. Le solide alors obtenu est filtré, lavé à l'eau puis séché sous vide à 40°C pendant une nuit. Le produit du titre est obtenu sous la forme d'un solide, qui est utilisé pour la suite sans autre purification.
**RMN¹H :** δ (400 MHz ; dmso-d6 ; 300K) : 12.10 (m, 1H, H acide carboxylique) ; 7.40-6.88 (2s, 2H, H aromatiques); 6.20 (s, 1H, H tétrahydroindolizine); 6.18 (s, 2H, H méthylènedioxy); 3.70 (s, 3H, méthyl ester) ; 3.55 (t, 2H tétrahydroindolizine) ; 3.00 (t, 2H tétrahydroindolizine) ; 1.80 (m, 4H, H tétrahydroindolizine)
**IR :** ν : -OH : 3000-2000 cm⁻¹ acide ; ν : >C=O 1686-1676 cm⁻¹ ester+acide ; ν : >C=C< 1608 cm⁻¹

### Préparation 2 : Acide 2-[2-(tert-butoxycarbonyl)-8-(méthoxycarbonyl)-1,2,3,4-tétrahydropyrrolo[1,2-a]pyrazin-6-yl]-4-chlorobenzoïque

### Stade A : 4-Formyl-1,3-pipérazinedicarboxylate de 1-tert-butyle et de 3-méthyle

A une solution de pentafluorophénol dans 520 mL d'éther anhydre placée à 0°C, on ajoute successivement 49 g d'1-éthyl-3-(3'-diméthylaminopropyl)-carbodiimide (286 mmol) par portions et 12 mL d'acide formique (312 mmol). L'ensemble est agité à température ambiante pendant 2h. On ajoute ensuite un mélange de 32 g de 1,3-pipérazinedicarboxylate de 1-*tert*-butyle et de 3-méthyle (130 mmol) et de 18 mL de triéthylamine (130 mmol) en solution dans 520 mL de CH₂Cl₂. L'ensemble est agité pendant une nuit à température ambiante. Le milieu réactionnel est hydrolysé avec une solution aqueuse de HCl, 1N et extrait avec du CH₂Cl₂. Les phases organiques sont alors regroupées, puis lavées avec une solution aqueuse de NaHCO₃ saturée, puis avec une solution aqueuse de NaCl saturée jusqu'à neutralité. Après séchage sur MgSO₄, filtration et concentration à sec, on isole le produit par chromatographie sur gel de silice (gradient éther de pétrole / AcOEt : 0-30%). On obtient le produit du titre sous la forme d'une huile.
**IR :** ν : C=O : 1674-1745 cm⁻¹
**m/z** (C₁₂H₂₀N₂O₅): 272.1(M+); 295.121 (M+Na)⁺; 567.253 (2M+Na)⁺

### Stade B : 4-(tert-Butoxycarbonyl)-1-formyl-2-pipérazinecarboxylate de lithium

A une solution de 28 g du composé obtenu au Stade A (103 mmol) dans 515 mL de dioxane, on ajoute 4,8 g de LiOH (113 mmol) en solution dans 100 mL d'H₂O. L'ensemble est agité à température ambiante durant 4 h. Le milieu réactionnel est ensuite concentré à sec, puis co-évaporé plusieurs fois avec de l'acétate d'éthyle. Le produit du titre est obtenu sous la forme d'un solide et est utilisé directement pour l'étape suivante de cyclisation.
**RMN ¹³C:** δ (500 MHz; dmso-d6; 300K) : 46 (s, C pipérazine) ; 42-38 (m, C pipérazine) ; 58-53 (s, C pipérazine) ; 28.5 (s, C ^{t}Bu)
**IR :** ν : C=O : 1650 cm⁻¹ ; 2800 cm⁻¹

### Stade C : 3,4-Dihydropyrrolo[1,2-a]pyrazine-2,8(1H)-dicarboxylate de 2-tert-butyle et de 8-méthyle

A une suspension de 29 g du composé obtenu au Stade B (103 mmol) dans 800 mL de dichlorométhane, on ajoute successivement 24 g de chlorure de tosyle (124 mmol), 12.6 mL de 2-chloroacrylate de méthyle (124 mmol), puis 35 mL de triéthylamine (247 mmol). L'ensemble est agité au reflux durant 2h. Après refroidissement, le mileu réactionnel est dilué avec de l'acétate d'éthyle et la phase organique est lavée avec une solution de NaCl saturée jusqu'à neutralité. Après séchage sur MgSO₄, filtration et concentration à sec, le produit du titre est isolé par chromatographie sur gel de silice (gradient éther de pétrole / AcOEt : 0-20%) sous la forme d'un solide.
**RMN ¹H:** δ (400 MHz ; dmso-d6 ; 300K) : 6.8-6.43 (m, 2H, H pyrrole); 4.75-3.75 (m, 6H, H pipérazine)) ; 3.73 (s, 3H, H COOCH3) ; 1.48 (s, 9H, H ^{t}Bu)
**IR :** ν : C=O (ester conjugué) : 1712 cm⁻¹ ; C=O (carbamate) : 1677 cm⁻¹

### Stade D: Acide 2-[2-(tert-butoxycarbotzyl)-8-(méthoxycarbonyl)-1,2,3,4-tétrahydropyrrolo[1,2-a]pyrazin-6-yl]-4-chlorobenzoïque

On procède selon le protocole décrit aux Stades C et D de la Préparation 1 en remplaçant le 6-bromo-1,3-benzodioxole-5-carbaldéhyde utilisé au Stade C par le 2-bromo-4-chlorobenzaldéhyde.

### Préparation 3: Acide 4-chloro-2-[1-(méthoxycarbonyl)-5,6,7,8-tétrahydro-3-indolizinyl]benzoïque

On procède selon le procédé de la Préparation 1 en remplaçant le 6-bromo-1,3-benzodioxole-5-carbaldéhyde utilisé au Stade C par le 2-bromo-4-chlorobenzaldéhyde.

### Préparation 4 : Acide 7-[2-(tert-butoxycarbonyl)-8-(méthoxycarbonyl)-1,2,3,4-tétrahydropyrrolo[1,2-a]pyrazin-6-yl]-2,3-dihydro-1,4-benzodioxine-6-carboxylique

### Stade A : 3,4-Dihydropyrrolo[1,2-a]pyrazine-2,8(1H)-dicarboxylate de 2-tert-butyle et de 8-méthyle

On procède selon le procédé décrit aux Stades A-C de la Préparation 2.
**RMN ¹H:** δ (400 MHz ; dmso-d6 ; 300K) : 6.8-6.43 (m, 2H, H pyrrole); 4.75-3.75 (m, 6H, H pipérazine)) ; 3.73 (s, 3H, H COOCH3) ; 1.48 (s, 9H, H ^{t}Bu)
**IR :** ν : C=O (ester conjugué) : 1712 cm⁻¹ ; C=O (carbamate) : 1677 cm⁻¹

### Stade B: Acide 7-[2-(tert-butoxycarbonyl)-8-(méthoxycarbonyl)-1,2,3,4-tétrahydropyrrolo[1,2-a]pyrazin-6-yl]-2,3-dihydro-1,4-benzodioxine-6-carboxylique

On procède selon le protocole décrit aux Stades C et D de la Préparation 1 en remplaçant le 6-bromo-1,3-benzodioxole-5-carbaldéhyde utilisé au Stade C par le 7-bromo-2,3-dihydro-1,4-benzodioxine-6-carbaldéhyde.

### Préparation 5: Acide 4-chloro-2-[1-(méthoxycarbonyl)-3-indolizinyl]benzoïque

### Stade A: Bromure de 1-(carboxyméthyl)-1,2-dihydropyridinium

A une solution de 16,2 mL de pyridine (200 mmol) dans 120 mL d'acétate d'éthyle, on ajoute par portions 27,8 g (200 mmol) d'acide bromoacétique. L'ensemble est ensuite agité à température ambiante pendant une nuit. Le précipité ainsi obtenu est filtré, puis lavé avec de l'actétate d'éthyle froid. Après séchage, on obtient le produit du titre sous la forme d'une poudre qui est utilisée directement pour l'étape suivante.
**RMN ¹H:** δ (400 MHz ; dmso-d6 ; 300K) : 9.15 (d, 2H, H aromatiques pyridine)) ; 8.7 (t, 1H, H aromatique) ; 8.25 (t, 2H, H aromatique); 5.65 (s, 2H, H CH₂COOH)
**IR :** ν : C=O : 1732 cm⁻¹ ; -OH acide : 2800 cm⁻¹

### Stade B: 1-Indolizinecarboxylate de méthyle

A une suspension de 6,55 g du sel de pyridinium obtenu au Stade A (30 mmol) dans 240 mL de toluène, on ajoute successivement 16,7 mL d'acrylate de méthyle (150 mmol), 4,2 mL de triéthylamine (30 mmol), puis par portions 20,9 g de MnO₂ (240 mmol). L'ensemble est ensuite chauffé à 90°C durant 3 h. Après refroisissement, le milieu réactionnel est filtré sur un gâteau de célite et concentré à sec. Le produit du titre est alors isolé par purification sur gel de silice (gradient heptane / AcOEt : 0-10%) sous la forme d'une huile qui cristallise à froid.
**RMN ¹H:** δ (300 MHz ; dmso-d6 ; 300K) : 8.5 (d, 1H, H indolizine) ; 8.05 (d, 1H, H indolizine); 7.6 (s, 1H, H indolizine); 7.15 (m, 2H, H indolizine); 6.85 (m, 1H, H indolizine); 4.25 (q, 2H, -C(O)CH₂CH₃); 1.35 (t, 3H, -C(O)CH₂CH₃)
**IR :** ν : C=O ester : 1675 cm⁻¹ ; C=C aromatiques : 1634 cm⁻¹

### Stade C : Acide 4-chloro-2-[1-(méthoxycarbonyl)-3-indolizinyl]benzoïque

On procède selon le protocole décrit aux Stades C et D de la Préparation 1 en remplaçant le 6-bromo-1,3-benzodioxole-5-carbaldéhyde utilisé au Stade C par le 2-bromo-4-chlorobenzaldéhyde.

### Préparation 6: Acide 2-[2-(tert-butoxycarbonyl)-8-(méthoxycarbonyl)-1,2,3,4-tétrahydropyrrolo [1,2-a]pyrazin-6-yl]-4-fluorobenzoïque

On procède selon le protocole décrit à la Préparation 2 en remplaçant le 2-bromo-4-chlorobenzaldéhyde utilisé au Stade D par le 2-bromo-4-fluorobenzaldéhyde.

### Préparation 7: Acide 6-[2-(tert-butoxycarbonyl)-8-(méthoxycarbonyl)-1,2,3,4-tétrahydropyrrolo[1,2-a]pyrazin-6-yl]-1,3-benzodioxole-5-carboxylique

On procède selon le protocole décrit à la Préparation 2 en remplaçant le 2-bromo-4-chlorobenzaldéhyde utilisé au Stade D par le 6-bromo-1,3-benzodioxole-5-carbaldéhyde.

### Préparation 8: Acide 6-[1'-(méthoxycarbonyl)-5,6'-dihydro-8'H-spiro[1,3-dioxolane-2,7'-indolizin]-3'-yl]-1,3-benzodioxole-5-carboxylique

### Stade A: 8-Formyl-1,4-dioxa-8-azaspiro[4.5]décane-7-carboxylate de méthyl

24 g de 1,4-dioxa-8-azaspiro[4.5]décane-9-carboxylate de méthyle (111 mmol) sont solubilisés dans 80 mL d'acétate d'éthyle et 80 mL de dichlorométhane. On ajoute 26 g de (4-nitrophényl)formate (155 mmol) et l'ensemble est agité à température ambiante pendant 1 h. Le milieu réactionnel est évaporé à sec et repris dans l'acétate d'éthyle. La phase organique est ensuite lavée successivement avec une solution de NaOH 1 N, de l'eau, puis avec une solution de NH₄Cl saturée jusqu'à atteindre un pH neutre. Elle est ensuite séchée sur sulfate de magnésium, filtrée et concentrée à sec. L'huile ainsi obtenue est purifiée par chromatographie flash (gradient heptane/acétate d'éthyle). On obtient le produit du titre sous la forme d'une huile.
**RMN ¹H:** δ (400 MHz; dmso-d6; 300K): 8.15 (s, 1H, CHO); 5.0-4.75 (m, 1H, H tertiaire) ; 4.3-3.7 (m, 5H, 4H éthylènedioxy + 1H aliphatique pipéridine) ; 3.70 (s, 3H, Me) ; 3.4-2.9 (2m, 1H, H aliphatique pipéridine) ; 2.3-1.75 (m, 2H, H aliphatique pipéridine) ; 1.7-1.5 (m, 2H, H aliphatique pipéridine)

### Stade B : Acide 8-formyl-1,4-dioxa-8-azaspiro[4.5]décane-7-carboxylique

15,25 g du composé obtenu au Stade A (62,7 mmol) est mis en solution dans 160 ml de dioxane. Une solution de 125 mL de KOH 1M est additionnée goutte à goutte et l'ensemble est agité à température ambiante pendant 1 h. On ajoute ensuite 125 mL de HCl 1 M et on extrait le composé avec du dichlorométhane. La phase organique est séchée sur MgSO₄, filtrée et concentré à sec. On obtient le produit du titre sous la forme d'une poudre.
**RMN ¹H:** δ (400 MHz ; dmso-d6 ; 300K) 13.5-12 (m, 1H, OH) ; 8.1 + 8.0 (2s, 1H, CHO); 4.9 + 4.6 (2m, 1H, H tertiaire) ; 4.0-3.8 (m, 4H, éthylènedioxy) ; 4.2 +3.7 (2ms, 1H, H aliphatique pipéridine) ; 3.4 + 2.9 (2m, 1H, H aliphatique pipéridine) ; 2.4-1.5 (m, 4H, H aliphatique pipéridine)
**IR:** ν : OH: 3500-2000 cm⁻¹; -C=O (acide + aldéhyde) :1731 + 1655 cm⁻¹

### Stade C: 5',6'-Dihydro-8'H-spiro[1,3-dioxolane-2,7'-indolizine]-1'-carboxylate de méthyl

A une solution de 13,5 g (62,7 mmol) de l'acide obtenu au Stade B dans 380 mL de dichlorométhane, on ajoute successivement 39,5 mL (238,4 mmol) de triéthylamine, puis par pelleté 12,5 g (65,6 mmol) de chlorure de *para*-toluène sulfonyle et 23,7 mL (238,4 mmol) de chloroacrylate de méthyle. L'ensemble est agité à 80°C pendant 18h. Le milieu réactionnel est ensuite filtré sur célite. Le filtrat est ensuite lavé avec une solution saturée de NaHCO₃ puis avec une solution saturée de NH₄Cl. La phase organique est séchée sur MgSO4, filtrée et concentré à sec. L'huile ainsi obtenue est purifiée par chromatographie flash (gradient heptane/acétate d'éthyle). Le produit est obtenu sous la forme d'un solide.
**RMN ¹H:** δ (400 MHz ; dmso-d6 ; 300K) 6.70 (d, 1H, pyrrol); 6.40 (d, 1H, pyrrole) ; 4.05 (t, 2H, H aliphatique, pipéridine) ; 4.00 (m, 4H, éthylènedioxy) ; 3.70 (s, 3H, méthyle); 3.15 (s, 2H, H aliphatique pipéridine) ; 2.05 (t, 2H, H aliphatique pipéridine)
**IR:** ν :-C=O (ester): 1689 cm⁻¹

### Stade D: 3'-(6-Formyl-1,3-benzodioxol-5-yl)-5',6'-dihydro-8'H-spiro[1,3-dioxolane-2,7'-indolizine]-1'-carboxylate de méthyle

On procède selon le procédé du Stade C de la Préparation 1.

### Stade E : Acide 6-[1'-(méthoxycarbonyl)-5',6'-dihydro-8'H-spiro[1,3-dioxolane-2,7'-indolizin]-3'-yl]-1,3-benzodioxole-5-carboxylique

On procède selon le procédé du Stade D de la Préparation 1.

### Préparation 9: Acide 6-[1-(méthoxycarbonyl)-3-indolizinyl]-1,3-benzodioxole-5-carboxylique

On procède selon le protocole décrit à la Préparation 5 en remplaçant le 2-bromo-4-chlorobenzaldéhyde utilisé au Stade C par le 6-bromo-1,3-benzodioxole-5-carbaldéhyde.

### Préparation 10: Acide 4-méthyl-2-[1-(méthoxycarbonyl)-5,6,7,8-tétrahydro-3-indolizinyl]benzoïque

On procède selon le protocole décrit à la Préparation 1 en remplaçant le 6-bromo-1,3-benzodioxole-5-carbaldéhyde utilisé au Stade C par le 2-bromo-4-méthylbenzaldéhyde.

### Préparation 11: Acide 2-[1-(méthoxycarbonyl)-5,6,7,8-tétrahydro-3-indolizinyl]benzoïque

On procède selon le protocole décrit à la Préparation 1 en remplaçant le 6-bromo-1,3-benzodioxole-5-carbaldéhyde utilisé au Stade C par le 2-bromo-benzaldéhyde.

### Préparation 12: Acide 6-[8-(méthoxycarbonyl)pyrrolo[1,2-a]pyrimidin-6-yl)-1,3-benzodioxole-5-carboxylique

### Stade A : Pyrrolo[1,2-a]pyrimidine-8-carboxylate de méthyle

A une solution de 6,2 g de 2-pyrimidin-2-ylacétate de méthyle (40,75 mmol) dans 250 mL d'acétone, on ajoute successivement 14,04 g (167 mmol) de NaHCO₃ en poudre, 13,2 mL (203,75 mmol) de chloroacetaldehyde, puis 3,54g (40,75 mmol) de bromure de lithium. L'ensemble est chauffé à 60°C pendant 24 h. Le milieu réactionnel est ensuite concentré à sec, repris avec de l'acétate d'éthyle, lavé avec de l'eau, séché sur MgSO₄, filtré puis concentré à sec. Le solide ainsi obtenu est alors purifié par chromatographie sur gel de silice (AcOEt). On obtient le produit attendu sous la forme d'une huile.
***Spectre de Masse :***
Formule brute : C₈H₈N₂O₂
LC/MS: m/z = [M+H]⁺ = 177

### Stade B: 6-(6-Formyl-1,3-benzodioxol-5-yl)pyrrolo[1,2-a]pyrimidine-8-carboxylate de méthyle

A une solution de 3,93 g du composé obtenu au Stade A (22,3 mmol) dans 80 mL de diméthylacétamide anhydre, on ajoute 7,66 g (33,45 mmol) de 6-bromo-1,3-benzodioxole-5-carbaldéhyde et 4,4g (44,6 mmol) d'acétate de potassium. L'ensemble est dégazé sous azote pendant 15 min. On ajoute ensuite 1,56 g (2,23 mmol) de catalyseur PdCl₂(PPh₃)₄. Le milieu réactionnel est chauffé à 130°C pendant 16h sous atmosphère inerte. Après une mise à sec, on reprend le résidu dans le dichlorométhane, on filtre sur un gâteau de célite puis le filtrat est lavé avec de l'eau, séché sur MgSO₄ et concentré à sec. Le solide noir est alors chromatographié sur gel de silice (CH₂Cl₂/MeOH 5%). On obtient le produit attendu sous la forme d'un solide.
***Spectre de Masse :***
Formule brute : C₁₇H₁₂N₂O₃
LC/MS: m/z = [M+H]⁺ = 325

### Stade C: Acide 6-[8-(méthoxycarbonyl)pyrrolo[1,2-a]pyrimidin-6-yl]1,3-benzodioxole-5-carboxylique

A une solution de 2,91 g (8,97 mmol) de l'aldéhyde obtenu au Stade B dans 140 mL d'acétone refroidie à 0°C, on ajoute le 2-méthylbutène, puis goutte à goutte un mélange de 2,8 g (17,94 mmol) de NaH₂PO₄.2H₂O et de 2,84g (31,4 mmol) de NaClO₂ en solution dans 30 mL d'eau. L'ensemble est agité à température ambiante pendant 4h. Le milieu réactionnel est ensuite concentré sous vide pour éliminer l'acétone, placé à 0°C puis acidifié jusqu'à pH = 2-3 en ajoutant goutte à goutte une solution HCl 5N. On observe la formation d'un précipité, qui est filtré, lavé avec de l'eau, puis avec de l'éther diéthylique et séché sous vide.

**RMN¹H:** δ (400 MHz ; dmso-d6 ; 300K) : 12.7 (m, 1H, COO**H**) ; 8.45 (d, 1H, H aromatique, H pyrrolo [1,2-a]pyrimidine); 8.19 (d, 1H, H aromatique, H pyrrolo [1,2-a]pyrimidine); 6.9 (dd, 1H, H aromatique, H pyrrolo [1,2-a]pyrimidine); 7.51 (s, 1H, H aromatique) ; 7.21 (s, 1H, H aromatique) ; 7.07 ( s, 1H, H aromatique) ; 6.2 (s, 2H, H aliphatiques, O-**CH₂**-O) ; 3.8 (s, 3H, H aliphatiques, COO**CH₃**)
**IR :** ν -OH- : 3300 à 1800 cm⁻¹; ν -CO- : 1705 cm⁻¹, ν >C=C< : 1616 cm⁻¹

### Préparation 13: Acide 4-méthoxy-2-[1-(méthoxycarbonyl)-5,6,7,8-tétrahydro-3-indolizinyl]benzoïque

On procède selon le protocole décrit à la Préparation 1 en remplaçant le 6-bromo-1,3-benzodioxole-5-carbaldéhyde utilisé au Stade C par le 2-bromo-4-méthoxybenzaldéhyde.

### Préparation 14: Acide 5-méthoxy-2-[1-(méthoxycarbonyl)-5,6,7,8-tétrahydro-3-indolizinyl]benzoïque

On procède selon le protocole décrit à la Préparation 1 en remplaçant le 6-bromo-1,3-benzodioxole-5-carbaldéhyde utilisé au Stade C par le 2-bromo-5-méthoxybenzaldéhyde.

### Préparation 15 : Acide 7-[1-(méthoxycarbonyl)-5,6,7,8-tétrahydro-3-indolizinyl]-2,3-dihydro-1,4-benzodioxine-6-carboxylique

On procède selon le procédé de la Préparation 1 en remplaçant le 6-bromo-1,3-benzodioxole-5-carbaldéhyde utilisé au Stade C par le 7-bromo-2,3-dihydro-1,4-benzodioxine-6-carbaldéhyde.

### Préparation 16: Acide 2-[1-(méthoxycarbonyl)-3-indolizinyl]benzoïque

On procède selon le procédé de la Préparation 5 en remplaçant le 2-bromo-4-chlorobenzaldéhyde utilisé au Stade C par le 2-bromo-benzaldéhyde.

### Préparation 17: Acide 4-fluoro-2-[1-(méthoxycarbonyl)-3-indolizinyl]benzoïque

On procède selon le procédé de la Préparation 5 en remplaçant le 2-bromo-4-chlorobenzaldéhyde utilisé au Stade C par le 2-bromo-4-fluorobenzaldéhyde.

### Préparation 18: Acide 4-fluoro-2-[1-(méthoxycarbonyl)-5,6,7,8-tétrahydro-3-indolizinyl]benzoïque

On procède selon le procédé de la Préparation 1 en remplaçant le 6-bromo-1,3-benzodioxole-5-carbaldéhyde utilisé au Stade C par le 2-bromo-4-fluorobenzaldéhyde.

### Préparation 1': Chlorhydrate de (3R)-3-méthyl-1,2,3,4-tétrahydroisoquinoline

### Stade A: {(3S)-2-[(4-Méthylphényl)sulfonyl]-1,2,3,4-tétrahydroisoquinolin-3-yl}méthyl 4-méthylbenzènesulfonate

A une solution de 30,2 g de [(3*S*)-1,2,3,4-tétrahydroisoquinolin-3-yl]méthanol (185 mmol) dans 750 mL de dichlorométhane sont ajoutés successivement 91,71 g de chlorure de tosyle (481 mmol), puis, au goutte à goutte, 122,3 mL de *N,N,N-*triéthylamine (740 mmol). Le milieu réactionnel est ensuite agité à température ambiante durant 20 h. Il est ensuite dilué avec du dichlorométhane, lavé successivement avec une solution HCl 1M, une solution saturée de NaHCO₃, puis une solution saturée de NaCl jusqu'à neutralité. La phase organique est alors séchée sur MgSO₄, filtrée, concentrée à sec. Le solide obtenu est alors solubilisé dans un volume minimum de dichlorométhane, puis est ajouté du cyclohexane jusqu'à formation d'un précipité. Ce précipité est alors filtré et lavé avec du cyclohexane. Après séchage, le produit du titre est obtenu sous forme de cristaux.

**RMN ¹H:** δ (400 MHz ; dmso-d6 ; 300K) : 7.75 (d, 2H , H aromatiques, *ortho* O-tosyle); 7.6 (d, 2H , H aromatiques, *ortho N*-tosyle); 7.5 (d, 2H , H aromatiques, *méta* O-tosyle); 7.3 (d, 2H , H aromatiques, *méta N*-tosyle); 7.15-6.9 (m, 4H, H aromatiques, tétrahydro isoquinoline); 4.4-4.15 (dd, 2H, H aliphatiques, tétrahydroisoquinoline); 4.25 (m, 1H, H aliphatique, tétrahydroisoquinoline); 4.0-3.8 (2dd, 2H, H aliphatiques, **CH₂**-O-tosyle) ; 2.7 (2dd, 2H, H aliphatiques, tétrahydroisoquinoline); 2.45 (s, 3H, O-SO₂-Ph- **CH₃**); 2.35 (s, 3H, *N*-SO₂-Ph- **CH₃**)
**IR** : ν : -SO₂ : 1339-1165 cm⁻¹

### Stade B: (3R)-3-Méthyl-2-[(4-méthylphényl)sulfonyl]-1,2,3,4-tétrahydroisoquinoliue

A une suspension de 8,15 g (214,8 mmol) de LiAlH₄ dans 800 mL de méthyl *tert-*butyl éther (MTBE), on ajoute 101,2 g du dérivé ditosylé obtenu au Stade A (214,8 mmol) en solution dans 200 mL de MTBE. L'ensemble est ensuite chauffé à 50°C pendant 2h. On laisse refroidir et on se place à 0°C, puis on ajoute, goutte à goutte, 12 mL d'une solution de NaOH 5N. L'ensemble est agité à température ambiante pendant 45 minutes. Le solide ainsi obtenu est alors filtré, lavé avec du MTBE puis avec du dichlorométhane. Le filtrat est ensuite concentré à sec. On obtient alors le produit du titre sous la forme d'un solide.

**RMN ¹H:** δ (400 MHz ; dmso-d6 ; 300K) : 7.70 (d, 2H , H aromatiques, *ortho N-*tosyle); 7.38 (d, 2H , H aromatiques, *méta N*-tosyle); 7.2-7.0 (m, 4H, H aromatiques, tétrahydroisoquinoline); 4.4 (m, 2H, H aliphatiques, tétrahydroisoquinoline); 4.3 (m, 1H, H aliphatique, tétrahydroisoquinoline); 2.85-2.51 (2dd, 2H, H aliphatiques, tétrahydro isoquinoline); 2.35 (s, 3H, *N*-SO₂-Ph- **CH₃**); 0.90 (d, 3H, tétrahydroisoquinoline-**CH₃**)
**IR** : ν : -SO₂ : 1332-1154 cm⁻¹

### Stade C: (3R)-3-Méthyl-1,2,3,4-tétrahydroisoquinoline

A une solution de 31,15 g (103,15 mmol) du dérivé monotosylé obtenu au Stade B dans 500 mL de méthanol anhydre, on ajoute 3,92g (161 mmol) de tournure de magnésium par pelletés. L'ensemble est agité en présence d'ultra-sons pendant 96 h. Le milieu réactionnel est ensuite filtré et le solide est lavé plusieurs fois avec du méthanol. Le filtrat est ensuite concentré à sec. Après purification par colonne chromatographique sur gel de silice (dichlorométhane /EtOH /NH₄OH), on obtient le produit du titre sous la forme d'une huile.

**RMN ¹H:** δ (400 MHz ; dmso-d6; 300K): 7.05 (m, 4H, H aromatiques, tétrahydroisoquinoline); 3.90 (m, 2H, H aliphatiques, tétrahydroisoquinoline); 2.85 (m, 1H, H aliphatique, tétrahydroisoquinoline); 2.68-2.4 (2dd, 2H, H aliphatiques, tétrahydro isoquinoline); 1.12 (d, 3H, tétrahydroisoquinoline-**CH₃**) ; 2.9-2.3 (m, large, 1H, **HN**(tétrahydroisoquinoline))
**IR :** ν : -NH : 3248 cm⁻¹

### Stade D: Chlorhydrate de (3R)-3-méthyl-1,2,3,4-tétrahydroisoquinoline

A une solution de 14,3 g (97.20 mmol) du composé obtenu au Stade C dans 20 mL d' éthanol anhydre, on ajoute ,goutte à goutte, 100 mL d'une solution d'éther chlorhydrique 1M. L'ensemble est agité à température ambiante pendant 1h, puis filtré. Les cristaux ainsi obtenus sont lavés avec de l'éther éthylique. Après séchage, le produit du titre sous la forme de cristaux.

**RMN ¹H:** δ (400 MHz ; dmso-d6 ; 300K): 9.57 (m, large, 2H, **NH₂**⁺(tétrahydro isoquinoline).7.22 (m, 4H, H aromatiques, tétrahydroisoquinoline); 4.27 (s, 2H, H aliphatiques, tétrahydroisoquinoline); 3.52 (m, 1H, H aliphatique, tétrahydroisoquinoline); 3.03--2.85 (2dd, 2H, H aliphatiques, tétrahydroisoquinoline); 1.39 (d, 3H, tétrahydro isoquinoline-**CH₃**)
**IR :** ν : -NH₂⁺ : 3000-2300 cm⁻¹ ; ν : -CH aromatique : 766 cm⁻¹

### Préparation 2': Chlorhydrate de (3S)-3-[2-(morpholin-4-yl)éthyl]-1,2,3,4-tétrahydroisoquinoline

### Stade A: (3S)-3-(2-Morpholino-2-oxo-éthyl)-3,4-dihydro-1H-isoquinoline-2-carboxylate de tert-butyle

A une solution de 3 g (10,30 mmol) d'acide **[(3*S*)-2-(*tert*-butoxycarbonyl)-1,2,3,4-**tétrahydroisoquinolin-3-yl]acétique dans 100mL de dichlorométhane, on ajoute goutte à goutte 1,10 mL (11,32 mmol) de morpholine, toujours goutte à goutte 4,3 mL (30,9 mmol) de triéthylamine, 2,20 g (12,40 mmol) de 1,2-dichlorométhane et 1,70g (1,68 mmol) d'hydroxybenzotriazole. L'ensemble est agité à température ambiante pendant 15 h. Le milieu réactionnel est ensuite dilué avec du dichlorométhane, lavé successivement avec une solution HCl 1M, une solution saturée de NaHCO₃, puis une solution saturée de NaCl jusqu'à neutralité. La phase organique est alors séchée sur MgSO₄, filtrée et concentrée à sec. Après purification par colonne chromatographique sur gel de silice (dichlorométhane /MeOH), on obtient le produit du titre sous la forme d'une huile.

**RMN ¹H:** δ (400 MHz; dmso-d6; 300K): 7.20-7.10 (m, 4H, H aromatiques, tétrahydroisoquinoline); 4.70 (m, 1H, H aliphatiques, CH tétrahydroisoquinoline); 4.75-4.20 (2m, 2H, H aliphatiques, CH₂ en alpha de N tétrahydroisoquinoline); 3.60 (m, 8H, H aliphatiques, morpholine); 3.00 et 2.70 (2dd, 2H, H aliphatique, tétrahydroisoquinoline); 2.50-2.20 (2d, 2H, H aliphatiques, CH₂CO); 1.40 (s, 9H, ^{t}Bu)
**IR :** ν : C=O : 1687 ;1625 cm⁻¹

### Stade B: Chlorhydrate de 1-(morpholin-4-yl)-2-[(3S)-1,2,3,4-tétrahydroisoquinolin-3-yl]éthanone

A une solution de 2,88 g (7,18 mmol) du composé obtenu au Stade A dans 16 mL de dichlorométhane, on ajoute goutte à goutte 80 mL (80 mmol) d'une solution d'éther chlorhydrique 1M. L'ensemble est agité à température ambiante pendant 15 h, puis la suspension est filtrée et le précipité lavé à l'éther. Après séchage, on obtient le produit du titre sous la forme d'un solide.

**RMN ¹H:** δ (400 MHz ; dmso-d6 ; 300K) : 9.80-9.50 (m, 2H, NH₂⁺); 7.30-7.10 (m, 4H, H aromatiques, tétrahydroisoquinoline); 4.30 (m, 2H, H aliphatiques, CH₂ en alpha de N tétrahydroisoquinoline); 3.80 (m, 1H, H aliphatiques, CH tétrahydroisoquinoline); 3.70-3.40 (2m, 8H, H aliphatiques, morpholine); 3.15 et 2.8 (m, 4H, H aliphatique, CH₂ tétrahydroisoquinoline et CH₂CO)
**IR :** ν : -NH₂⁺: 2800-1900 cm⁻¹; ν : C=O : 1620 cm⁻¹

### Stade C: Chlorhydrate de (3S)-3-[2-(morplrolin-4yl)éthyl]-1,2,3,4-tétrahydroisoquinoline

On prépare une solution de 2,2 g (7,44 mmol) du composé obtenu au Stade B dans 22 mL de MTBE et 5 mL de dichlorométhane. Après refroidissement dans un bain de glace à 0°C, on y ajoute goutte à goutte 15 mL (15 mmol) d'une solution de LiAlH₄ 1M dans le tétrahydrofurane. L'ensemble est ensuite agité à température ambiante pendant 6 h. On se place à 0°C, puis on ajoute goutte à goutte 1 mL d'une solution de NaOH 5N. L'ensemble est agité à température ambiante pendant 45 minutes. Le solide est alors filtré et lavé avec du MTBE, puis avec du dichlorométhane et le filtrat est concentré à sec. L'huile ainsi obtenue est diluée au dichlorométhane et on ajoute goutte à goutte 6,3 mL d'une solution d'éther chlorhydrique 1M. L'ensemble est agité à température ambiante pendant 1h, puis filtré. Les cristaux ainsi obtenus sont lavés avec de l'éther éthylique. Après séchage, on obtient le produit du titre sous la forme d'un solide.

**RMN ¹H:** δ (400 MHz ; dmso-d6 ; 300K) : 11.35 + 9.80 (2m, 2H, NH₂⁺); 10.00 (m, H, NH⁺); 7.20 (m, 4H, H aromatiques, tétrahydroisoquinoline); 4.30 (s, 2H, H aliphatiques, CH₂ en alpha de N tétrahydroisoquinoline); 4.00 + 3.85 (2m, 4H, H aliphatiques, CH₂ en alpha de N morpholine); 3.70 (m, 1H, H aliphatiques, CH tétrahydroisoquinoline); 3.55-3.30 (m, 4H, H aliphatiques, CH en alpha de O morpholine et CH₂-Morpholine ); 3.15 (dd, 1H, H aliphatique, CH₂ tétrahydroisoquinoline); 3.10 (m, 2H, H aliphatique, CH en alpha de O morpholine) ; 2.90 (dd, 1H, H aliphatique, CH₂ tétrahydroisoquinoline); 2.30 + 2.15 (2m, 2H, H aliphatique, CH₂-tétrahydroisoquinoline)
**IR :** ν : NH⁺ / -NH₂⁺ : entre 3500 et 2250 cm⁻¹; ν : C=C : faible 1593 cm⁻¹; ν : C-H aromatique :765 cm⁻¹

### Préparation 3': {2-[(3S)-1,2,3,4-tétrahydroisoquinolin-3-yl]éthyl}carbamate de tert-butyle

### Stnde A: (3S)-3-(2-Hydroxyéthyl)-3,4-dihydroisoquinoline-2(1H)-carboxylate de benzyle

On obtient le composé du titre à partir de l'acide (3*S*)-2-[(benzyloxy)carbonyl]-1,2,3,4-tétrahydroisoquinoline-3-carboxylique en se basant sur un protocole de la littérature (Jinlong Jiang et al Bioorganic & Medicinal Chemistry Letters, 14, 1795, 2004**).**

### Stade B: (3S)-3-{2-[(Méthylsulfonyl)oxy]éthyl}-3,4-dihydroisoquinoline-2(1H)-carboxylate de benzyle

A une solution de 10,6 g du composé du Stade A (35,6 mmol) dans 350 mL de CH₂Cl₂ anhydre placée à 0°C, on ajoute successivement la triéthylamine 10,1 mL (71,2 mmol) puis, goutte à goutte, le chlorure de méthanesulfonyle 3,1 mL (39 mmol). Le milieu réactionnel est ensuite agité à température ambiante pendant 2h. On hydrolyse ensuite en ajoutant lentement de l'eau. Le produit est extrait plusieurs fois avec CH₂Cl₂. Les phases organiques sont alors regroupées et lavées successivement avec une solution d'HCl 1N, une solution saturée de NaCl, une solution saturée de NaHCO₃ et une solution saturée de NaCl jusqu'à neutralité. Puis elles sont séchées sur MgSO₄ et concentrées à sec. Après purification par chromatographie sur gel de silice (gradient éther de pétrole/ AcOEt), on obtient le produit attendu sous la forme d'une mousse.
**LC/MS:** m/z = (M + H)⁺ = 375

### Stade C: (3S)-3-(Cyanométhyl)-3,4-dihydroisoquinoline-2(1H)-carboxylate de benzyle

A une solution de 15,4 g du dérivé obtenu au Stade B (41,02 mmol) dans 250 mL de DMSO anhydre, on ajoute 22g (449 mmol) de cyanure de sodium. L'ensemble est ensuite chauffé à 60°C pendant 12 h. On laisse refroidir, puis on dilue le milieu réactionnel en ajoutant de l'acétate d'éthyle. On hydrolyse ensuite avec une solution saturée en NaHCO₃. Après 2 nouvelles extractions avec de l'acétate d'éthyle, les phases organiques sont regroupées, lavées avec H₂O, séchées sur MgSO₄ et concentrées à sec. Après purification par chromatographie sur gel de silice (hexane/ AcOEt 7/3), on obtient le produit attendu sous la forme d'une huile.
**LC/MS:** m/z = [M+H]⁺ = 307.1

### Stade D: (3S)-3-(2-Aminoéthyl)-3,4-dihydroisoquinoline-2(1H)-carboxylate de benzyle

A une solution de 15,4 g du dérivé obtenu au Stade C (50,3 mmol) dans 300 mL de THF anhydre placée à 0°C, on ajoute goutte à goutte une solution de BH₃-THF 1N. On laisse le milieu réactionnel revenir progressivement à température ambiante, puis l'ensemble est agité pendant 14h. Le milieu réactionnel est ensuite hydrolysé par addition lente d'une solution saturée de NH₄Cl. Après 2 extractions avec de l'acétate d'éthyle, les phases organiques sont regroupées et séchées sur MgSO₄. Après concentration à sec, on obtient le produit attendu sous la forme d'une mousse qui est utilisée directement sans purification pour l'étape suivante de protection.

### Stade E: (3S)-3-{2-[(tert-Butoxycarbonyl)amino]éthyl}-3,4-dihydroisoquinoline-2(1H)-carboxylate de benzyle

A une solution de 15,6 g du dérivé obtenu au Stade D (50,3 mmol) dans 670 mL de CH₂Cl₂, on ajoute successivement 13.2 g (60,36 mmol) de Boc₂O par pelletés, la triéthylamine 14 mL (100,6 mmol) et la DMAP en quantité catalytique. L'ensemble est agité à température ambiante pendant 5h. Le milieu réactionnel est ensuite hydrolysé avec de l'eau et extrait 2 fois avec CH₂Cl₂. Les phases organiques sont regroupées, lavées avec de l'eau, et séchées sur MgSO₄. Après concentration à sec et purification par chromatographie sur gel de silice (gradient heptane/AcOEt), on obtient le produit attendu sous la forme d'une huile.
**LC/MS:** m/z = (M + H)⁺ = 411

### Stade F:{2-[(3S)-1,2,3,4-Tétrahydroisoquinolin-3-yl]éthyl}carbamate de tert-butyle

A une solution de 10,4 g du dérivé obtenu au Stade E (25,5 mmol) dans 210 mL de MeOH anhydre, on ajoute 2,71 g (2,55 mmol) de Pd/C 10%. L'ensemble est dégazé pendant 30 minutes puis agité s sous atmosphère d'hydrogène pendant 16h. Le milieu réactionnel est ensuite filtré et concentré à sec. On obtient le produit attendu sous la forme d'un solide qui est repris dans un mélange pentane/Et₂O (90/10), trituré et filtré. Après séchage, on obtient le produit sous la forme d'un solide.
**RMN ¹H :** δ (400 MHz; dmso-d6; 300K): 7.1-6.98 (m, 4H, H aromatiques, tétrahydroisoquinoline); 6.83 (m, 1H, CH₂**NH**Boc) ; 3.85 (s, 2H, H aliphatiques, tétrahydroisoquinoline); 3.09 (q, 2H, **CH₂**NHBoc); 2.73 (m, 1H, H aliphatiques, tétrahydroisoquinoline); 2.70 et 2.39 (2m, 2H, H aliphatiques, tétrahydroisoquinoline); 1.63 (m , 2H, H aliphatiques) ; 1.38 (s, 9H, NHCOO**tBu**)
**IR :** ν : >NH : 3378, -3201 cm⁻¹ (amine, amide) ; ν : >C=O : 1683 cm⁻¹ (amide) ; ν : >NH : 1524 cm⁻¹ (amide) ; ν : >C=O : 1168 cm⁻¹
**LC/MS:** m/z = [M+H]⁺ = 277

### Préparation 4': (3R)-3-[3-(Morpholin-4-yl)propyl]-1,2,3,4-tétrahydroisoquinoline

### Stade A: {(3S)-2-[(4-Méthylphényl)sulfonyl]-1,2,3,4-tétrahydroisoquinolin-3-yl}méthyl 4-méthylbenzènesulfonate

Le procédé est identique à celui du Stade A de la Préparation 1'.

### Stade B: 2-({(3R)-2-[(4-Méthylphényl)sulfonyl]-1,2,3,4-tétrahydroisoquinolin-3-yl}méthyl)-3-(morpholin-4-yl)-3-oxopropanoate de tert-butyle

A une suspension de 1 g de NaH (60%) (25,08 mmol) dans 30 mL de MTBE, on ajoute goutte à goutte une solution de 5 g de 3-morpholino-3-oxopropanoate de *tert*-butyle (21,81 mmol) dans 20 mL de MTBE anhydre. Cette suspension est agitée à température ambiante pendant 1h, puis on ajoute le composé obtenu au Stade A sous forme d'une poudre. L'ensemble est agité à 60°C pendant 30h. Une solution de 100 mL de chlorure d'ammonium saturée est ajoutée. Cette solution est extraite au dichlorométhane. La phase organique est alors séchée sur MgSO₄, filtrée et concentrée à sec. Après purification par colonne chromatographique sur gel de silice (dichlorométhane/MeOH), on obtient le produit attendu sous la forme d'une huile.
**RMN ¹H** (500 MHz, dmso-d6) δ ppm : 7.63/7.59 (2d, 2 H), 7.3/7.26 (2d, 2 H), 7.13 (m, 2 H), 7.09/6.97 (2t, 2 H), 4.64/4.55/4.36/4.28 (2AB, 2 H), 4.25/4.11 (2m, 1 H), 3.81 (m, 1 H), 3.73-3.48 (m, 4 H), 3.57-3.32 (m, 4 H), 2.51 (m, 2 H), 2.32/2.31 (2s, 3 H), 1.88/1.79 (2m, 2 H), 1.39/1.38 (2s, 9 H)
**IR (ATR)** cm⁻¹ : ν : >C=O : 1731 (ester); ν : >C=O : 1644 (amide) ; ν : -SO2 : 1334-1156 ; ν : >C-O-C< : 1115; γ: >CH-Ar : 815-746-709

### Stade C: Acide 2-({(3R)-2-[(4-méthylphényl)sulfonyl]-1,2,3,4-tétrahydroisoquinolin-3-yl}méthyl)-3-(morpholin-4-yl)-3-oxopropanoique

A une solution de 9,5 g (17,97 mmol) du composé obtenu au Stade B dans 40 mL de dioxane ,on ajoute goutte à goutte 20 mL d'une solution d'acide chlorhydrique 4M dans le dioxane. L'ensemble est agité à température ambiante pendant 48h, puis la solution est concentrée à sec. Après séchage, on obtient le produit attendu sous la forme d'une huile.
**RMN ¹H** (400 MHz, dmso-d6) δ ppm : 12.75 (m, 1 H), 7.6 (2*d, 2 H), 7.3 (2*d, 2 H), 7.1/6.95 (2*m, 4 H), 4.7-4.2 (d, 2 H), 4.25/4.12 (2*m, 1 H), 3.9-3.3 (m, 9 H), 2.55 (d, 2 H), 2.3 (2*s, 3 H), 1.8 (t, 2 H)
**IR (ATR)** cm⁻¹ : v : -OH : 3500 à 2000; ν : >C=O : 1727 (acide) ; ν : >C=O : 1634 (amide) ; v : -S02 : 1330-1155

### Stade D: 3-{(3R)-2-[(4-Méthylphényl)sulfonyl]-1,2,3,4-tétrahydroisoquinolin-3-yl}-1-(morpholin-4-yl)propan-1-one

A une solution de 7,80 g (16,51 mmol) du composé obtenu au Stade C dans 100mL de DMSO, on ajoute 1,16 g (19,83 mmol) de chlorure de sodium solide, puis goutte à goutte 5 mL d'eau. L'ensemble est agité à 130°C pendant 1h, puis la solution est concentrée au ¾. Le milieu réactionnel est ensuite dilué avec du dichlorométhane, lavé successivement avec une solution saturée de chlorure de lithium, puis une solution saturée de NaCl. La phase organique est alors séchée sur MgSO₄, filtrée, concentrée à sec. Après purification par colonne chromatographique sur gel de silice (cyclohexane/acétate d'éthyle), on obtient le produit attendu sous la forme d'une huile.
**RMN ¹H** (400 MHz, dmso-d6) δ ppm : 7.65 (d, 2 H), 7.3 (d, 2 H), 7.15/7 (2 m, 4 H), 4.6 (d, 1 H), 4.25 (d, 1 H), 4.2 (m, 1 H), 3.5 (m, 4 H), 3.4 (2 m, 4 H), 2.6 (2 dd, 2 H), 2.35 (s, 3 H), 2.3 (m, 2 H), 1.5 (quad., 2 H)
**IR (ATR)** cm⁻¹ : ν : >C=O : 1639 ; ν : -SO2 : 1331-1156 ; γ : >CH-Ar : 815-675

### Stade E: (3R)-2-[(4-Méthylphényl)sulfonyl]3-[3-(morpholin-4-yl)propyl]1,2,3,4-tétrahydroisoquinoline

A une solution de 6,0 g (14,0 mmol) du composé obtenu au Stade D dans 60mL de MTBE et 14 mL de dichlorométhane, on ajoute 1,06g (28 mmol) de LAH par portion sur 5 minutes. L'ensemble est agité à température ambiante pendant 15 h. On ajoute goutte à goutte 1,5 mL d'eau et on agite pendant 15min. Ensuite, on ajoute goutte à goutte 1,5mL de soude 5 M et on agite 15min. Le milieu réactionnel est ensuite dilué avec du MTBE et du dichlorométhane. Puis, la suspension est filtrée et le précipité est lavé avec du MTBE et du dichlorométhane. La phase organique est alors séchée sur MgSO₄, filtrée, et concentrée à sec. Après purification par colonne chromatographique sur gel de silice (dichlorométhane /EtOH /NH₄OH), on obtient le produit attendu sous la forme d'une huile.
**RMN ¹H** (400 MHz, dmso-d6) δ ppm : 7.68 (d, 2 H), 7.32 (d, 2 H), 7.1 (massif, 4 H), 4.65/4.23 (AB, 2 H), 4.2 (m, 1 H), 3.55 (t, 4 H), 2.7/2.6 (ABx, 2 H), 2.35 (s, 3 H), 2.25 (t, 4 H), 2.2 (t, 2 H), 1.4/1.3 (2m, 4 H).
**IR (ATR)** cm⁻¹: ν : -S02 : 1333-1158

### Stade F : (3R)-3-[3-(Morpholin-4-yl)propyl]-1,2,3,4-tétrahydroisoquinoline

A une solution de 1,50 g (3,62 mmol) du dérivé obtenu au Stade E dans 20 mL de méthanol anhydre, on ajoute 2,0 g (82,3 mmol) de tournure de magnésium par pelletés. L'ensemble est agité en présence d'ultrasons pendant 96 h. Le milieu réactionnel est ensuite filtré, le solide est lavé plusieurs fois avec du méthanol, et le filtrat est concentré à sec. Après purification par colonne chromatographique sur gel de silice (dichlorométhane /EtOH /NH₄OH), on obtient le produit attendu sous la forme d'une huile.
**RMN ¹H** (400 MHz, dmso-d6) δ ppm : 7.3 (d, 2 H), 7.1 (t, 2 H), 7.1 (d+t, 3 H), 7 (d, 2 H), 3.9 (s, 2 H), 3.55 (t, 4 H), 2.75 (m, 1 H), 2.72/2.45 (dd, 2 H), 2.35 (t, 4 H), 2.25 (t, 2 H), 1.6 (m, 2 H), 1.45 (m, 2 H)
**IR (ATR)** cm⁻¹: ν : >NH2+/NH+ : 3500-2300 ; ν : >C-O-C< : 1115
***Masse haute résolution (ESI*+-/FIA/HR*) :***
Formule brute : C₁₆ H₂₄ N₂ O
[M+H]⁺ calculé : 261.1961
[M+H]⁺ mesuré : 261.1959

### Préparation 5': Chlorhydrate de (3S)-3-[2-(3,3-difluoropipéridin-1-yl)éthyl]-1,2,3,4-tétrahydroisoquinoline

On procède selon le procédé de la Préparation 2' en remplaçant la morpholine utilisée au Stade A par la 3,3-difluoro-1-pipéridine.
**RMN ¹H :** δ (400 MHz ; dmso-d6 ; 300K) : 11.3 (m, 1H, **NH⁺**) ; 10.2-9.8 (m, 2H, **NH₂⁺**); 7.25 (m, 4H, H aromatiques, tétrahydroisoquinoline); 4.3 (large s, 2H, H aliphatiques, CH tétrahydroisoquinoline); 4.0-3.3 (m, 7H, H aliphatiques) ; 3.15-2.95 ( dd, 2H, H aliphatiques, CH tétrahydroisoquinoline); 2.4-1.9 (m, 6H, H aliphatiques, H 3,3-difluoro-1-pipéridine)
**IR :** ν : NH⁺ /NH₂⁺ : entre 300 et 2500 cm⁻¹ ; ν : C-F : 1204 cm⁻¹

### Préparation 6' : Chlorhydrate de (3S)-3-[2-(3-méthoxyazétidin-1-yl)éthyl]-1,2,3,4-tétrahydroisoquinoline

On procède selon le procédé de la Préparation 2' en remplaçant la morpholine utilisée au Stade A par la 3-méthoxyazétidine.
**RMN ¹H :** δ (400 MHz ; dmso-d6 ; 300K) : 11.3 (m, 1H, NH⁺); 10.00 (m, 2H, NH₂⁺); 7.20 (m, 4H, H aromatiques, tétrahydroisoquinoline); 4.4 (m, 1H, H aliphatique, 3-Méthoxy azétidine) ; 4.30 (s, 2H, H aliphatiques, tétrahydroisoquinoline); 4.2 -3.45 (m, 4H, 3-Méthoxyazétidine) ; 4.2 -3.6 (m, 3H , H aliphatiques) ; 3.1 et 2.95 (dd, 2H, H aliphatiques); 3.25 (s, 3H, **OCH₃**)

### Préparation 7': Chlorhydrate de (3S)-3-méthyl-1,2,3,4-tétrahydroisoquinoline

On procède selon le procédé de la Préparation 1' en remplaçant le [(3*S*)-1,2,3,4-tétrahydroisoquinolin-3-yl]méthanol utilisé au Stade A par le [(3*R*)-1,2,3,4-tétrahydroisoquinolin-3-yl]méthanol.

### Préparation 1": N-(4-{[tert-Butyl(diméthyl)silyl]oxy}phényl)-1-méthyl-1H-pyrazol-4-amine

### Stade A: 4-{[tert-Butyl(dimétltyl)silyl]oxy}aniline

Le composé du titre est obtenu à partir de la 4-aminophénol dans le THF en présence d'imidazole et de chlorure de *tert*-butyl(diméthyl)silyl selon le protocole décrit dans la littérature (S. Knaggs et al, Organic & Biomolecarlar Chemistry, 3(21), 4002-4010; 2005**).**
**RMN ¹H :** δ (400 MHz ; dmso-d6 ; 300K) : 6.45-6.55 (dd, 4H, H aromatiques); 4.60 (m, 2H, **NH₂**-Ph) ; 0.90 (s, 9H, Si (CH₂)₂CH(**CH₃**)₂); 0.10 (s, 6H, Si (**CH₂**)₂CH(CH₃)₂)
**IR :** ν : -NH₂⁺ : 3300-3400 cm⁻¹

### Stade B: N-14-[tert-Butyl(diméthyl)silyl]oxyphényl]-1-méthyl-pyrazol-4-amine

A une solution de 30,8 g (0,137 mol) du composé du Stade A dans 525 mL de toluène anhydre, on ajoute successivement 29,8g de *tert*-butylate de sodium (0,310 mol), 4,55 g de Pd₂(dba)₃ (aussi appelé tris(dibenzylidéneacétone)dipalladium(0)) (4,96 mmol), 4,81 g de 2-di-*tert*-butylphosphino-2',4',6'-tri-isopropyl-1,1'-biphényl (9,91 mmol) et 12,8 mL de 4-bromo-1-méthyl-1*H-*pyrazole (0,124 mol). L'ensemble est dégazé sous argon durant 30 mn puis chauffé à reflux pendant 3 h. On laisse refroidir. Le milieu réactionnel est concentré à sec, puis repris dans le dichlorométhane, filtré sur célite, puis de nouveau concentré à sec. Le résidu est alors purifié par chromatographie sur gel de silice (gradient CH₂Cl₂/AcOEt) pour fournir le produit attendu sous la forme d'un solide.
**RMN ¹H** : δ (400 MHz ; dmso-d6 ; 300K) : 7.55 (s, 1H, pyrazole); 7.23 (s, 1H, pyrazole); 7.18 (large s, 1H, **NH₂**-PI1) ;6.64 (m, 4H, H aromatiques); 3.77 (s, 3H, **CH₃**-pyrazole); 0.90 (s, 9H, Si (CH₂)₂CH(**CH**₃)₂); 0.12 (s, 6H, Si (**CH₂**)₂CH(CH₃)₂)
**IR :** v -NH⁺ : 3275 cm⁻¹; v Ar et C=N : 1577et 1502 cm⁻¹; v -Si-C-: 1236 cm⁻¹; v -Si-O-: 898 cm⁻¹; v -Si-C-: 828, 774 cm⁻¹

### Préparation 2" : N-(4-{[tert-Butyl(diméthyl)silyl]oxy}phényl)-1-méthyl-1H-indol-5-amine

On procède selon le procédé de la Préparation 1" en remplaçant le 4-bromo-1-méthyl-1*H-*pyrazole utilisé au Stade B par le 5-bromo-1-méthyl-1*H-*indole.

### Préparation 3" : N-(4-{[tert-Butyl(diméthyl)silyl]oxy}phényl)-1-[2-(morpholin-4-yl)éthyl]-1H-indol-5-amine

### Stade A : 5-Bromo-1[2-(morpholin-4 yl)éthyl]-1H-indole

A une suspension de NaH (4,5 g ; 112 mmol) dans le THF anhydre (300 mL) placée à 0°C, on ajoute par portions le 5-bromo-1*H-*indole (10,4 g ; 51 mmol). Après 20 minutes d'agitation à 0°C, le chlorhydrate de 4-(2-chloroéthyl)morpholine (10,4 g ; 56 mmol) est ajouté par portions en 1h. Après une nuit d'agitation à température ambiante, le milieu réactionnel est placé pendant 5h à 80°C. Il est ensuite coulé sur un mélange de bicarbonate de sodium aqueux et de dicholorométhane. La phase aqueuse est extraite au dichlorométhane. La phase organique est séchée sur du MgSO4, concentrée à sec et le résidu est purifié par chromatographie sur gel de silice (gradient CH₂Cl₂/MeOH) pour fournir le produit attendu sous la forme d'une huile.
**RMN ¹H :** δ (400 MHz ; CDCl3 ; 300K) :7.75 (d, 1H) ; 7.30 (dd, 1H) ; 7.20 (d, 1H) ; 7.15 (d, 1H) ; 6.40 (d, 1H) ; 4.20 (t, 2H) ; 3.70 (m, 4H) ; 2.75 (t, 2H) ; 2.45 (m, 4H)

### Stade B : 5-Bromo-1-[2-(morpholin-4yl)éthyl]-1H-indole

On procède selon le procédé de la Préparation 1" en remplaçant le 4-bromo-1-méthyl-1*H-*pyrazole utilisé au Stade B par le composé obtenu au Stade A.
**RMN ¹H :** δ (400 MHz ; dmso-d6 ; 300K) : 7.35 (d, 1H); 7.15 (s, 1H); 6.85 (d, 3H); 6.70 (d, 2H); 7.30 (d, 1H); 6.25 (d, 1H), 4.20 (t, 2H); 3.55 (m, 4H); 2.65 (t, 2H); 2.45 (m, 4H); 1.45 (s, 9H), 0.15 (s, 6H)

### Préparation 4" : N-(4-{[tert-Butyl(diméthyl)silylloxylphényl)-1-[2-(morpholin-4-yl)éthyl]-2,3-dihydro-1H-indol-5-amine

On procède selon le procédé de la Préparation 2" en remplaçant le 5-bromobindole utilisé au Stade A par le 5-bromo-2,3-dihydro-1*H-*indole.

### Préparation 5": N-(4-{[tert-Butyl(diméthyl)silyl]oxy}phényl)-4-fluoroaniline

On procède selon le procédé de la Préparation 1" en remplaçant le 4-bromo-1-méthyl-1*H-*pyrazole utilisé au Stade B par le 1-bromo-4-fluorobenzène.

### Préparation 6" : N-(4-{[tert-Butyl(diméthyl)silyl]oxy)phényl)-3-fluoro-4-méthylaniline

On procède selon le procédé de la Préparation 1" en remplaçant le 4-bromo-1-méthyl-1*H-*pyrazole utilisé au Stade B par le 4-bromo-2-fluoro-1-méthylbenzène.

### Préparation 7" : N-(4-{[tert-Butyl(diméthyl)silyl]oxy}phényl)-1-méthyl-1H-indazol-5-amine

On procède selon le procédé de la Préparation 1" en remplaçant le 4-bromo-1-méthyl-1*H-*pyrazole utilisé au Stade B par le 5-bromo-1-méthyl-1*H-*indazole.

### Préparation 8" : 4-{[tert-Butyl(diméthyl)silyl]oxy}-N-phénylaniline

A une solution de 12 g de 4-anilinophénol (64,7 mmol) dans 200 mL d'acétonitrile sont ajoutés à température ambiante 6,7 g d'imidazole (97,05 mmol) et 11,7 g de *tert-*butyl(chloro)diméthylsilane (77,64 mmol). L'ensemble est mis sous agitation à 70°C pendant 4 heures. Puis, le milieu réactionnel est versé sur de l'eau et extrait avec de l'éther. La phase organique est ensuite séchée sur sulfate de magnésium, puis filtrée et évaporée à sec. Le produit brut ainsi obtenu est alors purifié par chromatographie sur gel de silice (gradient éther de pétrole / dichlorométhane). Le produit du titre est obtenu sous la forme d'une poudre.
**RMN¹H:** δ (400 MHz ; dmso-d6 ; 300K) : 7.84 (s, 1H NH) ; 7.17 (t, 2H aniline) ; 6.98 (d, 2H phénoxy) ; 6.94 (d, 2H aniline) ; 6.76 (d, 2H phénoxy) ; 6.72(t, 1H aniline) ; 0.95 (s, 9H *tert-butyl*) ; 0.15 (s, 6H diméthyl)
**IR :** v : >NH : 3403 cm⁻¹ ; >Ar : 1597 cm⁻¹

### Préparation 9" : 4-Benzyloxy-N-phényl-aniline

A une solution de 4-hydroxy-*N-*phényl-aniline (30 g ; 162 mmol) dans de l'acétonitrile (400 mL), on ajoute 58 g de Cs2CO3 (178 mmol) et on agite pendant 15 minutes à température ambiante. Le bromure de benzyle (22.5 mL ; 178 mmol) est ensuite ajouté au goutte-à-goutte puis le milieu réactionnel est chauffé au reflux pendant 4 h. Après filtration et rinçage à l'acétonitrile, le filtrat est concentré et chromatographié sur gel de silice (gradient éther de pétrole / AcOEt). Le produit du titre est alors obtenu sous la forme d'un solide incolore.

### Préparation 10": N-(4-{[tert-Butyl(diméthyl)silyl]oxy)phényl)-3-fluoro-4-[2-(morpholin-4-yl)éthoxy] aniline

On procède selon le procédé de la Préparation 3" en remplaçant le 5-bromo-1*H-*indole utilisé au Stade A par le 4-bromo-2-fluorophénol.
**RMN ¹H :** δ (400 MHz ; dmso-d6 ; 300K) :7.75 (d, 1H) ; 7 (dd, 1H); 6.9 (d, 2H) ; 6.75 (m, 3H) ; 6.7 (ddd, 1H) ; 4.05 (t, 2H) ; 3.6 (t, 4H) ; 2.65 (t, 2H) ; 2.45 (t, 4H) ; 0.95 (s, 9H) ; 0.2 (s, 6H)

### Préparation 11": N-(4-{[tert-Butyl(diméthyl)silyl]oxy}phényl)pyridin-4-amine

On procède selon le procédé de la Préparation 1" en remplaçant le 4-bromo-1-méthyl-1*H-*pyrazole utilisé au Stade B par la 4-bromopyridine.
**IR :** v -NH- : 3200 et 2500 cm⁻¹; v -Si-O-: 902 cm⁻¹ ; v -Si-C-: 820 cm⁻¹

### Préparation 12": 3-[(4-{[tert-Butyl(diméthyl)silyl]oxy}phényl)amino]benzonitrile

On procède selon le procédé de la Préparation 1" en remplaçant le 4-bromo-1-méthyl-1*H-*pyrazole utilisé au Stade B par le 3-bromobenzonitrile.

### Préparation 13": N-(4-{[tert-Butyl(diméthyl)silyl]oxy)phényl)-3-fluoroaniline

On procède selon le procédé de la Préparation 1" en remplaçant le 4-bromo-1-méthyl-1*H-*pyrazole utilisé au Stade B par le 1-bromo-3-fluorobenzène.

### Préparation 14": N-(4-{[tert-Butyl(diméthyl)silyl]oxy}phényl)-3,4-difluoroaniline

On procède selon le procédé de la Préparation 1" en remplaçant le 4-bromo-1-méthyl-1*H-*pyrazole utilisé au Stade B par le 4-bromo-1,2-difluorobenzène.

### Préparation 15": 4-{[tert-Butyl(diméthyl)silyl]oxy}-N-{4-[(3,3-difluoropipéridin-1-yl)méthyl]phényl}aniline

### Stade A : 1-(4-Bromobenzyl)-3,3-difluoropipéridine

A une solution de 4-bromobenzaldéhyde (500 mg ; 2,7 mmol) dans 12 mL de dichlorométhane sont ajoutés, dans cet ordre, le chlorhydrate de 3,3-difluoropipéridine (470 mg ; 3 mmol), le triacétoxyborohydrure de sodium (860 mg ; 4 mmol) et l'acide acétique (0.17 mL ; 3 mmol). Après 1 h d'agitation à température ambiante, le milieu réactionnel est coulé sur un mélange de bicarbonate de sodium aqueux et de dicholorométhane. La phase aqueuse est extraite au dichlorométhane. La phase organique est séchée sur MgS04, concentrée à sec et le résidu est purifié par chromatographie sur gel de silice (gradient CH₂Cl₂/MeOH) pour fournir le produit attendu sous la forme d'une huile.
**RMN ¹H** : δ (400 MHz ; dmso-d6 ; 300K) :7.55 (dd, 2H); 7.25 (dd, 2H); 3.55 (s, 2H); 2.7 (t, 2H); 2.35 (t, 2H); 1.85 (m, 2H); 1.65 (m, 2H)

### Stade B : 4-{[tert-Butyl(diméthyl)silyl]oxy}-N-{4-[(3,3-difluoropipéridin-1-yl)méthyl]phényl}aniline

On procède selon le procédé de la Préparation 1" en remplaçant le 4-bromo-1-méthyl-1*H-*pyrazole utilisé au Stade B par 1-[(4-bromophényl)méthyl]-3,3-difluoro-pipéridine.

### Préparation 16": N-(4-{[tert-Butyl(diméthyl)silyl]oxy}phényl)quinolin-6-amine

On procède selon le procédé de la Préparation 1" en remplaçant le 4-bromo-1-méthyl-1*H-*pyrazole utilisé au Stade B par la 6-bromo-quinoline.
**IR :** v -NH- : 3300 cm⁻¹

### Préparation 17": N-(4-{[tert-Butyl(diméthyl)silyl]oxy}phényl)-2-méthylpyridin-4-amine

On procède selon le procédé de la Préparation 1" en remplaçant le 4-bromo-1-méthyl-1*H-*pyrazole utilisé au Stade B par la 4-bromo-2-méthyl-pyridine.
**IR :** v -NH- : 3200 et 3100 cm⁻¹

### Préparation 18" : N-(4-{[tert-Butyl(diméthyl)silyl]oxy}phényl)-1-méthyl-1H-pyrrolo[2,3-b]pyridin-5-amine

On procède selon le procédé de la Préparation 1" en remplaçant le 4-bromo-1-méthyl-1*H-*pyrazole utilisé au Stade B par la 5-bromo-1-méthyl-1*H-*pyrrolo[2,3-*b*]pyridine (obtenue selon un protocole de la littérature : Heterocycles, 60(4), 865, 2003).
**IR :** v :-NH- : 3278 cm⁻¹; v :-C=C- aromatiques: 1605 cm⁻¹

### Préparation 19": N-(4-{[tert-Butyl(diméthyl)silyl]oxy}phényl)pyridin-3-amine

On procède selon le procédé de la Préparation 1" en remplaçant le 4-bromo-1-méthyl-1*H-*pyrazole utilisé au Stade B par la 3-bromo-pyridine.

### Préparation 20": 4-{[tert-Butyl(diméthyl)silyl]oxy}-N-{4-[(3,3-difluoropipéridin-1-yl)éthyl]phényl}aniline

### Stade A : 2-(4-Bromophényl)-1-(3,3-difluoropipéridin-1-yl)éthanone

A une solution d'acide 4-bromophénylacetique (4 g ; 18,6 mmol) et de chlorhydrate de 3,3-difluoropipéridine (2,5 g ; 20,4 mmol) dans du dichlorométhane (190 mL) sont ajoutés de l'EDC (3,8 g ; 22,3 mmol), du HOBt (3 g ; 22,3 mmol) et de la triéthylamine (1,3 mL ; 593 mmol). Le milieu réactionnel est laissé sous agitation pendant 17h à température ambiante puis coulé sur un mélange de bicarbonate de sodium aqueux et d'acétate d'éthyle. La phase aqueuse est extraite à l'acétate d'éthyle. La phase organique est lavée avec de l'acide chlorhydrique 0,1 N, de l'eau, et de la saumure avant d'être séchée sur MgSO₄ concentrée à sec. Le résidu est purifié par chromatographie sur gel de silice (gradient éther de pétrole/acétate d'éthyle) pour fournir le produit attendu sous la forme d'un solide.
**RMN ¹H :** δ (400 MHz ; dmso-d6 ; 300K) :7.5 (d, 2H) ; 7.2 (d, 2H) ; 3.8 (t, 2H) ; 3.7 (s, 3H) ; 3.5 (t, 2H) ; 2 (m, 2H) ; 1.6 (m, 2H)

### Stade B : 1-[2-(4-Bromophényl)éthyl]-3,3-difluoropipéridine

A une solution du composé du Stade A (4,6 g ; 14,5 mmol) dans du THF anhydre (145 mL) est ajoutée une solution 1M de boranesulfure de diméthyle dans le THF (14.5 mL ; 14,5 mmol). Le milieu réactionnel est chauffé à 80°C pendant 3h, puis le solvant est évaporé sous pression réduite. Le résidu est traité par du méthanol (50 mL), puis par du HCl 5N (5,8 mL). Après une nuit d'agitation à température ambiante et 3h au reflux, le pH du milieu réactionnel est ajusté à 8 par une solution saturée de bicarbonate de sodium aqueux, puis la phase aqueuse est extraite au dichlorométhane. La phase organique est séchée sur MgS04, concentrée à sec et le résidu est purifié par chromatographie sur gel de silice (gradient CH₂Cl₂/MeOH) pour fournir le produit attendu sous la forme d'une huile.
**RMN ¹H :** δ (400 MHz ; dmso-d6 ; 300K) :7.45 (d, 2H); 7.20 (d, 2H); 2.71 (m, 2H); 2.69 (t, 2H); 2.58 (dd, 2H); 2.45 (dd, 2H); 1.86 (m, 2H); 1.63 (m, 2H)

### Stade C: 4-{[tert-Butyl(diméthyl)silyl]oxy}-N-{4-[(3,3-difluoropipéridin-1-yl)éthyl]phényl}aniline

On procède selon le procédé de la Préparation 1" en remplaçant le 4-bromo-1-méthyl-1*H-*pyrazole utilisé au Stade B par le composé du Stade B.
**RMN ¹H :** δ (400 MHz ; dmso-d6 ; 300K) :7.7 (s, 1H) ; 7.45 (d, 2H) ; 7.39 (t, 2H) ; 7.31 (t, 1H) ; 7.0 (m, 4H) ; 6.9 (d, 2H) ; 6.81 (d, 2H) ; 5.05 (s, 2H) ; 2.7 (t, 2H) ; 2.6 (t, 2H) ; 2.5 (t, 2H) ; 2.45 (t, 2H) ; 1.89 (m, 2H) ; 1.68 (m, 2H)

### Préparation 21": 4-{[tert-Butyl(diméthyl)silyl]oxy}-N-{4-[2-(3,3-difluoropyrrolidin-1-yl)éthyl]phényl}aniline

On procède selon le procédé de la Préparation 19" en remplaçant au Stade A le chlorhydrate de 3,3-difluoropipéridine par le chlorhydrate de 3,3-difluoropyrrolidine.
**RMN ¹H :** δ (400 MHz ; dmso-d6 ; 300K) :7.7 (s, 1H) ; 7.45 (d, 2H) ; 7.35 (t, 2H) ; 7.34 (t, 1H) ; 7.05-6.85 (m, 8H) ; 5.05 (s, 2H); 2.9 (t, 2H); 2.75-2.25 (m, 8H)

### Préparation 22": N-(4-{[tert-Butyl(diméthyl)silyl]oxy}phényl)-2,6-diméthylpyridin-4-amine

On procède selon le procédé de la Préparation 1" en remplaçant le 4-bromo-1-méthyl-1*H-*pyrazole utilisé au Stade B par la 4-bromo-2,6-diméthylpyridine.
**IR :** v : -NH- : 3300 et 2700 cm⁻¹; v :-Si-O-: 900 cm⁻¹; v : -Si-C-: 823 cm⁻¹

### Préparation 23": N-(4-{[tert-Butyl(diméthyl)silyl]oxy}phényl)-1-[2-(morpholin-4-yl)éthyl]-1H-pyrazol-4-amine

On procède selon le procédé de la Préparation 2" en remplaçant le 5-bromobindole utilisé au Stade A par le 4-bromo-1*H-*pyrazole.
**RMN ¹H :** δ (400 MHz ; dmso-d6 ; 300K) : 7.61 (s, 1H); 7.25 (s, 1H); 7.18 (s, 1H); 6.65 (m, 4H); 4.15 (t, 2H); 3.55 (t, 4H); 2.7 (t, 2H); 2.4 (t, 4H); 0.95 (s, 9H); 0.15 (s, 6h)

### Préparation 24": N-(4-{[tert-Butyl(diméthyl)silyl]oxy}phényl)-3-fluoropyridin-4-amine

On procède selon le procédé de la Préparation 1 " en remplaçant le 4-bromo-1-méthyl-1*H-*pyrazole utilisé au Stade B par la 4-bromo-3-fluoro-pyridine.
**IR :** v -NH- : 3200 et 3000 cm⁻¹; v -Si-O-: 900 cm⁻¹ ; v -Si-C-: 820 cm⁻¹

### Préparation 25": N-(4-{[tert-Butyl(diméthyl)silyl]oxy}phényl)imidazo[1,2-a] pyridin-7-amine

On procède selon le procédé de la Préparation 1 " en remplaçant le 4-bromo-1-méthyl-1*H-*pyrazole utilisé au Stade B par la 7-bromoimidazo[1,2-*a*]pyridine (préparée à partir de la 4-bromopyridin-2-amine selon un protocole de la littérature :WO 2008124323 A1).
**IR :** v -NH- : 3300-3000 cm⁻¹; v -C=N-: 1652 cm⁻¹ ; v -C=C-: 1610 cm⁻¹; v -Si-C-: 1236 cm⁻¹; v -Si-O-: 898 cm⁻¹ ; v -Si-C-: 828, 774 cm⁻¹

### Préparation 26": N-(4-{[tert-Butyl(diméthyl)silyl]oxy}phényl)-2-méthyl imidazo[1,2-a]pyridin-7-amine

On procède selon le procédé de la Préparation 1" en remplaçant le 4-bromo-1-méthyl-1*H-*pyrazole utilisé au Stade B par la 7-bromo-2-méthyl-imidazo[1,2-*a*]pyridine (préparée à partir de la 4-bromopyridin-2-amine selon un protocole de la littérature :A. J. Helliot et al J. Heterocyclic Chemistry 19, 1437, 1982).
**IR :** v -NH- : 3300-3000 cm⁻¹ ; v -C=N-: 1652 cm⁻¹; v -C=C-: 1610 cm⁻¹; v -Si-C-: 1236 cm⁻¹, v -Si-O-: 898 cm⁻¹ ; v -Si-C-: 828, 774 cm⁻¹

### Préparation 27": N-(4-{[tert-Butyl(diméthyl)silyl]oxy}phényl)-6-méthylpyridin-3-amine

On procède selon le procédé de la Préparation 1 " en remplaçant le 4-bromo-1-méthyl-1*H-*pyrazole utilisé au Stade B par la 3-bromo-6-méthyl-pyridine.
**IR :** v -NH- : 3251 cm⁻¹ ; v -C=C- aromatiques: 1605 cm⁻¹

### Préparation 28": N-(4-{[tert-Butyl(diméthyl)silyl]oxy}phényl)-5-fluoropyridin-3-amine

On procède selon le procédé de la Préparation 1" en remplaçant le 4-bromo-1-méthyl-1*H-*pyrazole utilisé au Stade B par la 3-bromo-5-fluoro-pyridine.
**IR :** v -NH- : 3400-3000 cm⁻¹ ; v -C-F-: 1245 cm⁻¹

### Préparation 29": N-(4-{[tert-Butyl(diméthyl)silyl]oxy}phényl)-2-méthoxypyridin-4-amine

On procède selon le procédé de la Préparation 1" en remplaçant le 4-bromo-1-méthyl-1*H-*pyrazole utilisé au Stade B par la 4-bromo-2-méthoxy-pyridine.
**IR :** v -NH- : 3200 et 3000 cm⁻¹ ; v -C=C aromatiques-: 1618, 1601 cm⁻¹

### Préparation 30": N-(4-{[tert-Butyl(diméthyl)silyl]oxy}phényl)-2-(propan-2-yl)pyridin-4-amine

On procède selon le procédé de la Préparation 1" en remplaçant le 4-bromo-1-méthyl-1*H-*pyrazole utilisé au Stade B par la 4-bromo-2-(propan-2-yl)pyridine.
**IR :** v -NH- : 3300 et 3100 cm⁻¹

### Préparation 31": N-(4-{[tert-Butyl(diméthyl)silyl]oxy}phényl)pyrazolo[1,5-a]pyrimidin-6-amine

On procède selon le procédé de la Préparation 1" en remplaçant le 4-bromo-1-méthyl-1*H-*pyrazole utilisé au Stade B par la 6-bromopyrazolo[1,5-*a*]pyrimidine.
**IR :** v -NH- : 3272 cm⁻¹; v -C=N-: 1634 cm⁻¹; v -C=C-: 1616 cm⁻¹

### Préparation 32": N-(4-{[tert-Butyl(diméthyl)silyl]oxy}phényl)-3,3a-dihydro[1,2,4]triazolo[1,5-a]pyrimidin-6-amine

On procède selon le procédé de la Préparation 1" en remplaçant le 4-bromo-1-méthyl-1*H-*pyrazole utilisé au Stade B par la 6-bromo-3,3a-dihydro[1,2,4]triazolo[1,5-*a*]pyrimidine préparée selon la littérature (WO 2011015343) à partir de 4*H*-1,2,4-triazol-3-amine et de 2-bromopropanedial.
**IR :** v -NH- : 3244 cm⁻¹

### Préparation 33": N-(4-{[tert-Butyl(diméthyl)silyl]oxy}phényl)pyridin-4-amine 1-oxyde

On procède selon le procédé de la Préparation 1" en remplaçant le 4-bromo-1-méthyl-1*H-*pyrazole utilisé au Stade B par la 4-bromopyridine 1-oxyde préparée selon la littérature (WO 2009117269) à partir de la 4-bromopyridine.
**IR :** v -NH- : 3246 cm⁻¹; v -C=C- aromatiques : 1618 cm⁻¹
***Spectre de Masse :***
Formule brute : C₁₇H₂₄N₂O₂Si
[M]⁺. mesuré m/z: 316
[M-O]⁺. mesuré m/z: 300
[M-C₄H_{9]}⁺. mesuré m/z: 259

### Préparation 34": Chlorure de N-[4-[tert-butyl(diméthyl)silyl]oxyphényl]-1-méthyl-pyridin-1-ium-4-amine

On procède selon le procédé de la Préparation 1" en remplaçant le 4-Bromo-1-méthyl-1*H-*pyrazole utilisé au Stade B par le chlorure de 4-bromo-1-méthyl-pyridin-1-ium préparé selon la littérature à partir de la 4-bromopyridine.

### Préparation 35": N-[4-[tert-butyl(diméthyl)silyl]oxyphényl]-1-méthyl-pyrazolo[3,4-b]pyridin-5-amine

On procède selon le procédé de la Préparation 1" en remplaçant le 4-bromo-1-méthyl-1*H-*pyrazole utilisé au Stade B par le 5-bromo-1-méthyl-pyrazolo[3,4-*b*]pyridine préparé selon la littérature (WO 2006052568).
**RMN ¹H** (400 MHz, dmso-d6) δ ppm: 8.33 (d, 1 H), 7.94 (sl, 1 H), 7.92 (s, 1 H), 7.71 (d, 1 H), 6.95 (d, 2 H), 6.76 (d, 2 H), 4.01 (s, 3 H), 0.95 (s, 9 H), 0.17 (s, 6 H)
***IR (ATR) cm⁻¹:*** 3290 v >OH ; 1503 v Ar ; 1249 γ -Si-CH₃

### Préparation 36": N-[4-[tert-butyl(diméthyl)silyl]oxyphényl]-3-méthyl-pyrazolo[1,5-a]pyrimidin-6-amine

On procède selon le procédé de la Préparation 1" en remplaçant le 4-bromo-1-méthyl-1*H-*pyrazole utilisé au Stade B par le 6-bromo-3-méthyl-pyrazolo[1,5-*a*]pyrimidine préparé selon la littérature (WO 2011015343 et WO2011049917).
**RMN ¹H** (400 MHz, dmso-d6) δ ppm: 8.49 (d, 1 H), 8.4 (d, 1 H), 7.98 (m, 1 H), 7.87 (s, 1 H), 7 (d, 2 H), 6.81 (d, 2 H), 2.29 (s, 3 H), 0.98 (s, 9 H), 0.2 (s, 6 H)
***IR (ATR) cm⁻¹*** : 3257 v >NH

Les amines NHR₃R₄ dans lesquelles R₃ et R₄ représentent indépendamment l'une de l'autre un groupement aryle ou hétéroaryle sont obtenues selon les procédés décrits dans la littérature (Surry D.S. et al., Chemical Science, 2011, 2, 27-50, Charles M.D. et al., Organic Letters, 2005, 7, 3965-3968). La réaction de protection de la fonction hydroxy du 4-anilinophénol décrite à la Préparation 8" peut être appliquée à diverses amines secondaires NHR₃R₄ (telles que définies précédemment), comportant une ou plusieurs fonctions hydroxy, lorsque celles-ci sont disponibles commercialement. Alternativement, les amines secondaires comportant au moins un substituant hydroxy peuvent être synthétisées directement sous une forme protégée, i.e. à partir de réactifs dont la fonction hydroxy a été préalablement protégée. Parmi les groupements protecteurs, le *tert-*butyl(diméthyl)silyloxy et le benzyloxy sont particulièrement préférés.

Parmi les amines NHR₃R₄ comportant un substituant hydroxy qui sont utilisées pour synthétiser les composés de l'invention, on peut citer : le 4-(4-toluidino)phénol, le 4-(4-chloroanilino)phénol, le 4-(3-fluoro-4-méthylanilino)phénol, le 4-[4-(trifluorométhoxy)anilino]phénol, le 4-[4-hydroxyanilino]phénol, le {4-[(1-méthyl-1*H-*indol-6-yl)amino]phényl}méthanol, le 4-(2,3-dihydro-1*H-*indol-6-ylamino)phénol, le 4-[(1-méthyl-2,3-dihydro-1*H-*indol-6-yl)amino]phénol, 4-[(1-méthyl-1*H-*indol-6-yl)amino]phénol, le 4-[(1-méthyl-1*H-*indol-6-yl)amino]cyclohexanol, le 4-[(1-méthyl-1,2,3,4-tétrahydro-6-quinolinyl)amino]phénol, le 4-[(4-méthyl-3,4-dihydro-2*H*-1,4-benzoxazin-7-yl)amino]phénol, le 4-[4-(diéthylamino)anilino]phénol, le 4-(2,3-dihydro-1*H*-indén-5-ylamino)phénol, le 4-[(1-méthyl-1*H-*indazol-5-yl)amino]phénol, le 4-[(1'-méthyl-1',2'-dihydrospiro[cyclopropane-1,3'-indol]-5'-yl)amino]phénol, le 4-[(1,3,3-triméthyl-2,3-dihydro-1*H-*indol-5-yl) amino] phénol, le 4-[4-méthoxy-3-(trifluorométhyl)anilino]phénol, le 4-[4-(méthylsulfanyl)-3-(trifluorométhyl)anilino]phénol, le 2-fluoro-4-[(1-méthyl-1H-indol-5-yl)amino]phénol, le 4-[(1-éthyl-1*H-*indol-5-yl)amino]phénol, le 4-[(1-éthyl-2,3-dihydro-1*H*-indol-5-yl)amino]phénol, le 4-[(1-isopropyl-2,3-dihydro-1*H*-indol-5-yl)amino]phénol, le 4-(butylamino)phénol, le 3-[(1-méthyl-1*H*-indol-5-yl)amino]-1-propanol, le 4-[(1-méthyl-1*H-*indol-5-yl)amino]-1-butanol, le 4-[(3-fluoro-4-méthylphényl)amino]phénol, le 4-[(3-chloro-4-méthylphényl)amino]phénol, le 4-[(4-fluorophényl)amino]phénol, le 4-[(1-méthyl-1*H-*pyrrolo[2,3-*b*]pyridin-5-yl)amino]phénol, le 4-[(4-fluorophényl)amino]phénol, 4-[(2-fluorophényl)amino]phénol, le 4-[(3-fluorophényl)amino]phénol, le 4-[(2,4-difluorophényl)amino]phénol, le 4-[(3,4-difluorophényl)amino]phénol, le 3-[(4-hydroxyphényl)amino]benzonitrile, le 4-[(3-méthoxyphényl)amino]phénol, le 4-[(3,5-difluorophényl)amino]phénol, le 4-[(3-méthylphényl)amino]phénol, le 4-[(4-hydroxy phényl)amino]benzonitrile, le 4-[(3-chlorophényl)amino]phénol, le 4-(pyrimidin-2-ylamino)phénol, le 4-[(cyclobutylméthyl)amino]phénol, le 2-[(4-hydroxyphényl)amino]benzonitrile, le 4-{[(1-méthyl-1*H-*pyrazol-4-yl)méthyl]amino} phénol, le 4-[(cyclopropylméthyl)amino]phénol, le 4-{[(1-méthyl-1*H-*pyrazol-3-yl)méthyl]amino}phénol, le 4-(but-2-yn-1-ylamino)phénol, le 4-(pyrazin-2-ylamino) phénol, le 4-(pyridin-2-ylamino)phénol, le 4-(pyridazin-3-ylamino)phénol, le 4-(pyrimidin-5-ylamino)phénol, le 4-(pyridin-3-ylamino)phénol, le 4-[(3,5-difluoro-4-méthoxyphényl)amino]phénol, le 4-(pyridin-4-ylamino)phénol, le 4-[(3-fluoro-4-méthoxy phényl)amino]phénol, le 2-(phénylamino)pyrimidin-5-ol, le 5-[(4-hydroxyphényl)amino]-2-méthoxybenzonitrile, le 4-{[3-(trifluorométhyl)phényl]amino}phénol, le 4-(méthylamino)phénol, le 4-(éthylamino)phénol et le 4-(propan-2-ylamino)phénol.

La ou les fonction(s) hydroxy des amines secondaires listées ci-dessus est (sont) préalablement protégée(s) par un groupement protecteur adapté avant tout couplage à un dérivé d'acide du composé de formule (VII) tel que défini dans le procédé général précédent.

### Exemple 1. N-(4-Hydroxyphényl)-3-(6-{[(3R)-3-méthyl-3,4-dihydroisoquinolin-2(1H)-yl]carbonyl}-1,3-benzodioxol-5-yl)-N-(1-méthyl-1H-indol-5-yl)-5,6,7,8-tétrahydroindolizine-1-carboxamide

### Stade A : 3-(6-{[(3R)-3-Méthyl-3,4-dihydroisoquinolin-2(1H)-yl]carbonyl}-1,3-benzodioxol-5-yl)-5,6,7,8-tétrahydroindolizine-1-carboxylate de méthyle

A une solution de 2 g du composé de la Préparation 1 dans 20 mL de dichlorométhane, on ajoute à température ambiante 5,5 mL de *N,N,N* triéthylamine (6,96 mmol), le composé de la Préparation l' (6,96 mmol), puis 0,94 g d'hydroxybenzotriazole (HOBT) et 1,34 g d'1-éthyl-3-(3'-diméthylaminopropyl)-carbodiimide (EDC) (6,96 mmol). Le milieu réactionnel est ensuite agité à température ambiante pendant 1 nuit, puis il est versé sur une solution de chlorure d'ammonium et extrait avec de l'acétate d'éthyle. La phase organique est ensuite séchée sur sulfate de magnésium, puis filtrée et évaporée à sec. Le produit brut ainsi obtenu est alors purifié par chromatographie sur gel de silice (gradient heptane/ AcOEt) pour conduire au produit attendu.
**RMN¹H :** δ (400 MHz; dmso-d6; 300K): 7.2-6.8 (m, 4H, H aromatiques, H tétrahydroisoquinoline) ; 7.10 (s, 1H, H aromatique, benzodioxole) ; 6.92 (s, 1H, H aromatique, benzodioxole) ; 6.25 (m, 1H, H tétrahydroindolizine); 6.10 (s, 2H, H aliphatiques, O**CH₂**O) ; 4.80 (m, 1H, H aliphatique, H tétrahydroisoquinoline) ; 4.20 (m, 1H, H aliphatique, H tétrahydroisoquinoline) ; 4.1-3.5 (m, 3H) ; 3.60 (s, 3H, COO**CH₃) ;** 2.90 (m, 2H, H aliphatiques, H tétrahydroindolizine); 2.45 (m, 2H, H aliphatiques, H tétrahydroisoquinoline) ; 1.70 (m, 4H, H aliphatiques, H tétrahydroindolizine); 0.80 (m, 3H, H aliphatiques, **CH₃**-THIQ).
**IR :** v : >C=O 1694 cm⁻¹ (ester conjugué) ; v : >C=O 1624 cm⁻¹ (amide) ; v : >C-Ar 772-742 cm⁻¹

### Stade B: 3-(6-{[(3R)-3-Méthyl-3,4-dihydroisoquinolin-2(1H)-yl]carbonyl}-1,3-benzodioxol-5-yl)-5,6,7,8-tétrahydroindolizine-1-carboxylate de lithium

A une solution contenant 8,26 mmol du composé du Stade A dans 24 mL de dioxane est ajoutée une solution d'hydroxyde de lithium (675 mg, 16,1 mmol). L'ensemble est placé dans un four à micro-ondes à 140 W, 100°C pour une durée de 2h30. Le milieu réactionnel est ensuite filtré et évaporé. Le solide ainsi obtenu est séché à 40°C dans une étuve en présence de P₂O₅**.**

### Stade C: N-(4-{[tert-Butyl(diméthyl)silyl]oxy}phényl)-3-(6-{[(3R)-3-méthyl-3,4-dihydroisoquinolin-2(1H)-yl}carbonyl}-1,3-benzodioxol-5-yl)-N-(1-métltyl-1H-indol-5-yl)-5,6,7,8-tétrahydroindolizine-1-carboxamide

A une solution contenant 4,73 mmol du composé du Stade B dans 47 mL de dichlorométhane sont ajoutés, au goutte à goutte, 1,2 mL de chlorure d'oxalyle à 0°C. Le milieu réactionnel est agité à température ambiante pendant 11 heures, puis co-évaporé le plusieurs fois avec du dichlorométhane. Le produit ainsi obtenu est mis en suspension dans 37 mL de dichlorométhane, puis est additionné sur une solution contenant 7,1 mmol du composé obtenu à la Préparation 2" dans 10 mL de dichlorométhane en présence de 0,6 mL de pyridine (7,1 mmol). L'ensemble est agité à température ambiante pendant une nuit. Le milieu réactionnel est concentré, purifié par chromatographie sur gel de silice (gradient dichlorométhane/méthanol) pour conduire au produit attendu.

### Stade D: N-(4-Hydroxyphényl)-3-(6-{[(3R)-3-méthyl-3,4-dihydroisoquinolin-2(1H)-yl]carbonyl}-1,3-benzodioxol-5-yl)-N-(1-méthyl-1H-indol-5-yl)-5,6,7,8-tétrahydroindolizine-1-carboxamide

A une solution contenant 2,3 mmol du composé obtenu au Stade C dans 4 mL de méthanol, on ajoute 0,646 g (11,5 mmol) d'hydroxyde de potassium solubilisé dans 8 mL de méthanol. L'ensemble est agité à température ambiante pendant 30 min. Le milieu réactionnel est ensuite dilué dans le dichlorométhane et lavé successivement avec une solution HCl 1N, une solution de NaHCO₃ saturée, puis une solution de NaCl saturée jusqu'à atteindre un pH neutre. La phase organique est ensuite séchée sur sulfate de magnésium, filtrée et évaporée. Le produit brut ainsi obtenu est purifié sur gel de silice (gradient dichlorométhane/méthanol), puis lyophilisé pour fournir le produit attendu.
***Masse haute résolution*** (***ESI***+) ***:***
Formule brute : C₄₂H₃₈CN₄O₅
[M+H]⁺ calculé : 679.2920
[M+H]⁺ mesuré : 679.2908

### Exemple 2. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-{1-[2-(morpholin-4-yl)éthyl]-1H-indol-5-yl}-5,6,7,8-tétrahydroindolizine-1-carboxamide

On procède selon les procédés décrits aux Stades A-D de l'Exemple 1 en utilisant les réactifs appropriés. Après l'étape de purification sur gel de silice (*cf.* Stade D), le solide est ensuite solubilisé dans le dichlorométhane et 2 mL d'éther chlorhydrique 1N sont ajoutés. Le tout est agité pendant 1 heure, puis évaporé à sec. Le chlorhydrate ainsi obtenu est dissout dans un mélange eau / acétonitrile jusqu'à solubilisation totale, puis est lyophilisé.
***Microanalyse élémentaire : (% théorique: mesuré**)*
%C=69.32:68.93; %H=5.94:5.74; %N=8.6:8.51; %Cl-=4.35:4.6

**Sauf mention contraire, les composés des Exemples suivants sont synthétisés selon le procédé de l'Exemple 1 en utilisant au Stade A : (i) l'acide approprié obtenu selon l'une des Préparations 1 à 18 et (ii) le dérivé de tétrahydroisoquinoline approprié obtenu selon l'une des Préparations 1' à 7', ainsi qu'au Stade C : (iii) l'amine NHR₃R₄ adéquate (une liste non exhaustive est proposée aux Préparations 1" à 36").**

### Exemple 3. Chlorhydrate de 6-(5-chloro-2-{[(3R)-3-méthyl-3,4-dihydroisoquinolin-2(1H)-yl]carbonyl}phényl)-N-(4-hydroxyphényl)-N-(1-méthyl-1H-indol-5-yl)-1,2,3,4-tétrahydropyrrolo[1,2-a]pyrazine-8-carboxamide

On procède selon le procédé de l'Exemple 1 en remplaçant d'une part le composé de la Préparation 1 utilisé au Stade A par le composé de la Préparation 2, et d'autre part le composé de la Préparation 1" utilisé au Stade C par la *N-*(*4-*{[*tert-*butyl(diméthyl)silyl]oxy}phényl)-1-méthyl-1*H-*indol-5-amine, étant entendu que le produit ainsi obtenu n'est pas soumis à une étape de salification en présence d'éther chlorhydrique comme cela est décrit au Stade D de l'Exemple 1. Le composé ainsi obtenu est déprotégé en présence de 10 équivalents d'acide trifluoroacétique dans le dichlorométhane (10 mL/mmol) à température ambiante pendant une nuit. Puis, le produit est ensuite isolé par concentration à sec du milieu réactionnel. Il est enfin soumis à une étape de salification en présence d'éther chlorhydrique.
***Microanalyse élémentaire : (% théorique : mesuré)***
%C=67.99:65.52; %H=5.28:4.49; %N=9.91:9.24; %Cl=10.03:9.95; %Cl-=5.02:5.45
***Masse haute résolution (ESI+) :***
Formule brute : C₄₀H₃₆ClN₅O₃
[M+H]⁺ calculé : 670.2585
[M+H]⁺ mesuré : 670.2587

### Exemple 4. 3-[5-Chloro-2-(3,4-dihydroisoquinolin-2(1H)-ylcarbonyl)phényl]-N-(4-hydroxyphényl)-N-{1-[2-(morpholin-4-yl)éthyl]-1H-indol-5-yl}-5,6,7,8-tétrahydroindolizine-1-carboxamide

***Microanalyse élémentaire :* %** ***mesuré** (**théorique**)*
%C=70.85(71.65);%H=5.39(5.88);%N=9.11(9.28);%Cl=4.48(4.7)

### Exemple 5. 3-[5-Chloro-2-(3,4-dihydroisoquinolin-2(1H)-ylcarbonyl)phényl]-N-(4-hydroxyphényl)-N-{1-[2-(morpholin-4-yl)éthyl]-2,3-dihydro-1H-indol-5-yl}-5,6,7,8-tétrahydroindolizine-1-carboxamide

### Stade A : N-(4-{[tert-Butyl(diméthyl)silyl]oxy}phényl)-3-[5-chloro-2-(3,4-dihydroisoquinolin-2(1H)-ylcarbonyl)phényl]-N-{1-[2-(morpholin-4-yl)éthyl]-2,3-dihydro-1H-indol-5-yl}-5,6,7,8-tétrahydroindolizine-1-carboxamide

On procède selon les protocoles décrits aux Stades A-C de l'Exemple 1 en utilisant le composé de la Préparation 3 et la 1,2,3,4-tétrahydroisoquinoline au Stade A, ainsi que le composé de la Préparation 4" au Stade C.

### Stade B : 3-[5-Chloro-2-(3,4-dihydroisoquinolin-2(1H)-ylcarbonyl)phényl]-N-(4-hydroxyphényl)-N-{1-[2-(morpholin-4-yl)éthyl]-2,3-dihydro-1H-indol-5-yl}-5,6,7,8-tétrahydroindolizine-1-carboxamide

A une solution de 1,3 g (1,45 mmol) du composé du Stade A dans 13 mL d'acide acétique est ajouté à température ambiante le cyanoborohydrure de sodium (900 mg ; 15 mmol). Après 2h d'agitation, le milieu réactionnel est concentré à sec, puis dilué au méthanol (8 mL) et traité par une solution molaire de potasse dans le méthanol (6,3 mL ; 6,3 mmol). Après 1h à température ambiante, le milieu réactionnel est concentré à sec, puis chromatographié sur gel de silice (gradient dichlorométhane/ méthanol), puis lyophilisé pour fournir le produit attendu sous la forme d'une poudre.
***Microanalyse élémentaire : % mesuré (théorique)***
%C=70.74(71.46);%H=5.74(6.13);%N=9(9.26);%Cl=4.46(4.69)

### Exemple 6. Chlorhydrate de N-(4-hydroxyphényl)-2-méthyl-6-(7-{[(3R)-3-méthyl-3,4-dihydroisoquinolin-2(1H)-yl]carbonyl}-2,3-dihydro-1,4-benzodioxin-6-yl)-N-(1-méthyl-1H-indol-5-yl)-1,2,3,4-tétrahydropyrrolo[1,2-a]pyrazine-8-carboxamide

Le procédé est analogue à celui décrit pour l'Exemple 7 en substituant au Stade A le composé de la Préparation 2 par le composé de la Préparation 4.
***Microanalyse élémentaire : (% théorique : mesuré)***
%C=69.39:69.13; %H=5.69:4.98; %N=9.41:9.37; %Cl-=4.76:4.65

### Exemple 7. 6-(5-Chloro-2-{[(3R)-3-méthyl-3,4-dihydroisoquinolin-2(1H)-yl]carbonyl}phényl)-N-(4-hydroxyphényl)-2-méthyl-N-(1-méthyl-1H-indol-5-yl)-1,2,3,4-tétrahydropyrrolo [1,2-a]pyrazine-8-carboxamide

### Stade A : 8-[(4-{[tert-Butyl(diméthyl)silyl]oxy}phényl)(1-méthyl-1H-indol-5-yl)carbamoyl]-6-(5-chloro-2-{[(3R)-3-méthyl-3,4-dihydroisoquinolin-2(1H)]yl]carbonyl} phényl)-3,4-dihydydropyrrolo[1,2-a]pyrazine-2(1H)-carboxylate de tert-butyle

On procède selon les protocoles décrits aux Stades A-C de l'Exemple 1 en utilisant les composés des Préparations 2 et l'au Stade A, ainsi que le composé de la Préparation 2" au Stade C.

### Stade B : 6-(5-Chloro-2-{[(3R)-3-méthyl-3,4-dihydroisoquinolin-2(1H)-yl]carbonyl}phényl)-8-[(4-hydroxyphényl)(1-méthyl-1H-indol-5-yl)carbamoyl]-3,4-dihydropyrrolo[1,2-a]pyrazine-2(1H)-carboxylate de tert-butyle

A une solution du composé du Stade A (1,1 g ; 1,25 mmol) dans du méthanol (6 mL) est ajoutée une solution 1M de potasse dans le méthanol (6,2 mL ; 6,2 mmol). Après 2 h à température ambiante, le méthanol est évaporé sous vide et le résidu est repris dans un mélange constitué de dichlorométhane et d'une solution saturée de bicarbonate de sodium. Les phases organiques réunies sont séchées au MgSO₄ et concentrées à sec. Le résidu obtenu est purifié par chromatographie sur gel de silice (gradient CH₂Cl₂/MeOH) pour fournir le produit attendu sous la forme d'un solide.
**IR :** v : NH : 3450 cm⁻¹ ; v : CO : 1745-1620 cm⁻¹

### Stade C : 6-(5-Chloro-2-{[(3R)-3-méthyl-3,4-dihydroisoquinolin-2(1H)-yl]carbonyl}phényl)-8-[{4-[(2,2-diméthylpropanoyl)oxy]phényl}(1-méthyl-1H-indol-5-yl)carbamoyl]-3,4-dihydropyrrolo[1,2-a]pyrazine-2(1H)-carboxylate de tert-butyle

A une solution du composé du Stade B (0,7 g ; 0,93 mmol) dans du dichlorométhane (7 mL) sont ajoutés, à température ambiante, de la triéthylamine (0,2 mL ; 1,39 mmol) puis du chlorure de pivaloyle (0,11 mL ; 0,93 mmol). Après 2 heures d'agitation à température ambiante, le milieu réactionnel est lavé à l'eau, à la saumure, séché sur MgSO₄ et concentré à sec. Le résidu obtenu est utilisé tel quel pour l'étape suivante sans analyse.

### Stade D : 4-[{[6-(5-Chloro-2-{[(3R)-3-méthyl-3,4-dihydroisoquinolin-2(1H)-yl]carbonyl}phényl)-1,2,3,4-tétrahydropyrrolo[1,2-a]pyrazin-8-yl]carbonyl}(1-méthyl-1H-indol-5-yl)amino]phényl propanoate de 2,2-diméthyle

A une solution du composé du stade précédent (0,82 g ; 0,93 mmol) dans du dichlorométhane (9 mL) est ajouté à 0°C de d'acide trifluoroacétique (0,7 mL ; 13,9 mmol) au goutte à goutte. Après 15h d'agitation à température ambiante, une solution saturée de bicarbonate de sodium est lentement ajoutée au milieu réactionnel, puis les phases sont séparées. La phase aqueuse est extraite au dichlorométhane. Les phases organiques réunies sont séchées sur MgSO₄ et concentrées à sec. Le résidu obtenu est purifié par chromatographie sur gel de silice (gradient CH₂Cl₂/MeOH) pour fournir le produit attendu sous la forme d'un solide.
**LC/MS:** m/z = [M+H]⁺= 754.30

### Stade E : 4-[{[6-(5-chloro-2-{[(3R)-3-méthyl-3,4-dihydroisoquinolin-2(1H)-yl]carbonyl}phényl)-2-méthyl-1,2,3,4-tétrahydropyrrolo[1,2-a]pyrazin-8-yl]carbonyl}(1-méthyl-1H-indol-5-yl)amino}phényl propanoate de 2,2-diméthyle

A une solution du composé du stade précédent (0,41 g ; 0,54 mmol) dans du dichlorométhane (2 mL) sont ajoutés à température ambiante du formaldéhyde (48 µL ; 1,74 mmol), puis du triacétoxyborohydrure de sodium (161 mg; 0,76 mmol). Après 2h d'agitation à température ambiante, le milieu réactionnel est dilué dans du dichlorométhane, puis lavé par une solution saturée de bicarbonate de sodium. La phase organique est séchée au MgSO₄ et concentrée à sec. Le résidu obtenu est purifié par chromatographie sur gel de silice (gradient CH₂Cl₂/MeOH). On obtient le produit attendu sous la forme d'un solide.
**LC/MS:** m/z = [M+H]⁺= 768.32

### Stade F : 6-(5-Chloro-2-{[(3R)-3-méthyl-3,4-dihydroisoquinolin-2(1H)-yl]carbonyl}phéhyl)-N-(4-hydroxyphényl)-2-méthyl-N-(1-méthyl-1H-indol-5-yl)-1,2,3,4-tétrahydropyrrolo[1,2-a]pyrazine-8-carboxamide

A une solution du composé du stade précédent (0,25 g ; 0,32 mmol) dans du dioxane (1 mL) est ajouté une solution d'hydroxyde de lithium (27 mg ; 0,65 mmol) dans l'eau (1 mL). Après 5h d'agitation à température ambiante, le milieu réactionnel est concentré et dilué par une solution saturée de bicarbonate de sodium. La phase aqueuse est extraite au CH₂Cl₂. La phase organique est séchée sur MgSO₄ et concentrée à sec.Le résidu obtenu est purifié par chromatographie sur gel de silice (gradient CH₂Cl₂/MeOH). On obtient alors le produit attendu sous la forme d'un solide.
***Microanalyse élémentaire** :* **(*% théorique : mesuré***)
%C=71.97:71.51; %H=5.6:5.25; %N=10.24:10.12

### Exemple 8. Chlorhydrate de 3-(5-chloro-2-{[(3R)-3-méthyl-3,4-dihydroisoquinolin-2(1H)-yl]carbonyl}phenyl)-N-(4-hydroxyphényl)-N-{1-[2-(morpholin-4-yl)éthyl]-1H-indol-5-yl}indolizine-1-carboxamide

***Microanalyse élémentaire : (% théorique : mesuré)***
%C=69:69.16; %H=5.41:4.82; %N=8.75:8.69; %Cl-=4.43:4.13

### Exemple 9. Chlorhydrate de 6-(5-fluoro-2-{[(3R)-3-méthyl-3,4-dihydroisoquinolin-2(1H-yl]carbonyl)phényl)-N-(4-fluorophényl)-N-(4-hydroxyphényl)-1,2,3,4-tétrahydropyrrolo[1,2-a]pyrazine-8-carboxamide

On procède selon le procédé de l'Exemple 1 en remplaçant d'une part le composé de la Préparation 1 utilisé au Stade A par le composé de la Préparation 6, et d'autre part le composé de la Préparation 1 " utilisé au Stade C par le composé de la Préparation 5", étant entendu que le produit ainsi obtenu n'est pas soumis à une étape de salification en présence d'éther chlorhydrique comme cela est décrit au Stade D de l'Exemple 1. Le composé ainsi obtenu est déprotégé en présence de 10 équivalents d'acide trifluoroacétique dans le dichlorométhane (10 mL/mmol) à température ambiante pendant une nuit. Puis, le produit est ensuite isolé par concentration à sec du milieu réactionnel. Il est enfin soumis à une étape de salification en présence d'éther chlorhydrique.
***Microanalyse élémentaire :* (*% théorique : mesuré***)
%C=67.83:67.41; %H=5.08:4.61; %N=8.55:8.39; %Cl-=5.41:5.28

### Exemple 10. Chlorhydrate de 6-(5-fluoro-2-{[(3R)-3-méthyl-3,4-dihydroisoquinolin-2(1H)-yl]carbonyl}phényl)-N-(3-fluoro-4-méthylphényl)-N-(4-hydroxyphényl)-1,2,3,4-tétrahydropyrrolo[1,2-a]pyrazine-8-carboxamide

On procède selon le procédé de l'Exemple 1 en remplaçant d'une part le composé de la Préparation 1 utilisé au Stade A par le composé de la Préparation 6, et d'autre part le composé de la Préparation 1" utilisé au Stade C par le composé de la Préparation 6", étant entendu que le produit ainsi obtenu n'est pas soumis à une étape de salification en présence d'éther chlorhydrique comme cela est décrit au Stade D de l'Exemple 1. Le composé ainsi obtenu est déprotégé en présence de 10 équivalents d'acide trifluoroacétique dans le dichlorométhane (10 mL/mmol) à température ambiante pendant une nuit. Puis, le produit est ensuite isolé par concentration à sec du milieu réactionnel. Il est enfin soumis à une étape de salification en présence d'éther chlorhydrique.
***Microanalyse élémentaire** :* ***(*% *théorique : mesuré)***
%C=68.21:68.29; %H=5.27:4.91; %N=8.37:8.34; %Cl-=5.3:5.17

### Exemple 11. Chlorhydrate de N-(4-hydroxyphényl)-6-(6-{[(3R)-3-méthyl-3,4-dihydroisoquinolin-2(1H)-yl]carbonyl}-1,3-benzodioxol-5-yl)-N-(1-méthyl-1H-indazol-5-yl)-1,2,3,4-tétrahydropyrrolo[1,2-a]pyrazine-8-carboxamide

On procède selon le procédé de l'Exemple 1 en remplaçant d'une part le composé de la Préparation 1 utilisé au Stade A par le composé de la Préparation 7, et d'autre part le composé de la Préparation 1" utilisé au Stade C par la *N-*(4*-*{[*tert-*butyl(diméthyl)silyl]oxy}phényl)-1-méthyl-1*H-*indazol-5-amine, étant entendu que le produit ainsi obtenu n'est pas soumis à une étape de salification en présence d'éther chlorhydrique comme cela est décrit au Stade D de l'Exemple 1. Le composé ainsi obtenu est déprotégé en présence de 10 équivalents d'acide trifluoroacétique dans le dichlorométhane (10 mL/mmol) à température ambiante pendant une nuit. Puis, le produit est ensuite isolé par concentration à sec du milieu réactionnel. Il est enfin soumis à une étape de salification en présence d'éther chlorhydrique.
***Microanalyse élémentaire : (% théorique: mesuré)***
%H=5.2:4.83; %N=11.72:11.64; %Cl-=4.94:5.34; %C=66.99:66.19

### Exemple 12. Chlorhydrate de 6-(5-chloro-2-{[(3R)-3-méthyl-3,4-dihydroisoquinolin-2(1H-yl]carbonyl}phényl)-N-(4-hydroxyphényl)-N-(1-méthyl-1H-indazol-5-yl)-1,2,3,4-tétrahydropyrrolo[1,2-a]pyrazine-8-carboxamide

On procède selon le procédé de l'Exemple 1 en remplaçant d'une part le composé de la Préparation 1 utilisé au Stade A par le composé de la Préparation 2, et d'autre part le composé de la Préparation 1" utilisé au Stade C par le *N*-(*4-*{[*tert-*buty(diméthyl)silyl]oxy}phényl)-1-méthyl-1*H*-indazol-5-amine, étant entendu que le produit ainsi obtenu n'est pas soumis à une étape de salification en présence d'éther chlorhydrique comme cela est décrit au Stade D de l'Exemple 1. Le composé ainsi obtenu est déprotégé en présence de 10 équivalents d'acide trifluoroacétique dans le dichlorométhane (10 mL/mmol) à température ambiante pendant une nuit. Puis, le produit est ensuite isolé par concentration à sec du milieu réactionnel. Il est enfin soumis à une étape de salification en présence d'éther chlorhydrique.
***Microanalyse élémentaire: (% théorique : mesuré)***
%C=66.19:65.83; %H=5.13:4.99; %N=11.88:11.85; %Cl-=5.01:5.36

### Exemple 13. Chlorhydrate de N-(4-hydroxyphényl)-3-(6-{[(3S)-3-[2-(morpholin-4-yl)éthyl]-3,4-dihydroisoquinolin-2(1H)-yl]carbonyl}-1,3-benzodioxol-5-yl)-N-phényl-5,6,7,8-tétrahydroindolizine-1-carboxamide

***Microanalyse élémentaire :* (*% théorique : mesuré***)
%C=69.42:69.47; %H=5.96:5.58; %N=7.36:7.36; %Cl-=4.66:4.42

### Exemple 14. Chlorhydrate de N-(4-hydroxyphényl)-N-(1-méthyl-1H-indazol-5-yl)-3-(6-{[(3S-3-[2-(morpholin-4-yl)éthyl]-3,4-dihydroisoquinolin-2(1H)-yl]carbonyl}-1,3-benzodioxol-5-yl)-5,6,7,8-tétrahydroindolizine-1-carboxamide

***Microanalyse élémentaire** : **(% théorique : mesuré***)
%C=67.76:67.81; %H=5.81:5.63; %N=10.31:10.13; %Cl-=4.35:4.22

### Exemple 15. Chlorhydrate de 7-amino-N-(4-hydroxyphényl)-3-(6-{[(3R)-3-méthyl-3,4-dihydroisoquinolin-2(1H)-yl]carbonyl}-1,3-benzodioxol-5-yl)-N-phényl-5,6,7,8-tétrahydroindolizine-1-carboxamide

### Stade A : 3'-(6-{(3R)-3-Méthyl-3,4-dihydroisoquinolin-2(1H)-yl]carbonyl}-1,3-benzodioxol-5-yl)-5',6'-dihydro-8'H-spiro[1,3-dioxolane-2,7'-indolizine]-1'-carboxylate de méthyle

On procède selon le protocole du Stade A de l'Exemple 1 en remplaçant le composé de la Préparation 1 par le composé de la Préparation 8.

### Stade B : 3-(6-{[(3R)-3-Méthyl-3,4-dihydroisoquinolin-2(1H)-yl]carbonyl}-1,3-benzodioxol-5-yl)-7-oxo-5,67,8-tétrahydroindolizine-1-carboxylate de méthyle

4,47 mmol du composé du Stade A en solution dans 75 mL de THF sont agitées en présence de 37 mL de HCl 1 M au reflux pendant 15 h. 100 mL d'eau et 100 mL d'acétate d'éthyle sont ajoutés au milieu réactionnel. On ajoute ensuite 4 g de NaHCO₃ (4,7 mmol) en poudre jusqu'à atteindre un pH basique. On extrait le composé avec de l'acétate d'éthyle, la phase organique est séchée sur MgSO₄, filtrée et concentrée à sec.

### Stade C : 7-Hydroxy-3-(6-{[(3R)-3-méthyl-3,4-dihydroisoquinolin-2(1H)-yl]carbonyl}-1,3-benzodioxol-5-yl)-5,6,7,8-tétrahydroindolizine-1-carboxylate de méthyle

A une solution de 4,47 mmol du composé obtenu au Stade B dans 30 mL de méthanol sont ajoutés par portion 558 mg (14,75 mmol) de borohydrure de sodium. Le milieu réactionnel est agité pendant 1 h à température ambiante. 50 mL de HCl 1M sont ensuite ajoutés et le méthanol est évaporé. La phase aqueuse est ensuite neutralisée avec NaHCO₃, puis extraite avec du dichlorométhane. La phase organique est successivement lavée avec H₂O, séchée sur MgSO₄, filtrée et concentrée à sec. L'huile ainsi obtenue est purifiée par chromatographie flash (gradient dichlorométhane/éthanol-ammoniac) pour conduire au produit attendu.

### Stade D : 3-(6-{[(3R)-3-Méthyl-3,4-dihydroisoquinolin-2(1H)-yl]carbonyl}-1,3-benzodioxol-5-yl)-7-(prop-2-én-1-yloxy)-5,6,7,8-tétrahydroindolizine-1-carboxylate de méthyle

A une suspension de 331 mg (8,26 mmol) d'hydrure de sodium dans 15 mL de THF anhydre refroidi à 0°C, on ajoute 4,13 mmol du composé obtenu au Stade C. Cette suspension est agitée pendant 15 min à 0°C, puis une solution de 790 µL (9,1 mmol) de bromure d'allyle dans 10 mL de THF est additionnée lentement (sur 15 min). Le milieu réactionnel est agité pendant 1h à 0°C, puis 15 h à température ambiante. Cette solution est hydrolysée avec une solution aqueuse saturée avec NH₄Cl. On extrait le composé avec de l'acétate d'éthyle ; la phase organique est séchée sur MgSO₄, filtrée et concentrée à sec. L'huile ainsi obtenue est purifiée par chromatographie flash (gradient cyclohexane/acétate d'éthyle) pour conduire au produit attendu.

### Stade E : N-(4-{[tert-Butyl(diméthyl)silyl]oxy}phényl)-3-(6-{[(3R)-3-méthyl-3,4-dihydroisoquinolin-2(1H)-yl]carbonyl}-1,3-benzodioxol-5-yl)-N-phényl-7-(prop-2-én-1-yloxy)-5,6,7,8-tétrahydroindolizine-1-carboxamide

On procède selon les procédés décrits aux Stades B et C de l'Exemple 1 en utilisant les réactifs appropriés.

### Stade F : N-(4-{[tert-Butyl(diméthyl)silyl]oxy}phényl)-hydroxy-3-(6-{[(3R)-3-méthyl-3,4-dihydroisoquinolin-2(1H)-yl]carbonyl}-1,3-benzodioxol-5-yl)-N-phényl-5,6,7,8-tétrahydroindolizine-1-carboxamide

On réalise ensuite une réaction de déprotection du groupement allyle en présence de 1,3-diméthylpyrimidine-2,4,6(1*H*,3*H*,5*H*)-trione (aussi appelé diméthylbarbiturate) et de tétrakis(triphénylphosphine)palladium dans un mélange de méthanol et de dichlorométhane.

### Stades G : 7-Azido-N-(4-{[tert-butyl(diméthyl)silyl]oxy}phényl)-3-(6-{[(3R)-3-méthyl-3,4-dihydroisoquinolin-2(1H)-yl]carbonyl}-1,3-benzodioxol-5-yl)-N-phényl-5,6,7,8-tétrahydroindolizine-1-carboxamide

A une solution du composé du Stade F (550 mg ; 0,72 mmol) dans le chlorure de méthylène (6 mL), sont ajoutés à température ambiante la triéthylamine (300 µL ; 1,8 mmol) et le chlorure de mésyle (0,14 mL ; 1.8 mmol). Après 20 minutes d'agitation, le milieu réactionnel est concentré à sec, puis dilué dans 10 mL de DMSO. On y ajoute 470 mg de NaN3 en poudre (7,2 mmol). Le milieu réactionnel est laissé 20 h à température ambiante, puis 20 h à 50°C. Il est ensuite coulé sur un mélange de dichlorométhane et d'eau. La phase organique est lavée 3 fois à l'eau, puis à la saumure, séchée sur MgSO4, puis concentrée à sec pour donner le produit attendu qui est engagé tel quel dans l'étape suivante.

### Stade H : Chlorhydrate de 7-amino-N-(4-hydroxyphényl)-3-(6-{[(3R)-3-méthyl-3,4-dihydroisoquinolin-2(1H)-yl]carbonyl}-1,3-benzodioxol-5-yl)-N-phényl-5,6,7,8-tétrahydroindolizine-1-carboxamide

A une solution de 550 mg du composé du Stade G (0,7 mmol) dans l'éthanol (10 mL), est ajouté à température ambiante 20 mg de le Pd/C 10%. Après 15 h d'agitation sous 1 bar d'hydrogène, le milieu réactionnel est filtré sur Whatman et concentré à sec. Après purification par colonne chromatographique sur gel de silice (gradient dichlorométhane/ méthanol), le solide est ensuite solubilisé dans le dichlorométhane et 2 mL d'éther chlorhydrique 1N sont ajoutés. Le tout est agité pendant 1 heure, puis évaporé à sec. Le chlorhydrate ainsi obtenu est dissout dans un mélange eau / acétonitrile jusqu'à solubilisation totale, puis est lyophilisé pour conduire au composé attendu sous la forme d'une poudre.
***Microanalyse élémentaire : (% théorique : mesuré)***
%C=69.17:68.68; %H=5.51:5.09; %N=8.27:8.41; %Cl-=5.24:5.28

### Exemple 16. 3-(6-{{(3S)-3-(Hydroxyméthyl)-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

### Stade A : 3-(6-{[(3S)-3-(Hydroxyméthyl)-3,4-dihydroisoquinolin-2(1H)-yl]carbonyl}-1,3-benzodioxol-5-yl)-5,6,7,8-tétrahydroindolizine-1-carboxylate de méthyle

On procède selon le procédé au Stade A de l'Exemple 1 en utilisant le (3*S*)-1,2,3,4-tétrahydroisoquinolin-3-ylméthanol.

### Stade B : 3-(6-{(3S)-3-[(Prop-2-èn-1-yloxy)méthyl]-3,4-dihydroisoquinolin-2(1H)-yl]carbonyl}-1,3-benzodioxol-5-yl)-5,6,7,8-tétrahydroindolizine-1-carboxylate de méthyle

A une suspension de NaH (703 mg ; 17,6 mmol) dans le THF (20 mL) est ajouté une solution 7,8 g du composé du Stade A (16 mmol) en solution dans un mélange de THF (50 mL) et de DMF (30 mL). Après 1 heure d'agitation, on ajoute le bromure d'allyle (1,7 mL ; 19 mmol). Le milieu réactionnel est laissé sous agitation pendant 48 h à température ambiante, puis il est coulé sur un mélange d'acétate d'éthyle et d'eau. La phase organique est lavée 3 fois avec de l'eau, avec une solution de LiOH saturé, séchée sur MgSO4, concentrée à sec. Après purification par chromatographie sur gel de silice (gradient dichlorométhane/méthanol), on obtient le produit attendu sous la forme d'un solide.
**RMN ¹H:** δ : (500MHz ; dmso-d6 ; 300K) : 7.2-6.9 (m, 4H) ; 7.05 (m, 1H); 6.9 (m, 1H); 6.45-6.1 (m, 1H); 6.15 (m, 2H); 5.9-5.65 (m, 1H); 5.2-5.0 (m, 2H); 5.05-3.8 (m, 1H); 4.85-4.25 (m, 2H); 4.3-3.45 (m, 7H); 3.4-2.4 (m, 6H); 1.95-1.45 (m, 4H)

### Stade C : N-[4-(Benzyloxy)phényl]-N-phényl-3-(6-[1(3S)-3-[(prop-2-n-1-yloxy)méthyl]-3,4-dihydroisoquinolin-2(1H) yl]carbonyl}-1,3-benzodioxol-5-yl)-5,6,7,8-tétrahydroindolizine-1-carboxamide

On procède selon les procédés des Stade B et C de l'Exemple 1 en utilisant la 4-(benzyloxy)-*N-*phénylaniline (*cf.* Préparation 9").

### Stade D : 3-(6-{[(3S)-3-(Hydroxyméthyl)-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

A une suspension de 5,1 g (6,65 mmol) du composé du Stade C dans un mélange de dichlorométhane (7 mL) et de méthanol (2 mL), on ajoute l'acide diméthylbarbiturique (2,1 g ; 13,3 mmol) et le tétrakis(triphénylphosphine)palladium(0) (300 mg ; 0,3 mmol). Après 15 heures d'agitation à 45°C, le milieu réactionnel est coulé sur un mélange d'acétate d'éthyle et d'eau. La phase organique est lavée 2 fois à l'eau, séchée sur MgSO4, concentrée à sec et diluée dans du méthanol (5 mL). L'ensemble est ensuite agité pendant 24 h sous atmosphère d'hydrogène en présence de Pd/C (100 mg). Le milieu réactionnel est ensuite filtré sur Whatman, concentré à sec, puis chromatographié sur gel de silice (gradient dichlorométhane/méthanol) et enfin lyophilisé pour conduire au produit attendu sous la forme d'une poudre.
***Microanalyse élémentaire :*** %***mesuré (théorique)***
%C=72.38(73);%H=5.22(5.5);%N=6.59(6.55)

### Exemple 17. Chlorhydrate de N-{3-fluoro-4-[2-(morpholin-4-yl)éthoxy]phényl}-N-(4-hydroxyphényl)-3-(6-{[(3R)-3-méthyl-3,4-dihydroisoquinolin-2(1H)-yl]carbonyl}-1,3-benzodioxol-5-yl)indolizine-1-carboxamide

***Microanalyse élémentaire : (% théorique : mesuré*)**
%C=67.12:66.79; %H=5.26:4.98; %N=6.96:7.17; %Cl-=4.4:4.77

### Exemple 18. Chlorhydrate de 3-[6-(3,4-dihydroisoquinolin-2(1H)-ylcarbonyl)-1,3-benzodioxol-5-yl]-N-{3-fluoro-4-[2-(morpholin-4-yl)éthoxy]phényl}-N-(4-hydroxyphényl)indolizine-1-carboxamide

***Microanalyse élémentaire : %mesuré* (*théorique*)**
%C=66.99(66.79);%H=4.93(5.1);%N=7.11(7.08);%Cl-=4.46(4.48)

### Exemple 19. Chlorhydrate de N-(4-hydroxyphényl)-3-(5-méthyl-2-{[(3S)-3-[2-(morpholin-4-yl)éthyl]-3,4-dihydroisoquinolin-2(1H)-yl]carbonyl}phényl)-N-phényl-5,6,7,8-tétrahydroindolizine-1-carboxamide

***Microanalyse élémentaire : (% théorique : mesuré**)*
%C=72.26:72.51; %H=6.48:6.13; %N=7.66:7.71; %Cl=4.85:4.95; %Cl-=4.85:4.64

### Exemple 20. Chlorhydrate de N-(4-hydroxyphényl)-3-(2-{[(3S)-3-[2-(morpholin-4-yl)éthyl]-3,4-dihydroisoquinolin-2(1H)-yl]carbonyl)phényl)-N-phényl-5,6,7,8-tétrahydroindolizine-1-carboxamide

***Microanalyse élémentaire : (% théorique : mesuré***)
%C=72:71.11; %H=6.32:5.94; %N=7.81:7.65; %Cl-=4.94:5.08

### Exemple 21. Chlorhydrate de N-(4-hydroxyphényl)-3-(6-{[(3R)-3-méthyl-3,4-dihydroisoquinolin-2(1H-yl]carbonyl}-1,3-benzodioxol-5-yl)-N-(pyridin-4-yl)indolizine-1-carboxamide

***Microanalyse élémentaire : (% théorique : mesuré**)*
%C=69.24:69.12; %H=4.74:4.23; %N=8.5:8.45; %Cl-=5.38:5.2

### Exemple 22. Chlorhydrate de N-(4-hydroxyphényl)-6-(6-{[(3S)-3-[2-(morpholin-4-yl)éthyl]-3,4-dihydroisoquinolin-2(1H)-yl]carbonyl}-1,3-benzodioxol-5-yl)-N-phénylpyrrolo[1,2-a]pyrimidine-8-carboxamide

***Microanalyse élémentaire : (% théorique : mesuré**)*
%C=68.11:66.66; %H=5.32:4.93; %N=9.24:8.84; %Cl-=4.68:5.78
***Masse haute résolution (ESI+) :***
   Formule brute : C₄₃H₃₉N₅O₆
   [M+H]⁺ calculé : 655.2915
   [M+H]⁺ mesuré : 655.2915

### Exemple 23. Chlorhydrate de N-(3-cyanophényl)-N-(4-hydroxyphényl)-3-(6-{[(3S)-3-[2-(morpholin-4-yl)éthyl]-3,4-dihydroisoquinolin-2(1H)-yl]carbonyl}-1,3-benzodioxol-5-yl)-5,6,7,8-tétrahydroindolizine-1-carboxamide

***Microanalyse élémentaire : (% théorique : mesuré)***
%C=68.74:68.59; %H=5.64:5.5; %N=8.91:8.98; %Cl-=4.51:4.48

### Exemple 24. Chlorhydrate de N-(3-fluorophényl)-N-(4-hydroxyphényl)-3-(6-{[(3S)-3-[2-(morpholin-4-yl)éthyl]-3,4-dihydroisoquinolin-2(1H)-yl]carbonyl}-1,3-benzodioxol-5-yl)-5,6,7,8-tétrahydroindolizine-1-carboxamide

***Microanalyse élémentaire : (% théorique : mesuré**)*
%C=67.81:67.45; %H=5.69:5.61; %N=7.19:7.42; %Cl-=4.55:4.84

### Exemple 25. Chlorhydrate de N-(3,4-difluorophényl)-N-(4-hydroxyphényl)-3-(6-{[(3S)-3-[2-(morpholin-4-yl)éthyl]-3,4-dihydroisoquinolin-2(1H)-yl]carbonyl}-1,3-benzodioxol-5-yl)-5,6,7,8-tétrahydroindolizine-1-carboxamide

***Microanalyse élémentaire** : **(% théorique : mesuré)***
%C=66.28:66.56; %H=5.44:5.25; %N=7.03:7.21; %Cl-=4.45:4.32

### Exemple 26. Chlorhydrate de N-(3-fluorophényl)-3-(6-{[(3S)-3-[2-(morpholin-4-yl)éthyl]-3,4-dihydroisoquinolin-2(1H)-yl]carbonyl}-1,3-benzodioxol-5-yl)-N-phényl-5,6,7,8-tétrahydroindolizine-1-carboxamide

***Microanalyse élémentaire : (% théorique** : **mesuré)***
%C=69.24:70.16; %H=5.81:5.79; %N=7.34:7.47; %Cl-=4.64:4.58

### Exemple 27. Chlorhydrate de 3-(5-chloro-2-{[(3S)-3-[2-(morpholin-4-yl)éthyl]-3,4-dihydroisoquinolin-2(1H)-yl]carbonyl}phényl)-N-(3-fluorophényl)-N-(4-hydroxyphényl)-5,6,7,8-tétrahydroindolizine-1-carboxamide

***Microanalyse élémentaire : (% théorique : mesuré)***
%C=67.1:67.68; %H=5.63:5.4; %N=7.28:7.34; %Cl-=4.61:4.59

### Exemple 28. Chlorhydrate de N-(4-hydroxyphényl)-3-(5-méthoxy-2-{[(3S)-3-[2-(morpholin-4-yl)éthyl]-3,4-dihydroisoquinolin-2(1H)-yl]carbonyl)phényl)-N-phényl-5,6,7,8-tétrahydroindolizine-1-carboxamide

***Microanalyse élémentaire** :* ***(*% *théorique : mesuré)***
%C=70.72:70.05; %H=6.34:5.95; %N=7.5:7.33; %Cl-=4.74:4.74

### Exemple 29. Chlorhydrate de N-(4-hydroxyphényl)-3-(4-méthoxy-2-{[(3S)-3-[2-(morpholin-4-yl)éthyl]-3,4-dihydroisoquinolin-2(1H)-yl]carbonyl}phényl)-N-phényl-5,6,7,8-tétrahydroindolizine-1-carboxamide

***Microanalyse élémentaire : (% théorique : mesuré)***
%C=70.72:68.96; %H=6.34:5.78; %N=7.5:7.24; %Cl-=4.74:4.62

### Masse haute résolution (ESI+) :

Formule brute : C₄₄ H₄₆ N₄ O₅
[M+H]⁺ calculé: 711.3546
[M+H]⁺ mesuré : 711.3540

### Exemple 30. Chlorhydrate de N-{4-[(3,3-difluoropipéridin-1-yl)méthyl]phényl}-N-(4-hydroxyphényl)-3-(6-{[(3R)-3-méthyl-3,4-dihydroisoquinolin-2(1H)-yl]carbonyl}-1,3-benzodioxol-5-yl)indolizine-1-carboxamide

***Microanalyse élémentaire : (% théorique : mesuré***)
%C=68.31:69.12; %H=5.22:4.93; %N=7.08:6.96; %Cl-=4.48:4.07

### Exemple 31. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(quinolin-6-yl)indolizine-1-carboxamide

***Microanalyse élémentaire : (% théorique : mesuré**)*
%C=71.13:71.29; %H=4.69:4.39; %N=7.9:8.14; %Cl-=5:4.5

### Exemple 32. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(2-méthylpyridin-4-yl)indolizine-1-carboxamide

***Microanalyse élémentaire : (% théorique : mesuré**)*
%C=69.59:69.81; %H=4.94:4.53; %N=8.32:8.59; %Cl-=5.27:5.01

### Exemple 33. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(1-méthyl-1H-pyrrolo[2,3-b]pyridin-5-yl)indolizine-1-carboxamide

***Microanalyse élémentaire* : *(% théorique : mesuré)***
%C=69.14:70.09; %H=4.81:4.55; %N=9.83:10.09; %Cl-=4.98:3.26

### Exemple 34. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(pyridin-3-yl)indolizine-1-carboxamide

***Microanalyse élémentaire :* (% *théorique : mesuré)***
%C=69.24:70.21; %H=4.74:4.42; %N=8.5:8.51; %Cl-=5.38:3.33

### Exemple 35. Chlorhydrate de N-{4-[2-(3,3-difluoropipéridin-1-yl)éthyllphényl}-N-(4-hydroxyphényl)-3-(6-{[(3R)-3-méthyl-3,4-dihydroisoquinolin-2(1H)-yl]carbonyl)-1,3-benzodioxol-5-yl)indolizine-1-carboxamide

***Microanalyse élémentaire : (% théorique** : **mesuré***)
%C=68.61:67.96; %H=5.38:5.14; %N=6.96:6.76; %Cl-=4.4:4.36

### Exemple 36. Chlorhydrate de N-{4-{2-(3,3-difluoropyrrolidin-1-yl)éthyl]phényl)-N-(4-hydroxyphényl)-3-(6-{[(3R)-3-méthyl-3,4-dihydroisoquinolin-2(1H)-yl]carbonyl}-1,3-benzodioxol-5-yl)indolizine-1-carboxamide

***Microanalyse élémentaire** : **(% théorique : mesuré***)
%C=68.31:68.51; %H=5.22:4.85; %N=7.08:6.83; %Cl-=4.48:4.48

### Exemple 37. Chlorhydrate de 3-(6-{[(3S)-3-(2-aminoéthyl)-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

### Stade A : 3-(6-{[(3S)-3-[2-[(tert-Butoxycarbonyl)amino]éthyl}-3,4-dihydroisoquinolin-2(1H)-yl]carbonyl}-1,3-benzodioxol-5-yl)-5,6,7,8-tétrahydroindolizine-1-carboxylate de méthyle

A une solution de 2 g du composé de la Préparation 1 dans 20 mL de dichlorométhane, on ajoute à température ambiante 5,5 mL de *N,N,N* triéthylamine (6,96 mmol), le composé de la Préparation 3' (6,96 mmol), puis 0,94 g d'hydroxybenzotriazole (HOBT) et 1,34 g d'1-éthyl-3-(3'-diméthylaminopropyl)-carbodiimide (EDC) (6,96 mmol). Le milieu réactionnel est ensuite agité à température ambiante pendant 1 nuit, puis il est versé sur une solution de chlorure d'ammonium et extrait avec de l'acétate d'éthyle. La phase organique est ensuite séchée sur sulfate de magnésium, puis filtrée et évaporée à sec. Le produit brut ainsi obtenu est alors purifié par chromatographie sur gel de silice (gradient heptane/ AcOEt) pour conduire au produit attendu.
**RMN¹H :** δ (400 MHz; dmso-d6 ; 300K): 7.2-6.8 (m, 4H, H aromatiques, H tétrahydroisoquinoline) ; 7.15-6.90 (m, 4H, H aromatique, tétrahydroisoquinoline) ; 7.00-6.80 (m, 2H, H aromatique, benzodioxole) ; 6.68+6.55+6.25 (m, 1H, **NH**); 6.50-6.05 (m, 1H, H aromatique, tétrahydroindolizine); 6.12 (m, 2H, H aliphatiques, O**CH₂**O) ; 4.95+4.20+4.10 (m, 2H, H aliphatique, **CH₂**N tétrahydroisoquinoline); 4.85+4.78+3.80 (m, 1H, H aliphatique, **CH** tétrahydroisoquinoline) ; 4.00-3.40 (m, 2H, H aliphatiques, **CH₂**N tétrahydroindolizine) ; 3.70-3.50 (m, 3H, COO**CH₃**) ; 2.95-2.45 (m, 2H, H aliphatiques, **CH₂**NHBoc); 2.98-2.30 (m, 2H, H aliphatiques, **CH₂**C tétrahydroindolizine) ; 3.00+2.60+2.42 (m, 2H, H aliphatiques, **CH₂**CH tétrahydroindolizine); 1.95-1.40 (m, 4H, H aliphatiques, **CH₂CH₂** tétrahydroindolizine); 1.35-1.25 (m, 9H, H aliphatiques, **tBu**); 1.50-1.15 (m, 2H, H aliphatiques, **CH₂**CH₂NHBoc)

### Stade B: 3-(6-{[(3S)-3-{2-[(tert-Butoxycarbonyl)amino]éthyl}-3,4-dihydroisoquinolin-2(1H)-yl]carbonyl}-1,3-benzodioxol-5-yl)-5,6,7,8-tétrahydroindolizine-1-carboxylate de lithium

A une solution contenant 8,26 mmol du composé du Stade A dans 24 mL de dioxane est ajoutée une solution d'hydroxyde de lithium (675 mg, 16,1 mmol). L'ensemble est placé dans un four à micro-ondes à 140 W, 100°C pour une durée de 2h30. Le milieu réactionnel est ensuite filtré et évaporé. Le solide ainsi obtenu est séché à 40°C dans une étuve en présence de P₂O₅.

### Stade C: (2-{3S)-2-[(6-{1-[(4-{[tert-Butyl(diméthyl)silyl]oxy}phényl)(phényl) carbamoyl]-5,6,7,8-tétrahydroindolizin-3-yl}-1,3-benzodioxol-5-yl)carbonyl}-1,2,3,4-tétrahydroisoquinolin-3-yl}éthyl)carbamate de tert-butyle

A une solution contenant 4,73 mmol du composé du Stade B dans 47 mL de dichlorométhane sont ajoutés, au goutte à goutte, 1,2 mL de chlorure d'oxalyle à 0°C. Le milieu réactionnel est agité à température ambiante pendant 11 heures, puis co-évaporé le plusieurs fois avec du dichlorométhane. Le produit ainsi obtenu est mis en suspension dans 37 mL de dichlorométhane, puis est additionné sur une solution contenant 7,1 mmol du composé obtenu à la Préparation 8" dans 10 mL de dichlorométhane en présence de 0,6 mL de pyridine (7,1 mmol). L'ensemble est agité à température ambiante pendant une nuit. Le milieu réactionnel est concentré, purifié par chromatographie sur gel de silice (gradient dichlorométhane/méthanol) pour conduire au produit attendu.
**RMN¹H :** δ (400 MHz; dmso-d6; 300K): 7.0 (m, 11H, H aromatiques, Ph + 4H, tétrahydroisoquinoline + 2H, PhO) ; 6.80-6.65 (m, 2H, H aromatiques, PhO) ; 6.95-6.85 (m, 2H, H aromatique, benzodioxole); 6.70+6.40 (3tl, 1 H, **NH**); 6.10 (m, 2H, H aliphatiques, O**CH₂**O) ; 5.25-4.85 (m, 1H, H aromatique, tétrahydroindolizine); 5.00+4.00 (m, 2H, H aliphatique, **CH₂**N tétrahydroisoquinoline) ; 4.90-3.60 (m, 1H, H aliphatique, **CH** tétrahydroisoquinoline) ; 4.10-3.40 (m, 2H, H aliphatiques, **CH₂**N tétrahydroindolizine) ; 3.00-2.50 (m, 2H, H aliphatiques, **CH₂**C tétrahydroindolizine) ; 3.00+2.40 (m, 2H, H aliphatiques, **CH₂**CH tétrahydroindolizine); 3.00-2.50 (m, 2H, H aliphatiques, **CH₂**NHBoc); 1.80-1.50 (m, 4H, H aliphatiques, **CH₂CH₂** tétrahydroindolizine); 1.50-1.30 (m, 2H, H aliphatiques, **CH₂**CH₂NHBoc); 1.35 (2s, 9H, H aliphatiques, **tBu**); 0.90 (s, 9H, H aliphatiques, **tBu**-Si); 0.10 (m, 6H, H aliphatiques, **Me-**Si)

### Stade D: Chlorhydrate de 3-(6-{[(3S)-3-(2-aminoéthyl)-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

A une solution de 800 mg (0,92 mmol) du composé du Stade C dans 10 mL de méthanol, est ajouté 258 mg (4,60 mmol) de KOH. Après 3h d'agitation à température ambiante, le milieu réactionnel est traité par une solution de HCl 4M dans 6 mL de dioxane. Après 2h d'agitation à température ambiante, le milieu réactionnel est concentré et traité par une solution aqueuse saturée de NaHCO₃ et extraite au chlorure de méthylène. La phase organique est ensuite séchée sur sulfate de magnésium, puis filtrée et évaporée à sec. Le produit brut ainsi obtenu est alors purifié par chromatographie sur gel de silice (gradient dichlorométhane/ méthanol). Le composé est ensuite dissout dans 5 mL de dichlorométhane et on ajoute 2,5 mL d'éther chlorhydrique 1M. Le composé est filtré et séché sous vide. On obtient le produit attendu sous la forme d'une mousse.
***Microanalyse élémentaire : (% théorique : mesuré)***
%C=69.51:69.53; %H=5.69:5.27; %N=8.11:8.04; %Cl-=5.13:5.2
***Masse haute résolution (ESI+) :***
Formule brute : C₄₀H₃₈N₄O₅
[M+H]⁺ calculé : 655.2915
[M+H]⁺ mesuré : 655.2915
**RMN¹H :** δ (400 MHz ; dmso-d6 ; 300K) : 9.55+9.45 (2s, 1H, OH); 7.80+7.75 (2s, 3H, **NH3**⁺); 7.46-6.55 (m, 11H, H aromatiques, Ph + 4H, tétrahydroisoquinoline + 2H, PhO); 6.90-6.55 (m, 2H, H aromatiques, PhO); 7.00-6.70 (plusieurs s, 2H, H aromatique, benzodioxole); 5.35-5.00 (plusieurs s, 1H, H aromatique, tétrahydroindolizine); 6.10 (plusieurs s, 2H, H aliphatiques, O**CH₂**O); 5.00-3.35 (plusieurs m, 4H, H aliphatique, **CH₂**N tétrahydroisoquinoline + **CH₂**N tétrahydroindolizine) ; 4.85+4.75+3.60 (plusieurs m, 1H, H aliphatique, **CH** tétrahydroisoquinoline); 2.85-2.45 (plusieurs m, 2H, H aliphatiques, **CH₂**NH₂); 3.00-2.45 (plusieurs m, 2H, H aliphatiques, **CH₂**C tétrahydroindolizine) ; 3.05+2.30 (plusieurs m, 2H, H aliphatiques, **CH₂**CH tétrahydroisoquinoline); 1.85-1.40 (plusieurs m, 2H, H aliphatiques, **CH₂** tétrahydroisoquinoline); 1.95-1.35 (plusieurs m, 2H, H aliphatiques, **CH₂** tétrahydroisoquinoline); 1.75-1.40 (plusieurs m, 2H, H aliphatiques, **CH₂**CH₂NH₂)
**IR :** ν : -OH: 3375 cm⁻¹ (phénol) ; ν : -NH3⁺: 3500-2300 cm⁻¹ (sel d'amine primaire); ν : >C=O 1612 cm⁻¹ + épaulement (amide)

### Exemple 38. Chlorhydrate de N-(4-hydroxyphényl)-3-(6-{[(3R)-3-[3-(morpholin-4-yl)propyl]-3,4-dihydroisoquinolin-2(1H)-yl]carbonyl}-1,3-benzodioxol-5-yl)-N-phényl-5,6,7,8-tétrahydroindolizine-1-carboxamide

***Microanalyse élémentaire : (% théorique : mesuré)***
%C=69.71:69.62; %H=6.11:5.67; %N=7.23:7.12; %Cl-=4.57:4.81

### Exemple 39. Chlorhydrate de N-(2,6-diméthylpyridin-4-yl)-N-(4-hydroxyphényl)-3-(6-{[(3R)-3-méthyl-3,4-dihydroisoquinolin-2(1H)-yl]carbonyl}-1,3-benzodioxol-5-yl)indolizine-1-carboxamide

***Microanalyse élémentaire : (% théorique : mesuré)***
%C=69.91:69.68; %H=5.13:4.78; %N=8.15:8.03; %Cl-=5.16:5.16

### Exemple 40. N-(4-Hydroxyphényl)-3-(6-{[(3R)-3-méthyl-3,4-dihydroisoquinolin-2(1H)-yl]carbonyl}-1,3-benzodioxol-5-yl)-N-phényl-5,6,7,8-tétrahydroindolizine-1-carboxamide

***Microanalyse élémentaire : (% théorique : mesuré)***
%C=74.86:74.88; %H=5.64:5.31; %N=6.72:6.78

### Exemple 41. Chlorhydrate de 3-(6-{[(3S)-3-[2-(3,3-difluoropipéridin-1-yl)éthyl]-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

***Microanalyse élémentaire : (% théorique : mesuré)***
%C=67.96:68.34; %H=5.7:5.4; %N=7.04:6.97; %Cl-=4.46:4.27

### Exemple 42. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(pyridin-4-yl)-5,6,7,8-tétrahydroindolizine-1-carboxamide

***Microanalyse élémentaire : (% théorique : mesuré)***
%C=68.82:69.46; %H=5.32:4.95; %N=8.45:8.48; %Cl-=5.35:4.6

### Exemple 43. 3-(6-{[(3S)-3-{2-[(2,2-Difluoroéthyl)amino]éthyl}-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

### Stade A : 3-(6-{[(3S)-3-{2-[(tert-Butoxycarbonyl)amino]éthyl}-3,4-dihydroisoquinolin-2(1H)-yl]carbonyl}-1,3-benzodioxol-5-yl)-5,6,7,8-tétrahydroindolizine-1-carboxylate d'éthyle

Le procédé est analogue à celui décrit au Stade A de l'Exemple 37.

### Stade B : 3-(6-{[(3S)-3-{2-[(tert-Butoxycarbonyl)(2,2-difluoroéthyl)amino]éthyl}-3,4-dihydroisoquinolin-2(1H)yl]carbonyl}-1,3-benzodioxol-5-yl)-5,6,7,8-tétrahydroindolizine-1-carboxylate d'éthyle

A une suspension de 337 mg de NaH (60%) (8,41 mmol) dans 13 mL de diméthylformamide, on ajoute goutte à goutte une solution de 1,01g (1,68 mmol) du composé du Stade A dans 13 mL de diméthylformamide. Cette suspension est agitée température ambiante pendant 15 min, puis on ajoute 1,08 g (5,04 mmol) de 2,2-difluoroéthyl trifluorométhanesulfonate dans 13 mL de diméthylformamide. L'ensemble est agité à température ambiante pendant 2h. Une solution de 20 mL de chlorure d'ammonium saturée est ajoutée. La solution est extraite à l'acétate d'éthyle. La phase organique est alors séchée sur MgSO₄, filtrée, concentrée à sec. Après purification par colonne chromatographique sur gel de silice (cyclohexane /acétate d'éthyle), on obtient le produit attendu sous la forme d'une huile.
***Masse haute résolution (ESI*+*) :***
Formule brute : C₃₇H₄₃CN₃O₇
[M+H]⁺ calculé : 680.3142
[M+H]⁺ mesuré : 680.3145
**RMN¹H :** δ (400 MHz; dmso-d6; 300K): 7.25-6.90 (m, 4H, H aromatiques, tétrahydroisoquinoline); 7.10-6.75 (m, 2H, H aromatique, benzodioxole); 6.40-6.05 (m, 1H, H aromatique, tétrahydroindolizine); 6.10 (m, 2H, H aliphatiques, O**CH₂**O); 6.25-5.90 (m, 1H, H aliphatiques, **CHF₂**); 4.95-4.10 (m, 2H, H aliphatique, **CH₂**N tétrahydroisoquinoline) ; 4.80+3.80 (2m, 1H, H aliphatique, **CH** tétrahydroisoquinoline); 4.10-4.00 (m, 2H, **CH₂**Et); 4.05-3.40 (m, 2H, H aliphatique, **CH₂**N tétrahydroindolizine) ; 3.60-2.60 (m, 4H, H aliphatique, **CH₂**CHF₂ +**CH₂**NBoc) ; 3.00-2.35 (m, 2H, H aliphatiques, **CH₂**C tétrahydroindolizine); 3.00+2.45 (m, 2H, H aliphatiques, **CH₂**CH tétrahydroisoquinoline); 1.95+1.40 (m, 4H, H aliphatiques, **CH₂CH₂** tétrahydroindolizine); 1.40 (m, 9H, H aliphatiques, **^{t}Bu**); 1.65-1.20 (m, 2H, H aliphatiques, **CH₂**CH₂NBoc); 1.18+1.10 (2t, 3H, H aliphatiques **CH₃** Et)

### Stade C : (2-{(3S)-2-[(6-{1-[(4-{[tert-Butyl(diméthyl)silyl]oxy}phényl)(phényl) carbamoyl]-5,6,7,8-tétrahydroindolizin-3-yl}-1,3-benzodioxol-5-yl)carbonyl]-1,2,3,4-tétrahydroisoquinolin-3yl}éthyl)(2,2-difluoroéthyl)carbamate de tert-butyle

Le procédé est analogue à celui décrit aux Stades B et C de l'Exemple 37.
**RMN¹H :** δ (400 MHz ; dmso-d6 ; 300K) : 7.30-6.60 (m, 9H, H aromatiques, 4H tétrahydroisoquinoline + Ph);6.90-6.70 (m, 2H, H aromatique, benzodioxole);6.80-6.60 (m, 4H, PhO); 6.10 (m, 2H, H aliphatiques, O**CH₂O**); 6.20-5.90 (m, 1H, H aliphatiques, **CHF₂**); 5.50-4.80 (4s, 1H, H aromatique, tétrahydroindolizine); 5.20-4.00 (m, 2H, H aliphatique, **CH₂**N tétrahydroisoquinoline) ; 4.80+4.70+3.50 (3m, 1H, H aliphatique, **CH** tétrahydroisoquinoline); 4.20-3.40 (m, 2H, H aliphatique, **CH₂**N tétrahydroindolizine) ; 3.60-3.10 (m, 4H, H aliphatique, **CH₂**CHF₂ +**CH₂**NBoc) ; 3.00+2.60 (m, 2H, H aliphatiques, **CH₂**CH tétrahydroisoquinoline); 3.00-2.50 (m, 2H, H aliphatiques, **CH₂**C tétrahydroindolizine); 1.80+1.50 (m, 4H, H aliphatiques, **CH₂CH₂** tétrahydroindolizine); 1.60-1.30 (m, 2H, H aliphatiques, **CH₂**CH₂NBoc); 1.40-1.30 (m, 9H, H aliphatiques, **tBu**); 0.90 (4s, 9H, H aliphatiques, **tBu**-Si); 0.10 (4s, 6H, H aliphatiques, **Me**-Si)

### Stade D : 3-(6-{[(3S)-3-{2-[(2,2-Difluoroéthyl)amino]éthyl}-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

A une solution de 933 mg (1,00 mmol) du composé du Stade C dans 10 mL de méthanol, sont ajoutés 280 mg (5,00 mmol) de KOH. Après 3h d'agitation à température ambiante, le milieu réactionnel est traité par une solution de HCl 4M dans 6 mL de dioxane. Après 2h d'agitation à température ambiante, le milieu réactionnel est concentré et traité par une solution aqueuse saturée de NaHCO₃, puis extraite au chlorure de méthylène. La phase organique est ensuite séchée sur sulfate de magnésium, puis filtrée et évaporée à sec. Le produit brut ainsi obtenu est alors purifié par chromatographie sur gel de silice (gradient dichlorométhane/méthanol) pour donner le produit attendu sous la forme d'une mousse.
***Microanalyse élémentaire : (% théorique : mesuré)***
%C=70.18:69.79; %H=5.61:5.67; %N=7.79:7.7
***Masse haute résolution (ESI*+*) :***
   Formule brute : C₄₂H₄₀F₂N₄O₅
   [M+H]⁺ calculé : 655.2915
   [M+H]⁺ mesuré : 655.2915

### Exemple 44. N-(4-Hydroxyphényl)-3-(6-{[(3S)-3-[2-(3-méthoxyazétidin-1-yl)éthyl]-3,4-dihydroisoquinolin-2(1H)-yl]carbonyl]-1,3-benzodioxol-5-yl)-N-phényl-5,6,7,8-tétrahydroindolizine-1-carboxamide

***Microanalyse élémentaire : (% théorique : mesuré)***
%C=72.91:72.73; %H=6.12:5.67; %N=7.73:7.74

### Exemple 45. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-{1-[2-(morpholin-4-yl)éthyl]-1H-pyrazol-4-yl}indolizine-1-carboxamide

***Microanalyse élémentaire : (% théorique : mesuré)***
%C=66.27:66.05; %H=5.43:5.27; %N=11.04:11.07; %Cl-=4.66:4.61

### Exemple 46. Chlorhydrate de N-(3-fluoropyridin-4-yl)-N-(4-hydroxyphényl)-3-(6-{[(3R)-3-méthyl-3,4-dihydroisoquinolin-2(1H)-yl]carbonyl}-1,3-benzodioxol-5-yl)indolizine-1-carboxamide

***Masse haute résolution (ESI+) :***
Formule brute : C₃₈H₂₉FN₄O₅
[M+H]⁺ calculé: 641.2195
[M+H]⁺ mesuré: 641.2195

### Exemple 47. 3-(5-Chloro-2-{[(3R)-3-méthyl-3,4-dihydroisoquinolin-2(1H)-yl]carbonyl}phényl)-N-(4-hydroxyphényl)-N-(1-méthyl-1H-pyrazol-4-yl)-5,6,7,8-tétrahydroindolizine-1-carboxamide

***Microanalyse élémentaire : (% théorique : mesuré)***
%C=69.72:69.53; %H=5.53:5.6; %N=11.29:10.85

### Exemple 48. N-(4-Hydroxyphényl)-3-(7-{[(3R)-3-méthyl-3,4-dihydroisoquinolin-2(1H)-yl]carbonyl}-2,3-dihydro-1,4-benzodioxin-6-yl)-N-(1-méthyl-1H-pyrazol-4-yl)-5,6,7,8-tétrahydroindolizine-1-carboxamide

***Microanalyse élémentaire : (% théorique : mesuré)***
%C=70.9:70.89; %H=5.79:5.56; %N=10.88:10.8

### Exemple 49. Chlorhydrate de 3-(5-chloro-2-{[(3R)-3-méthyl-3,4-dihydroisoquinolin-2(1H)-yl]carbonyl}phényl)-N-(4-hydroxyphényl)-N-(pyridin-4-yl)indolizine-1-carboxamide

***Microanalyse élémentaire : (% théorique : mesuré)***
%C=68.42:68.17; %H=4.65:4.48; %N=8.63:8.48; %Cl-=5.46:5.13

### Exemple 50. 3-(5-Chloro-2-{[(3R)-3-méthyl-3,4-dihydroisoquinolin-2(1H)-yl]carbonyl}phényl)-N-(4-hydroxyphényl)-N-(1-méthyl-1H-pyrrolo[2,3-b]pyridin-5-yl)indolizine-1-carboxamide

***Microanalyse élémentaire : (% théorique : mesuré)***
%C=72.12:71.58; %H=4.84:4.84; %N=10.51:10.48

### Exemple 51. Chlorhydrate de N-(4-hydroxyphényl)-N-(imidazo[1,2-a]pyridin-7-yl)-3-(6-{[(3R)-3-méthyl-3,4-dihydroisoquinolin-2(1H)-yl]carbonyl}-1,3-benzodioxol-5-yl)indolizine-1-carboxamide

***Microanalyse élémentaire : (% théorique : mesuré)***
%C=68.81:68.28; %H=4.62:4.59; %N=10.03:9.66; %Cl-=5.08:4.81

### Exemple 52. N-(4-Hydroxyphényl)-3-(2-{[(3R)-3-méthyl-3,4-dihydroisoquinolin-2(1H)-yl]carbonyl}phényl)-N-(1-méthyl-1H-pyrrolo[2,3-b]pyridin-5-yl)indolizine-1-carboxamide

***Microanalyse élémentaire : (% théorique: mesuré)***
%C=76.05:75.88; %H=5.26:5.24; %N=11.09:11.09

### Exemple 53. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(2-méthylimidazo[1,2-a]pyridin-7-yl)indolizine-1-carboxamide

***Microanalyse élémentaire : (% théorique : mesuré)***
%C=69.14:69.65; %H=4.81:4.75; %N=9.83:9.79; %Cl-=4.98:4.7

### Exemple 54. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-(6-méthylpyridin-3-yl)indolizine-1-carboxamide

***Microanalyse élémentaire : (% théorique : mesuré)***
%C=69.59:68.78; %H=4.94:5; %N=8.32:8.33; %Cl-=5.27:5.18

### Exemple 55. N-(5-Fluoropyridin-3-yl)-N-(4-hydroxyphényl)-3-(6-{[(3R)-3-méthyl-3,4-dihydroisoquinolin-2(1H)-yl]carbonyl}-1,3-benzodioxol-5-yl)indolizine-1-carboxamide

***Microanalyse élémentaire : (% théorique : mesuré)***
%C=71.24:70.77; %H=4.56:4.36; %N=8.75:8.82

### Exemple 56. N-(4-Hydroxyphényl)-N-(2-méthoxypyridin-4-yl)-3-(6-{[(3R)-3-méthyl-3,4-dihydroisoquinolin-2(1H)-yl]carbonyl}-1,3-benzodioxol-5-yl)indolizine-1-carboxamide

***Masse haute résolution (ESI+) :***
Formule brute : C₃₉H₃₂N₄O₆
[M+H]⁺ calculé: 653.2395
[M+H]⁺ mesuré : 653.2385

### Exemple 57. 3-[6-(3,4-Dihydroisoquinolin-2(1H)-ylcarbonyl)-1,3-benzodioxol-5-yl]-N-(4-hydroxyphényl)-N-phényl-5,6,7,8-tétrahydroindolizine-1-carboxamide

***Microanalyse élémentaire : % mesuré (théorique)***
%C=74.17(74.62);%H=5.43(5.44);%N=6.87(6.87)

### Exemple 58. Chlorhydrate de N-(4-hydroxyphényl)-3-(6-{[(3R)-3-méthyl-3,4-dihydroisoquinolin-2(1H)-yl]carbonyl}-1,3-benzodioxol-5-yl)-N-[2-(propan-2-yl)pyridin-4-yl]indolizine-1-carboxamide

***Microanalyse élémentaire : (% théorique : mesuré)***
%C=70.23:69.95; %H=5.32:5.4; %N=7.99:7.99; %Cl-=5.06:4.92

### Exemple 59. N-(4-Hydroxyphényl)-3-(6-{[(3R)-3-méthyl-3,4-dihydroisoquinolin-2(1H)-yl]carbonyl}-1,3-benzodioxol-5-yl)-N-(pyrazolo[1,5-a]pyrimidin-6-yl)indolizine-1-carboxamide

***Microanalyse élémentaire : (% théorique : mesuré)***
%C=70.68:70.47; %H=4.56:4.61; %N=12.68:12.45

### Exemple 60. 3-(5-Fluoro-2-{[(3R)-3-méthyl-3,4-dihydroisoquinolin-2(1H)-yl]carbonyl}phényl)-N-(4-hydroxyphényl)-N-(1-méthyl-1H-pyrazol-4-yl)indolizine-1-carboxamide

***Microanalyse élémentaire : %mesuré (théorique)***
%C=71.85(72.11);%H=4.78(5.04);%N=10.79(11.68)

### Exemple 61. 3-(5-Fluoro-2-{[(3R)-3-méthyl-3,4-dihydroisoquinolin-2(1H)-yl]carbonyl}phényl)-N-(4-hydroxyphényl)-N-(1-méthyl-1H-pyrazol-4-yl)-5,6,7,8-tétrahydroindolizine-1-carboxamide

***Microanalyse élémentaire : %mesuré (théorique)***
**%C=72.31(71.62);%H=5.6(5.68);%N=10.94(11.6)**

### Exemple 62. 3-(5-Fluoro-2-{[(3R)-3-méthyl-3,4-dihydroisoquinolin-2(1H)-yl]carbonyl}phényl)-N-(4-hydroxyphényl)-N-(pyridin-4-yl)indolizine-1-carboxamide

***Microanalyse élémentaire : %mesuré (théorique)***
%C=74.08(74.48);%H=4.82(4.9);%N=8.59(9.39)

### Exemple 63. 3-(5-Fluoro-2-{[(3R)-3-méthyl-3,4-dihydroisoquinolin-2(1H)-yl]carbonyl}phényl)-N-(4-hydroxyphényl)-N-(1-méthyl-1H-pyrrolo[2,3-b]pyridin-5-yl)indolizine-1-carboxamide

***Microanalyse élémentaire : %mesuré (théorique)***
%C=73.14(73.95);%H=4.83(4.96);%N=10.29(10.78)

### Exemple 64. 3-(5-Fluoro-2-{[(3R)-3-méthyl-3,4-dihydroisoquinolin-2(1H)-yl]carbonyl}phényl)-N-(4-hydroxyphényl)-N-(pyridin-4-yl)-5,6,7,8-tétrahydroindolizine-1-carboxamide

***Microanalyse élémentaire : %mesuré (théorique)***
%C=74.61(73.98);%H=5.26(5.54);%N=8.94(9.33)

### Exemple 65. 3-(5-Fluoro-2-{[(3R)-3-méthyl-3,4-dihydroisoquinolin-2(1H)-yl]carbonyl}phényl)-N-(4-hydroxyphényl)-N-(1-méthyl-1H-pyrrolo[2,3-b]pyridin-5-yl)-5,6,7,8-tétrahydroindolizine-1-carboxamide

***Microanalyse élémentaire : %mesuré (théorique)***
%C=73.59(73.49);%H=5.22(5.55);%N=9.93(10.71)

### Exemple 66. N-(4-Hydroxyphényl)-3-(6-{[(3R)-3-méthyl-3,4-dihydroisoquinolin-2(1H)-yl]carbonyl}-1,3-benzodioxol-5-yl)-N-([1,2,4]triazolo[1,5-a]pyrimidin-6-yl)indolizine-1-carboxamide

***Microanalyse élémentaire : %mesuré (théorique)***
%C=68.57(68.77);%H=3.92(4.4);%N=14.21(14.77)

### Masse haute résolution (ESI+) :

Formule brute : C₃₈H₂₉N₇O₅
[M+H]⁺ calculé : 664.2303
[M+H]⁺ mesuré : 664.2310

### Exemple 67. N-(4-Hydroxyphényl)-3-(6-{[(3R)-3-méthyl-3,4-dihydroisoquinolin-2(1H)-yl]carbonyl}-1,3-benzodioxol-5-yl)-N-(1-oxidopyridin-4-yl)indolizine-1-carboxamide

***Microanalyse élémentaire : %mesuré (théorique)***
%C=69.7(71.46);%%H=4.43(4.73);%N=8.54(8.77)

### Masse haute résolution (ESI+) :

Formule brute : C₃₈H₃₀N₄O₆
[M+H]⁺ calculé : 639.2238
[M+H]⁺ mesuré : 639.2234

### Exemple 68. Chlorhydrate de N-(4-hydroxyphényl)-3-(2-{[(3R)-3-méthyl-3,4-dihydroisoquinolin-2(1H)-yl]carbonyl}phényl)-N-(pyridin-4-yl)indolizine-1-carboxamide

***Microanalyse élémentaire : %mesuré (théorique)***
%C=71.97(72.25);%H=5.21(5.08);%N=8.99(9.11);%Cl-=5.32(5.76)

### Masse haute résolution (ESI+) :

Formule brute : C₃₇H₃₀N₄O₃
[M+H]⁺ calculé : 579.2391
[M+H]⁺ mesuré : 579.2403

### Exemple 69. Chlorhydrate de N-(4-hydroxyphényl)-N-(1-méthyl-1H-pyrrolo[2,3-b]pyridin-5-yl)-3-(6-{[(3R)-3-[3-(morpholin-4-yl)propyl]-3,4-dihydroisoquinolin-2(1H)-yl]carbonyl}-1,3-benzodioxol-5-yl)-5,6,7,8-tétrahydroindolizine-1-carboxamide

***Microanalyse élémentaire : %mesuré (théorique)***
%C=67.63(68.06);%H=5.27(5.95);%N=10.08(10.13);%Cl-=4.53(4.27)

### Masse haute résolution (ESI+) :

Formule brute : C₄₇H₄₈N₆O₆
[M+H]⁺ calculé : 793.3708
[M+H]⁺ mesuré : 793.3704

### Exemple 70. N-(4-Hydroxyphényl)-3-(6-{[(3R)-3-méthyl-3,4-dihydroisoquinolin-2(1H)-yl]carbonyl}-1,3-benzodioxol-5-yl)-N-(1-méthyl-1H-pyrazolo[3,4-b]pyridin-5-yl)indolizine-1-carboxamide

### Stade A : N-[4-tert-Butyl(diméthyl)silyl]oxyphényl]-3-[6-[(3R)-3-méthyl-3,4-dihydro-1H-isoquinoline-2-carbonyl]-1,3-benzodioxol-5-yl]-N-(1-méthyl-1H-pyrazolo[3,4-b]pyridin-5-yl)indolizine-1-carboxamide

Le produit du titre est obtenu selon le procédé du Stade A de l'Exemple 86 en remplaçant le composé de la Préparation 36" par celui de la Préparation 35".
**LCMS :** [M+H]⁺ = 791.4 pour 791.3 calculé

### Stade B : N-(4-Hydroxyphényl)-3-(6-{[(3R)-3-méthyl-3,4-dihydroisoquinolin-2(1H)-yl]carbonyl}-1,3-benzodioxol-5yl)-N(1-méthyl-1H-pyrazolo[3,4-b]pyridin-5-yl) indolizine-1-carboxamide

On procède selon un protocole analogue à celui décrit au Stade D de l'Exemple 1. Le produit ainsi obtenu est soumis à une étape de salification en présence d'éther chlorhydrique.
***IR (ATR) cm⁻¹* :** 2500 à 3000 v-OH, 1614 ν >C=O amides, 1236 ν >C-O-C<, 740 γ >CH-Ar
***Microanalyse élémentaire : %mesuré (théorique)***
%C=71.07(70.99);%H=4.45(4.77);%N=12.37(12.42)
***Masse haute résolution (ESI*+*) :***
Formule brute : C₄₀H₃₂N₆O₅
[M+H]⁺ calculé : 677.2507
[M+H]⁺ mesuré : 677.2510

### Exemple 71. Chlorure de 4-((4-hydroxyphényl){[3-(6-{[(3R)-3-méthyl-3,4-dihydroisoquinolin-2(1H)-yl]carbonyl}-1,3-benzodioxol-5-yl)indolizin-1-yl)carbonyl} amino]-1-méthylpyridinium

### Stade A : Iodure de 4-[(4-hydroxyphényl){[3-(6-{[(3R)-3-méthyl-3,4-dihydroisoquinolin-2(1H)-yl]carbonyl}-1,3-benzodioxol-5-yl)indolizin-1-yl]carbonyl}amino]-1-méthyl pyridinium

Le composé de l'Exemple 21 (311 mg, 0,5 mmol) est mis en solution dans le dichlorométhane et lavé par une solution aqueuse saturée d'hydrogénocarbonate de sodium. Après séchage de la phase organique au sulfate de magnésium et évaporation à sec, le résidu est mis en solution dans l'éthanol (30 mL). L'iodure de méthyle (45 µL, 0,7 mmole) est ensuite ajouté et le milieu réactionnel est chauffé à 40°C. La solution ainsi obtenue est évaporée à sec. Le brut réactionnel est purifié sur colonne de gel de silice en utilisant le dichlorométhane et le méthanol comme solvants. Le composé est obtenu sous la forme d'une poudre blanche, qui est engagée directement dans l'étape suivante.
**RMN** ¹**H** (500 MHz, dmso-d6) δ ppm : 9.95 (sl, 1 H), 8.6-8.45 (m, 2 H), 8.35-8.05 (plusieurs m, 1 H), 8.3-8 (plusieurs m, 1 H), 7.45-6.7 (plusieurs m, 8 H), 7.4-6.9 (plusieurs m, 4 H), 6.45-6.3 (plusieurs s, 1 H), 6.45-6.3 (m, 2 H), 6.15 (s, 2 H), 5.05-3.55 (plusieurs d, 2 H), 4.75/3.8 (m+m, 1 H), 4.15 (2*s, 3 H), 2.95-2.1 (plusieurs m, 2 H), 1-0.15 (plusieurs m, 3 H)

### Stade B : Chlorure de 4-[4-hydroxyphényl){[3-(6-{[3R)-3-méthyl-3,4-dihydroisoquinolin-2(1H)yl]carbohyl}-1,3-benzodioxol-5-yl)indolizin-1-yl]carbonyl}amino]-1-méthyl pyridinium

Le composé du Stade précédent (320 mg, 0,42 mmole) est dissout dans le méthanol (20 mL), puis du carbonate d'argent (173 mg, 0,628 mmole) est ajouté par portions en 10 minutes. Cette suspension est agitée 1 heure à température ambiante ; le précipité est filtré et lavé avec du méthanol. Le filtrat est concentré à sec, puis traité par 50 mL d'une solution d'acide chlorhydrique 2N, chauffé à 60°C pendant 30 minutes puis évaporé à sec. Le produit final est obtenu après purification sur colonne de silice C18 en utilisant une solution d'acide chlorhydrique 0.1% et de l'acétonitrile comme solvants. Le composé du titre est obtenu sous la forme d'une poudre blanche qui est lyophilisée dans un mélange eau / acétonitrile.
***IR (ATR) cm⁻¹ :*** 3388 v -OH phénol, 1650 + 1627 ν >C=O amides
***Masse haute résolution (ESI*+*) :***
Formule brute : C₃₉H₃₃N₄O₅
[M]⁺ calculé = 637.2445.
[M]⁺ mesuré = 637.2431

**Les composés des Exemples 72, 73, 77, 78-80, 84 et 85 sont synthétisés selon le procédé de l'Exemple 3 en utilisant l'acide de la Préparation 7, la 1,2,3,4-tétrahydroisoquinoline ou le dérivé approprié obtenu selon l'une des Préparations 1' à 7', ainsi que l'amine NHR₃R₄ adéquate.**
**Exemple 72. *N*-(4-Hydroxyphényl)-*N*-méthyl-6-(6-{[(3*R*)-3-méthyl-3,4-dihydroisoquinolin-2(1*H*)-yl]carbonyl}-1,3-benzodioxol-5-yl)-1,2,3,4-tétrahydropyrrolo[1,2-*a*]pyrazine-8-carboxamide**
   **LC/MS** (C₃₃H₃₂N₄O₅) 565 [M+H]⁺; RT 1.47 (Méthode B) étant entendu que RT indique le temps de rétention
**Exemple 73. *N*-Éthyl-*N*-(4-hydroxyphényl)-6-(6-{[(3*R*)-3-méthyl-3,4-dihydroisoquinolin-2(1*H*)-yl]carbonyl}-1,3-benzodioxol-5-yl)-1,2,3,4-tétrahydropyrrolo[1,2-*a*]pyrazine-8-carboxamide**
   **LC/MS** (C₃₄H₃₄N₄O₅) 579 [M+H]⁺; RT 1.55 (Méthode B)
**Exemple 74. 3-[6-(3,4-Dihydroisoquinolin-2(1*H*)-ylcarbonyl)-1,3-benzodioxol-5-yl]-*N*-(4-hydroxyphényl)-*N*-méthyl-5,6,7,8-tétrahydroindolizine-1-carboxamide**
   **LC/MS** (C₃₃H₃₁N₃O₅) 550 [M+H]⁺; RT 1.24 (Méthode B)
**Exemple** 75. **3-[6-(3,4-Dihydroisoquinolin-2(1*H*)-ylcarbonyl)-1,3-benzodioxol-5-yl]-*N*-éthyl-*N*-(4-hydroxyphényl)-5,6,7,8-tétrahydroindolizine-1-carboxamide**
   **LC/MS** (C₃₄H₃₃N₃O₅) 564 [M+H]⁺; RT 1.30 (Méthode B)
**Exemple 76. *N*-Butyl-3-[6-(3,4-dihydroisoquinolin-2(1*H*)-ylcarbonyl)-1,3-benzodioxol-5-yl]-*N*-(4-hydroxyphényl)-5,6,7,8-tétrahydroindolizine-1-carboxamide**
   **LC/MS** (C₃₆H₃₇N₃O₅) 592 [M+H]⁺; RT 1.39 (Méthode B)
**Exemple 77. *N*-Éthyl-*N*-(4-hydroxyphényl)-6-(6-{[(3*S*)-3-méthyl-3,4-dihydroisoquinolin-2(1*H*)-yl]carbonyl}-1,3-benzodioxol-5-yl)-1,2,3,4-tétrahydropyrrolo[1,2-*a*]pyrazine-8-carboxamide**
   **LC/MS** (C₃₄H₃₄N₄O₅) 579 [M+H]⁺; RT 1.50 (Méthode B)
**Exemple 78. *N,N*-Dibutyl-6-(6-{[(3*R*)-3-méthyl-3,4-dihydroisoquinolin-2(1*H*)-yl]carbonyl}-1,3-benzodioxol-5-yl)-1,2,3,4-tétrahydropyrrolo[1,2-*a*]pyrazine-8-carboxamide**
   LC/MS (C₃₄H₄₂N₄O₄) 571 [M+H]⁺; RT 1.79 (Méthode B)
**Exemple 79. *N*-Butyl-*N*-(4-hydroxyphényl)-6-(6-{[(3*R*)-3-méthyl-3,4-dihydroisoquinolin-2(1*H*)-yl]carbonyl}-1,3-benzodioxol-5-yl)-1,2,3,4-tétrahydropyrrolol[1,2-*a*]pyrazine-8-carboxamide**
   LC/MS (C₃₆H₃₈N₄O₅) 607 [M+H]⁺; RT 1.65 (Méthode B)
**Exemple 80. *N*-(4-Hydroxyphényl)-6-(6-{[(3*R*)-3-méthyl-3,4-dihydroisoquinolin-2(1*H*)-yl]carbonyl}-1,3-benzodioxol-5-yl)-*N*-(propan-2-yl)-1,2,3,4-tétrahydropyrrolo[1,2-*a*]pyrazine-8-carboxamide**
   **LC/MS** (C₃₅H₃₆N₄O₅) 593 [M+H]⁺; RT 1.58 (Méthode B)
**Exemple 81. *N*-(4-Hydroxyphényl)-*N*-méthyl-3-(6-{[(3*R*)-3-méthyl-3,4-dihydroisoquinolin-2(1*H*)-yl]carbonyl}-1,3-benzodioxol-5-yl)-5,6,7,8-tétrahydroindolizine-1-carboxamide**
   LC/MS (C₃₄H₃₃N₃O₅) 564 [M+H]⁺; RT 2.48 (Méthode A)
**Exemple 82. *N*-(4-Hydroxyphényl)-*N*-méthyl-3-(6-{[(3*S*)-3-méthyl-3,4-dihydroisoquinolin-2(1*H*)-yl]carbonyl}-1,3-benzodioxol-5-yl)-5,6,7,8-tétrahydroindolizine-1-carboxamide**
   **LC/MS** (C₃₄H₃₃N₃O₅) 564 [M+H]⁺; RT 2.55 (Méthode A)
**Exemple 83. 3-[6-(3,4-Dihydroisoquinolin-2(1*H*)-ylcarbonyl)-1,3-benzodioxol-5-yl]-*N*-(4-hydroxyphényl)-*N*-méthylindolizine-1-carboxamide**
   **LC/MS** (C₃₃H₂₇N₃O₅) 546 [M+H]⁺; RT 2.40 (Méthode A)
**Exemple 84. 6-[6-(3,4-Dihydroisoquinolin-2(1*H*)-ylcarbonyl)-1,3-benzodioxol-5-yl]-*N*-(4-hydroxyphényl)-*N*-méthyl-1,2,3,4-tétrahydropyrrolo[1,2-*a*]pyrazine-8-carboxamide**
   **LC/MS** (C₃₂H₃₀N₄O₅) 551 [M+H]⁺; RT 1.45 (Méthode B)
**Exemple 85. 6-[6-(3,4-Dihydroisoquinolin-2(1*H*)-ylcarbonyl)-1,3-benzodioxol-5-yl]-*N*-éthyl-*N*-(4-hydroxyphényl)-1,2,3,4-tétrahydropyrrolo[1,2-*a*]pyrazine-8-carboxamide**
   **LC/MS** (C₃₃H₃₂N₄O₅) 565 [M+H]⁺; RT 1.49 (Méthode B)
**Exemple 86. Chlorhydrate de *N*-(4-hydroxyphényl)-3-[6-[(3*R*)-3-méthyl-3,4-dihydro-1*H*-isoquinoline-2-carbonyl]-1,3-benzodioxol-5-yl]-*N*-(3-méthylpyrazolo[1,5-*a*] pyrimidin-6-yl)indolizine-1-carboxamide**

### Stade A : N[4-[tert-burtyl(diméthyl)silyl]oxyphényl]-3-[6-[(3R)-3-méthyl-3,4-dihydro-1H-isoquinoline-2-carbonyl]-1,3-benzodioxol-5-yl]-N-(3-méthylpyrazolo[1,5-a]pyrimidin-6-yl)indolizine-1-carboxamide

A une solution de 0,6 g d'acide 3-(6-{[(3*R*)-3-méthyl-3,4-dihydroisoquinolin-2(1*H*)-yl]carbonyl}-1,3-benzodioxol-5-yl)indolizine-1-carboxylique (1,3 mmol) dans 6 mL de dichloroéthane est additionné 0,18 mL de 1-chloro-*N,N*,2-triméthyl-prop-1-èn-1-amine (2 mmol). Le milieu réactionnel est agité à température ambiante pendant 2 heures, puis on ajoute 0,8 g du composé de la Préparation 36" (2,2 mmol). L'ensemble est chauffé à reflux pendant 20 heures, puis refroidi et dilué dans un mélange de dichlorométhane et d'une solution saturée en NaHCO₃. Après décantation, la phase organique est séchée sur MgSO₄ et concentrée à sec Le produit brut ainsi obtenu est purifié par chromatographie sur gel de silice (gradient dichlorométhane / méthanol).
**LC/MS :** [M+H]⁺ = 791.4 pour 791.3 calculé

### Stade B : Chlorhydrate de N-(4-hydroxyphényl)-3-[6-[(3R)-3-méthyl-3,4-dihydro-1H-isoquinoline-2-carbonyl]-1,3-benzodioxol-5-yl]-N-(3-méthylpyrazolo[1,5-a]pyrimidin-6-yl)indolizine-1-carboxamide

On procède selon un protocole analogue à celui décrit au Stade D de l'Exemple 1. Le produit ainsi obtenu est soumis à une étape de salification en présence d'éther chlorhydrique.
***IR (ATR) cm⁻¹ :*** 2500 à 3000 ν -OH, 1614 ν >C=O amides, 1236 ν >C-O-C<,
740 γ >CH-Ar

### Masse haute résolution (ESI+) :

Formule brute : C₄₀H₃₂N₆O₅
[M+H]⁺ calculé : 677.2507
[M+H]⁺ mesuré : 677.2506

### ETUDE PHARMACOLOGIQUE

### EXEMPLE A : Inhibition de Bcl-2 par la technique de polarisation de fluorescence

Les essais de polarisation de fluorescence ont été réalisés en microplaques (384 puits). La protéine Bcl-2 étiquetée (histag-Bcl-2 tel que Bcl-2 correspond au numéro d'ordre primaire UniProtKB^{®} : P10415), à la concentration finale de 2,50.10⁻⁸M, est mélangée avec un peptide fluorescent (Fluorescein-REIGAQLRRMADDLNAQY), à la concentration finale de 1,00. 10⁻⁸M dans une solution tampon (Hepes 10 mM, NaCl 150 mM, Tween20 0,05%, pH 7,4), en présence ou en absence de concentrations croissantes de composés à tester. Après incubation de 2 heures, la polarisation de fluorescence est mesurée.
Les résultats sont exprimés en IC₅₀ (concentration en composé qui inhibe à 50 % la polarisation de fluorescence) et sont présentés dans le tableau 1 ci-dessous.

Les résultats montrent que les composés de l'invention inhibent l'interaction entre la protéine Bcl-2 et le peptide fluorescent décrit précédemment.

### EXEMPLE B : Cytotoxicité in vitro.

Les études de cytotoxicité ont été réalisées sur la lignée tumorale de leucémie RS4 ; 11. Les cellules sont réparties dans des microplaques et exposées aux composés à tester pendant 48 heures. La viabilité cellulaire est ensuite quantifiée par un essai colorimétrique, le Microculture Tétrazolium Assay (Cancer Res., 1987, 47, 939-942).
Les résultats sont exprimés en IC₅₀ (concentration en composé qui inhibe à 50 % la viabilité cellulaire) et sont présentés dans le tableau 1 ci-dessous.

Les résultats montrent que les composés de l'invention sont cytotoxiques.

**Tableau 1 : IC₅₀ d'inhibition de Bcl-2 (test de polarisation de fluorescence) et de cytotoxicité pour les cellules RS4 ; 11**

| | IC₅₀ (nM) Bcl-2 FP | IC₅₀ (nM) MTT RS4;11 | | IC₅₀ (nM) Bcl-2 FP | IC₅₀ (nM) MTT RS4;11 |
|---|---|---|---|---|---|
| **Exemple 1** | 17,9 | 11,3 | **Exemple 29** | 19,0 | 163 |
| **Exemple 2** | 17,0 | 36 | **Exemple 30** | 10,4 | 52,3 |
| **Exemple 3** | 33,6 | 66,5 | **Exemple 31** | 5,4 | 13,7 |
| **Exemple 4** | 56,4 | 251 | **Exemple 32** | 5,0 | 32,7 |
| **Exemple 5** | 55,9 | 416 | **Exemple 33** | 4,6 | 6,33 |
| **Exemple 6** | 60,3 | 161 | **Exemple 34** | 5,6 | 27,3 |
| **Exemple 7** | 46,4 | 108 | **Exemple 35** | 15,1 | 62,2 |
| **Exemple 8** | 24,5 | 20,5 | **Exemple 36** | 12,6 | 49,7 |
| **Exemple 9** | 40,6 | 780 | **Exemple 37** | 2,9 | 24,7 |
| **Exemple 10** | 24,7 | 439 | **Exemple 38** | 4,6 | 9,52 |
| **Exemple 11** | 10,9 | 83,7 | **Exemple 39** | 4,6 | 26,3 |
| **Exemple 12** | 10,4 | 116 | **Exemple 40** | 6,0 | 49 |
| **Exemple 13** | 5,8 | 33,65 | **Exemple 41** | 41,5 | 294 |
| **Exemple 14** | 3,7 | 7,6 | **Exemple 42** | 5,1 | 57,6 |
| **Exemple 15** | 5,7 | 166 | **Exemple 43** | 4,8 | 26 |
| **Exemple 16** | 7,5 | 252 | **Exemple 44** | 2,9 | 8,56 |
| **Exemple 17** | 3,4 | 11,8 | **Exemple 45** | 3,8 | 63,8 |
| **Exemple 18** | 7,5 | 47,7 | **Exemple 46** | 4,1 | 27,9 |
| **Exemple 19** | 8,0 | 235 | **Exemple 47** | 4,3 | 90,1 |
| **Exemple 20** | 11,1 | 205 | **Exemple 48** | 3,6 | 24,7 |
| **Exemple 21** | 4,6 | 25,3 | **Exemple 49** | 3,7 | 84,7 |
| **Exemple 22** | 12,9 | 263 | **Exemple 50** | 2,2 | 28,2 |
| **Exemple 23** | 3,8 | 9,99 | **Exemple 51** | 4,8 | 68,8 |
| **Exemple 24** | 6,2 | 28,4 | **Exemple 52** | 7,9 | 20,9 |
| **Exemple 25** | 7,9 | 30 | **Exemple 53** | 5,4 | 70,9 |
| **Exemple 26** | 16,6 | 300 | **Exemple 54** | 6,6 | 45 |
| **Exemple 27** | 7,7 | 44,1 | **Exemple 55** | 5,5 | 22,8 |
| **Exemple 28** | 8,8 | 112 | **Exemple 56** | 4,7 | 36,7 |
| **Exemple 57** | 21,2 | 282 | **Exemple 72** | 90,2 | 1520 |
| **Exemple 58** | 6,4 | 68,5 | **Exemple 73** | 83,6 | 1320 |
| **Exemple 59** | 4,0 | 21,2 | **Exemple 74** | 68,7 | 1340 |
| **Exemple 60** | 5,4 | 60,3 | **Exemple 75** | 67,7 | 1360 |
| **Exemple 61** | 7,0 | 61,3 | **Exemple 76** | 77,6 | 1630 |
| **Exemple 62** | 5,6 | 96,6 | **Exemple 77** | 25,1% @10 µM | 1880 |
| **Exemple 63** | 6,2 | 25,4 | **Exemple 78** | 823,3 | 1880 |
| **Exemple 64** | 7,8 | 282 | **Exemple 79** | 99,1 | 1010 |
| **Exemple 65** | 5,3 | 62,8 | **Exemple 80** | 299,3 | 1880 |
| **Exemple 66** | 4,7 | 42 | **Exemple 81** | 12,1 | 778 |
| **Exemple 67** | ND | ND | **Exemple 82** | 42% @10 µM | 1880 |
| **Exemple 68** | 8,3 | 82,4 | **Exemple 83** | 35,8 | 1500 |
| **Exemple 69** | 4,6 | 1,38 | **Exemple 84** | 524,9 | ND |
| **Exemple 70** | 5,2 | 6,17 | **Exemple 85** | 242,7 | ND |
| **Exemple 71** | 49 | ND | **Exemple 86** | 5 | 20,1 |

| | | | | | |
|---|---|---|---|---|---|
| ND : non déterminé | | | | | |

Pour les inhibiteurs partiels, le pourcentage d'inhibition de la polarisation de fluorescence pour une concentration donnée du composé testé est indiqué. Ainsi, 25.1% @10 µM signifie qu'on observe 25.1% d'inhibition de la polarisation de fluorescence pour une concentration de composé testé égale à 10 µM.

### EXEMPLE C : Induction de l'activité caspase in vivo.

La capacité des composés de l'invention à activer la caspase 3 est évaluée dans un modèle de xénogreffe de cellules leucémiques RS4 ; 11.
1.10⁷ cellules RS4 ; 11 sont greffées par voie sous-cutanée dans des souris immunodéprimées (souche SCID). 25 à 30 jours après la greffe, les animaux sont traités par voie orale par les différents composés. Seize heures après le traitement, les masses tumorales sont récupérées, lysées et l'activité caspase 3 est mesurée dans les lysats tumoraux.

Cette mesure enzymatique est réalisée en dosant l'apparition d'un produit de clivage fluorigénique (activité DEVDase, Promega). Elle est exprimée sous la forme d'un facteur d'activation correspondant au rapport entre les deux activités caspases : celle pour les souris traitées divisée par celle pour les souris contrôles.

Les résultats obtenus montrent que les composés de l'invention sont capables d'induire l'apoptose dans les cellules tumorales RS4 ; 11 *in vivo.*

### EXEMPLE D : Quantification de la forme clivée de la caspase 3 in vivo.

La capacité des composés de l'invention à activer la caspase 3 est évaluée dans un modèle de xénogreffe de cellules leucémiques RS4 ;11.
1.10⁷ cellules RS4 ;11 sont greffées par voie sous-cutanée dans des souris immunodéprimées (souche SCID). 25 à 30 jours après la greffe, les animaux sont traités par voie orale par les différents composés. Après le traitement, les masses tumorales sont récupérées (au bout d'un temps T), lysées et la forme clivée (activée) de la caspase 3 est quantifiée dans les lysats tumoraux.
Cette quantification est réalisée en utilisant l'essai « Meso Scale Discovery (MSD) ELISA platform » qui dose spécifiquement la forme clivée de la caspase 3. Elle est exprimée sous la forme d'un facteur d'activation correspondant au rapport entre la quantité de caspase 3 clivée chez les souris traitées divisée par la quantité de caspase 3 clivée chez les souris contrôles.

Les résultats montrent que les composés de l'invention sont capables d'induire l'apoptose dans les cellules tumorales RS4 ; 11 *in vivo.*

**Tableau 2 : Facteurs d'activation des caspases (caspase 3 clivée essai MSD dans les tumeurs des souris traitées versus souris contrôles) in vivo, après traitement par voie orale (doses précisées entre parenthèses)**

| Composé testé | Temps au bout duquel est prélevé la tumeur (T) | Facteur d'activation ± SEM (versus contrôle) |
|---|---|---|
| **Exemple 2** | 6 h | 14,6 (50 mg/kg) |
| **Exemple 13** | 2 h | 23,1 (50 mg/kg) |
| **Exemple 17** | 2 h | 15,3 (50 mg/kg) |
| **Exemple 21** | 2 h | 24,8 ± 1,4 (50 mg/kg) |
| **Exemple 32** | 2 h | 54,4 ± 2,8 (25 mg/kg) |
| **Exemple 33** | 2 h | 31,1 ± 10,8 (25 mg/kg) |
| **Exemple 38** | 2 h | 27,5 ± 2,6 (25 mg/kg) |
| **Exemple 39** | 2 h | 34,1 ± 2,4 (25 mg/kg) |
| **Exemple 42** | 2 h | 77,5 ± 4,8 (25 mg/kg) |
| **Exemple 50** | 2 h | 45,2 ± 3,9 (25 mg/kg) |
| **Exemple 56** | 2 h | 10,3 ± 4,2 (25 mg/kg) |

### EXEMPLE E : Activité anti-tumorale in vivo.

L'activité anti-tumorale des composés de l'invention est évaluée dans un modèle de xénogreffe de cellules leucémiques RS4 ; 11.
1.10⁷ cellules RS4 ;11 sont greffées par voie sous-cutanée dans des souris immunodéprimées (souche SCID). 25 à 30 jours après la greffe, lorsque la masse tumorale a atteint environ 150 mm³, les souris sont traitées par voie orale par les différents composés dans 2 schémas différents (traitement quotidien pendant cinq jours par semaine durant deux semaines, ou deux traitements par semaine pendant deux semaines). La masse tumorale est mesurée 2 fois par semaine depuis le début du traitement.

Les résultats obtenus montrent donc que les composés de l'invention sont capables d'induire une régression tumorale significative durant la période de traitement.

### EXEMPLE F : Composition pharmaceutique : Comprimés

| | |
|---|---|
| 1000 comprimés dosés à 5 mg d'un composé choisi parmi les exemples 1 à 86 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 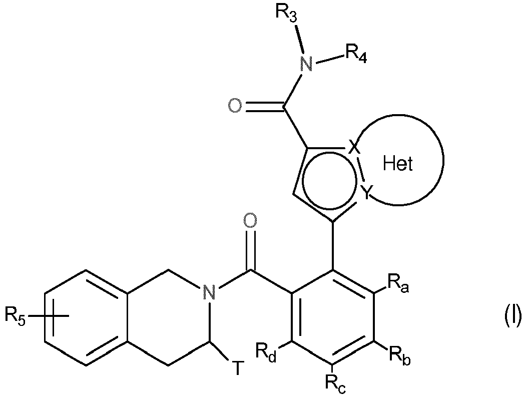 dans laquelle :
◆ X et Y représentent un atome de carbone ou un atome d'azote, étant entendu qu'ils ne peuvent représenter simultanément deux atomes de carbone ou deux atomes d'azote,
◆ la partie Het du groupe 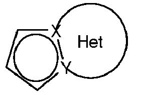 représente un cycle éventuellement substitué, aromatique ou non, constitué de 5, 6 ou 7 chaînons, et pouvant contenir, en plus de l'azote représenté par X ou par Y, un à 3 hétéroatomes choisis indépendamment parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié,
◆ T représente un atome d'hydrogène, un alkyle (C₁-C₆) linéaire ou ramifié éventuellement substitué par un à trois atomes d'halogène, un groupement alkyl(C₂-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié, ou bien R₁ et R₂ forment avec l'atome d'azote qui les porte un hétérocycloalkyle,
◆ R₃ représente un groupement alkyle (C₁-C₆) linéaire, aryle ou hétéroaryle, ces deux derniers groupements pouvant être substitués par un à trois groupements choisis parmi halogène, alkyle (C₁-C₆) linéaire ou ramifié, alkoxy (C₁-C₆) linéaire ou ramifié et cyano, étant entendu qu'un ou plusieurs atomes de carbone des groupements précédents, ou de leurs éventuels substituants, peut(vent) être deutéré(s),
◆ R₄ représente un groupement 4-hydroxyphényle, étant entendu qu'un ou plusieurs atomes de carbone du groupement précédent, ou de ses éventuels substituants, peut(vent) être deutéré(s),
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ et R_{d} représentent chacun un atome d'hydrogène et (R_{b},R_{c}) forment ensemble avec les atomes de carbone qui les portent un groupe 1,3-dioxolane ou un groupe 1,4-dioxane, ou bien Rₐ, R_{c} et R_{d} représentent chacun un atome d'hydrogène et R_{b} représente un hydrogène, un halogène, un méthyle ou un méthoxy, ou encore Rₐ, R_{b} et R_{d} représentent chacun un atome d'hydrogène et R_{c} représente un groupement hydroxy ou méthoxy,
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote, les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié, spiro (C₃-C₆), alkoxy (C₁-C₆) linéaire ou ramifié, (C₁-C₆)alkyl-S-, hydroxy, oxo (ou N-oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle, hétéroaryle, aryloxy, arylthio, cycloalkyle, hétérocycloalkyle éventuellement substitué par un ou plusieurs atomes d'halogène ou groupements alkyles, étant entendu que R' et R" représentent, indépendamment l'un de l'autre, un atome d'hydrogène ou un groupement alkyle (C₁-C₆) linéaire ou ramifié, la partie Het du groupe 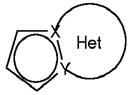 défini dans la formule (I) pouvant être substituée par un à trois groupements choisis parmi alkyle (C₁-C₆) linéaire ou ramifié, hydroxy, alkoxy (C₁-C₆) linéaire ou ramifié, NR₁'R₁", ou halogène, étant entendu que R₁' et R₁" ont les mêmes définitions que les groupes R' et R" mentionnés précédemment,
leurs énantiomères et diastéréoisomères, ainsi que leurs sels d'addition à un acide ou à une base pharmaceutiquement acceptable.

2. Composé de formule (I) selon la revendication 1 dans lequel le groupe 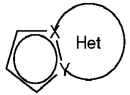 représente l'un des groupements suivants : 5,6,7,8-tétrahydroindolizine éventuellement substitué par un groupe amino ; indolizine ; 1,2,3,4-tétrahydropyrrolo[1,2-a]pyrazine éventuellement substitué par un méthyle ; pyrrolo[1,2-*a*]pyrimidine.

3. Composé de formule (I) selon la revendication 1 ou 2 dans lequel T représente un atome d'hydrogène, un groupement méthyle, un groupement 2-(morpholin-4-yl)éthyle, 3-(morpholin-4-yl)propyle, -CH₂-OH, 2-aminoéthyle, 2-(3,3-difluoropipéridin-1-yl)éthyle, 2-[(2,2-difluoroéthyl)amino]éthyle ou 2-(3-méthoxyazétidin-1-yl)éthyle.

4. Composé de formule (I) selon l'une des revendication 1 à 3 dans lequel R₃ représente un groupement hétéroaryle choisi parmi le groupe suivant : 1*H*-indole, 2,3-dihydro-1*H*-indole, 1*H*-indazole, pyridine, 1*H*-pyrrolo[2,3-*b*]pyridine, 1*H*-pyrazole, imidazo[1,2-*a*]pyridine, pyrazolo[1,5-*a*]pyrimidine, [1,2,4]triazolo[1,5-*a*]pyrimidine, 1*H-*pyrazolo[3,4-*b*]pyridine, tous pouvant être substitués un groupement alkyle (C₁-C₆) linéaire ou ramifié.

5. Composés de formule (I) selon la revendication 1 choisis parmi le groupe suivant :
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-{1-[2-(morpholin-4-yl)éthyl]-1*H*-indol-5-yl}-5,6,7,8-tétrahydroindolizine-1-carboxamide,
- *N*-(4-hydroxyphényl)-3-(6-{[(3*S*)-3-[2-(morpholin-4-yl)éthyl]-3,4-dihydroisoquinolin-2(1*H*)-yl]carbonyl}-1,3-benzodioxol-5-yl)-*N*-phényl-5,6,7,8-tétrahydroindolizine-1-carboxamide,
- *N*-{3-fluoro-4-[2-(morpholin-4-yl)éthoxy]phényl}-*N*-(4-hydroxyphényl)-3-(6-{[(3*R*)-3-méthyl-3,4-dihydroisoquinolin-2(1*H*)-yl]carbonyl}-1,3-benzodioxol-5-yl)indolizine-1-carboxamide,
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-(pyridin-4-yl)indolizine-1-carboxamide,
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-(2-méthylpyridin-4-yl)indolizine-1-carboxamide,
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-(1-méthyl-1*H*-pyrrolo[2,3-*b*]pyridin-5-yl)indolizine-1-carboxamide,
- *N*-(4-hydroxyphényl)-3-(6-{[(3*R*)-3-[3-(morpholin-4-yl)propyl]-3,4-dihydroisoquinolin-2(1*H*)-yl]carbonyl}-1,3-benzodioxol-5-yl)-*N*-phényl-5,6,7,8-tétrahydroindolizine-1-carboxamide,
- *N*-(2,6-diméthylpyridin-4-yl)-*N*-(4-hydroxyphényl)-3-(6-{[(3*R*)-3-méthyl-3,4-dihydroisoquinolin-2(1*H*)-yl]carbonyl}-1,3-benzodioxol-5-yl)indolizine-1-carboxamide,
- *N*-(4-hydroxyphényl)-3-(6-{[(3*R*)-3-méthyl-3,4-dihydroisoquinolin-2(1*H*)-yl]carbonyl}-1,3-benzodioxol-5-yl)-*N*-(pyridin-4-yl)-5,6,7,8-tétrahydroindolizine-1-carboxamide,
- 3-(5-Chloro-2-{[(3*R*)-3-méthyl-3,4-dihydroisoquinolin-2(1*H*)-yl]carbonyl}phényl)-*N*-(4-hydroxyphényl)-*N*-(1-méthyl-1*H*-pyrrolo[2,3-*b*]pyridin-5-yl)indolizine-1-carboxamide,
- *N*-(4-Hydroxyphényl)-*N*-(2-méthoxypyridin-4-yl)-3-(6-{[(3*R*)-3-méthyl-3,4-dihydroisoquinolin-2(1*H*)-yl]carbonyl}-1,3-benzodioxol-5-yl)indolizine-1-carboxamide,
leurs énantiomères et diastéréoisomères, ainsi que leurs sels d'addition à un acide ou à une base pharmaceutiquement acceptable.

6. Procédé de préparation des composés de formule (I) selon la revendication 1 **caractérisé en ce que** l'on utilise comme produit de départ le composé de formule (II) : 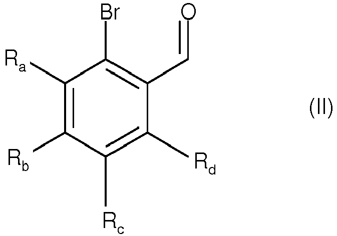 dans laquelle Rₐ, R_{b}, R_{c} et R_{d} sont tels que définis dans la formule (I),
composé de formule (II) qui est soumis à une réaction de Heck, en milieu aqueux ou organique, en présence d'un catalyseur au palladium, d'une base, d'une phosphine et du composé de formule (III) : 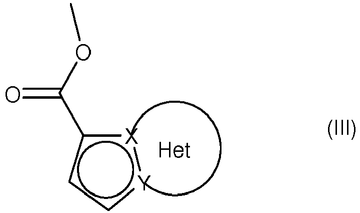 dans laquelle les groupements X, Y et Het sont tels que définis dans la formule (I), pour obtenir le composé de formule (IV) : 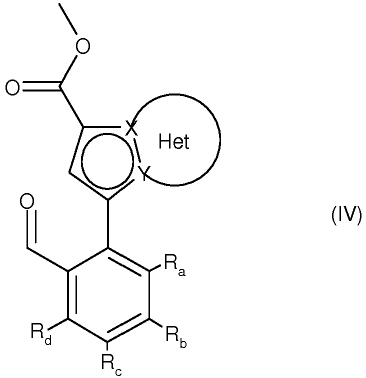 dans laquelle Rₐ, R_{b}, R_{c}, R_{d}, X, Y et Het sont tels que définis dans la formule (I),
composé de formule (IV) dont la fonction aldéhyde est oxydée en acide carboxylique pour former le composé de formule (V) : 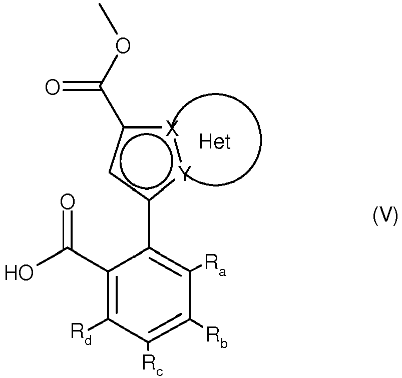 dans laquelle Rₐ, R_{b}, R_{c}, R_{d}, X, Y et Het sont tels que définis dans la formule (I),
composé de formule (V) qui subit ensuite un couplage peptidique avec un composé de formule (VI) : 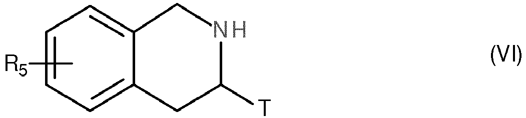 dans laquelle T et R₅ sont tels que définis dans la formule (I), pour conduire au composé de formule (VII) : 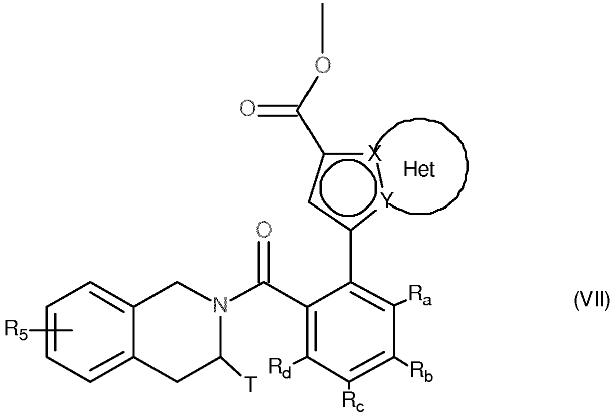 dans laquelle Rₐ, R_{b}, R_{c}, R_{d}, T, R₅, X, Y et Het sont tels que définis dans la formule (I),
composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

7. Procédé de préparation selon la revendication 6 d'un composé de formule (I) dans lequel l'un des groupements R₃ ou R₄ est substitué par une fonction hydroxy **caractérisé en ce que** l'amine NHR₃R₄ est préalablement soumise à une réaction de protection de la fonction hydroxy avant tout couplage avec l'acide carboxylique du composé de formule (VII), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

8. Composition pharmaceutique contenant un composé de formule (I) selon l'une quelconque des revendications 1 à 5 ou un de ses sels d'addition avec un acide ou une base pharmaceutiquement acceptable en combinaison avec un ou plusieurs excipients pharmaceutiquement acceptables.

9. Composition pharmaceutique selon la revendication 8 pour son utilisation en tant qu'agent pro-apoptotique.

10. Composition pharmaceutique selon la revendication 8 pour son utilisation dans le traitement des cancers, des maladies auto-immunes et du système immunitaire.

11. Composition pharmaceutique selon la revendication 8 pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

12. Composé de formule (I) selon l'une des revendications 1 à 5, ou un de ses sels d'addition avec un acide ou une base pharmaceutiquement acceptable, pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

13. Association d'un composé de formule (I) selon l'une quelconque des revendications 1 à 5 avec un agent anticancéreux choisi parmi les agents génotoxiques, les poisons mitotiques, les anti-métabolites, les inhibiteurs du protéasome, les inhibiteurs de kinases ou les anticorps.

14. Composition pharmaceutique contenant une association selon la revendication 13 en combinaison avec un ou plusieurs excipients pharmaceutiquement acceptables.

15. Association selon la revendication 13 pour son utilisation dans le traitement des cancers.

16. Composé de formule (I) selon l'une quelconque des revendications 1 à 5 pour son utilisation en association avec une radiothérapie dans le traitement des cancers.

## Patentansprüche

1. Verbindung der Formel (I): 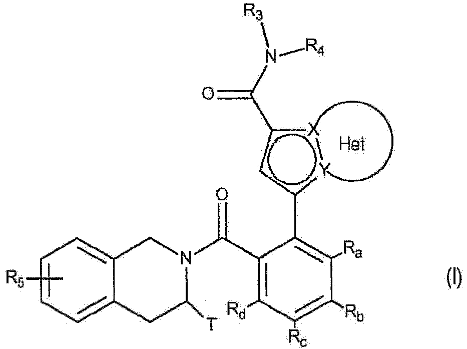 in der:
◆ X und Y ein Kohlenstoffatom oder ein Stickstoffatom bedeuten, wobei es sich versteht, dass sie nicht gleichzeitig zwei Kohlenstoffatome oder zwei Stickstoffatome darstellen können,
◆ der Rest Het der Gruppe 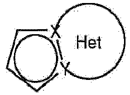 einen gegebenenfalls substituierten, aromatischen oder nichtaromatischen Ring mit 5, 6 oder 7 Kettengliedern bedeutet, der zusätzlich zu dem durch X oder Y dargestellten Stickstoff 1 bis 3 Heteroatome unabhängig voneinander ausgewählt aus Sauerstoff, Schwefel und Stickstoff enthalten kann, wobei es sich versteht, dass der in Rede stehende Stickstoff durch eine Gruppe substituiert sein kann, die ein Wasserstoffatom, eine geradkettige oder verzweigte (C₁-C₆)-Alkylgruppe oder eine Gruppe - C(O)-O-Alk, worin Alk eine geradkettige oder verzweigte (C₁-C₆)-Alkylgruppe darstellt, ist,
◆ T ein Wasserstoffatom, eine geradkettige oder verzweigte, gegebenenfalls durch ein bis drei Halogenatome substituierte (C₁-C₆)-Alkylgruppe, eine (C₂-C₄)-Alkyl-NR₁R₂-gruppe oder eine (C₁-C₄)-Alkyl-OR₆-gruppe bedeutet,
◆ R₁ und R₂ unabhängig voneinander ein Wasserstoffatom oder eine geradkettige oder verzweigte (C₁-C₆)-Alkylgruppe bedeuten,
oder R₁ und R₂ zusammen mit dem sie tragenden Stickstoffatom eine Heterocycloalkylgruppe bilden,
◆ R₃ eine geradkettige (C₁-C₆)-Alkylgruppe, Arylgruppe oder Heteroarylgruppe bedeutet, wobei die beiden letzteren Gruppen durch eine bis drei Gruppen ausgewählt aus Halogen, geradkettigem oder verzweigtem (C₁-C₆)-Alkyl, geradkettigem oder verzweigtem (C₁-C₆)-Alkoxy und Cyano substituiert sein können, wobei es sich versteht, dass eines oder mehrere der vorstehenden Kohlenstoffatome oder ihrer eventuellen Substituenten deuteriert sein kann (können),
◆ R₄ eine 4-Hydroxyphenylgruppe bedeutet, wobei es sich versteht, dass eines oder mehrere Kohlenstoffatome der vorstehenden Gruppe oder ihrer eventuellen Substituenten deuteriert sein kann (können),
◆ R₅ ein Wasserstoffatom oder ein Halogenatom, eine geradkettige oder verzweigte (C₁-C₆)-Alkylgruppe oder eine geradkettige oder verzweigte (C₁-C₆)-Alkoxygruppe bedeutet,
◆ R₆ ein Wasserstoffatom oder eine geradkettige oder verzweigte (C₁-C₆)-Alkylgruppe bedeutet,
◆ Rₐ und R_{d} jeweils ein Wasserstoffatom bedeuten und (R_{b}, R_{c}) gemeinsam mit den sie tragenden Kohlenstoffatomen eine 1,3-Dioxolangruppe oder eine 1,4-Dioxangruppe bilden oder Rₐ, R_{c} und R_{d} jeweils ein Wasserstoffatom bedeuten und R_{b} Wasserstoff, Halogen, Methyl oder Methoxy bedeutet, oder Rₐ, R_{b} und R_{d} jeweils ein Wasserstoffatom bedeuten und R_{c} eine Hydroxy- oder Methoxygruppe darstellt,
wobei es sich versteht, dass man:
- unter "Aryl" eine Phenyl-, Naphthyl-, Biphenyl- oder Indenylgruppe versteht,
- unter "Heteroaryl" jede mono- oder bicyclische Gruppe aus 5 bis 10 Kettengliedern, die mindestens einen aromatischen Teil aufweist und 1 bis 4 Heteroatome ausgewählt aus Sauerstoff, Schwefel oder Stickstoff (einschließlich quaternären Stickstoff) enthält, versteht,
- unter "Cycloalkyl" jede carbocyclische, nichtaromatische, mono- oder bicyclische Gruppe mit 3 bis 10 Kettengliedern versteht,
- unter "Heterocycloalkyl" jede nichtaromatische, mono- oder bicyclische, kondensierte oder spiro-gebundene, aus 3 bis 10 Kettengliedern gebildete Gruppe versteht, die 1 bis 3 Heteroatome ausgewählt aus Sauerstoff, Schwefel, SO, SO₂ oder Stickstoff enthält,
wobei die in dieser Weise definierten Aryl-, Heteroaryl-, Cycloalkyl- und Heterocycloalkylgruppen und die Alkyl-, Alkenyl-, Alkinyl- und Alkoxygruppen durch 1 bis 3 Gruppen substituiert sein können, ausgewählt aus geradkettigem oder verzweigtem (C₁-C₆)-Alkyl, (C₃-C₆)-Spiro, geradkettigem oder verzweigtem (C₁-C₆)-Alkoxy, (C₁-C₆)-Alkyl-S-, Hydroxy, Oxo (oder gegebenenfalls *N*-Oxid), Nitro, Cyano, -COOR',-OCOR', NR'R", geradkettigem oder verzweigtem (C₁-C₆)-Polyhalogenalkyl, Trifluormethoxy, (C₁-C₆)-Alkylsulfonyl, Halogen, Aryl, Heteroaryl, Aryloxy, Arylthio, Cycloalkyl, Heterocycloalkyl gegebenenfalls substituiert durch ein oder mehrere Halogenatome oder Alkylgruppen, wobei es sich versteht, dass R' und R" unabhängig voneinander ein Wasserstoffatom oder eine geradkettige oder verzweigte (C₁-C₆)-Alkylgruppe darstellen,
wobei der Rest Het der bezüglich der Formel (I) definierten Gruppen 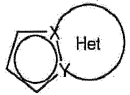 durch eine bis drei Gruppen ausgewählt aus geradkettigem oder verzweigtem (C₁-C₆)-Alkyl, Hydroxy, geradkettigem oder verzweigtem (C₁-C₆)-Alkoxy, NR₁'R₁" oder Halogen substituiert sein kann, wobei es sich versteht, dass R₁' und R₁" die gleichen Bedeutungen besitzen wie die oben erwähnten Gruppen R' und R",
deren Enantiomere und Diastereoisomere sowie deren Additionssalze mit einer pharmazeutisch annehmbaren Säure oder Base.

2. Verbindung der Formel (I) nach Anspruch 1, worin die Gruppe 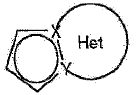 eine der folgenden Gruppen darstellt: gegebenenfalls durch eine Aminogruppe substituiertes 5,6,7,8-Tetrahydroindolizin; Indolizin; gegebenenfalls durch eine Methylgruppe substituiertes 1,2,3,4-Tetrahydropyrrolo[1,2-a]pyrazin; Pyrrolo[1,2-a]pyrimidin.

3. Verbindung der Formel (I) nach Anspruch 1 oder 2, worin T ein Wasserstoffatom, eine Methylgruppe, eine 2-(Morpholin-4-yl)-ethylgruppe, 3-(Morpholin-4-yl)-propylgruppe, eine Gruppe -CH₂-OH, 2-Aminoethylgruppe, 2-(3,3-Difluorpiperidin-1-yl)-ethylgruppe, 2-[(2,2-Difluorethyl)-amino]-ethylgruppe oder 2-(3-Methoxy-azetidin-1-yl)-ethylgruppe bedeutet.

4. Verbindung der Formel (I) nach einem der Ansprüche 1 bis 3, worin R₃ eine Heteroarylgruppe ausgewählt aus der folgenden Gruppe bedeutet: 1*H-*Indol, 2,3-Dihydro-1*H*-indol, 1*H-*Indazol, Pyridin, 1*H*-Pyrrolo[2,3-b]pyridin, 1*H-*Pyrazol, Imidazo[1,2-*a*]pyridin, Pyrazolo[1,5-*a*]pyrimidin, [1,2,4]Triazolo[1,5-*a*]pyrimidin, 1*H-*Pyrazolo[3,4-*b*]pyridin, die sämtlich durch eine geradkettige oder verzweigte (C₁-C₆)-Alkylgruppe substituiert sein können.

5. Verbindungen der Formel (I) nach Anspruch 1 ausgewählt aus der folgenden Gruppe:
- N-(4-Hydroxyphenyl)-3-(6-{[(3R)-3-methyl-3,4-dihydroisochinolin-2(1*H*)-yl]-carbonyl}-1,3-benzodioxol-5-yl)-*N-*{1-[2-(morpholin-4-yl)-ethyl]-1*H*-indol-5-yl}-5,6,7,8-tetrahydroindolizin-1-carboxamid,
- *N-*(4-Hydroxyphenyl)-3-(6-{[(3*S*)-3-[2-(morpholin-4-yl)-ethyl]-3,4-dihydro-isochinolin-2(1*H*)-yl]-carbonyl}-1,3-benzodioxol-5-yl)-*N*-phenyl-5,6,7,8-tetrahydro-indolizin-1-carboxamid,
- *N*-{3-Fluor-4-[2-(morpholin-4-yl)-ethoxy]-phenyl}-*N*-(4-hydroxyphenyl)-3-(6-{[(3*R*)-3-methyl-3,4-dihydroisochinolin-2(1*H*)-yl]-carbonyl}-1,3-benzodioxol-5-yl)-indolizin-1-carboxamid,
- *N-*(4-Hydroxyphenyl)-3-(6-{[(3*R*)-3-methyl-3,4-dihydroisochinolin-2(1*H*-yl]-carbonyl}-1,3-benzodioxol-5-yl)-*N-*(pyridin-4-yl)-indalizin-1-carboxamid,
- *N-*(4-Hydroxyphenyl)-3-(6-{[(3*R*)*-*3-methyl-3,4-dihydroisochinolin-2(1*H*)-yl-[carbonyl}-1,3-benzodioxol-5-yl)-*N-*(2-methylpyridin-4-yl)-indalizin-1-carboxamid,
- *N*-(4-Hydroxyphenyl)-3-(6-{[(3*R*)-3-methyl-3,4-dihydroisoehinolin-2(1*H*)-yl]-carbonyl}-1,3-benzodioxol-5-yl)-*N-*(1-methyl-1*H-*pyrrolo[2,3-*b*]pyrldin-5-yl)-indolizin-1-carboxamid,
- *N*-(4-Hydroxyphenyl)-3-(6-{[(3*R*)-3-[3-(morpholin-4-yl)-propyl]-3,4-dihydro-isochinolin-2(1*H*)-yl]-carbonyl}-1,3-benzodioxol-5-yl)-*N*-phenyl-5,6,7,8-tetrahydro-indolizin-1-carboxamid,
- *N*-(2,6-Dimethylpyridin-4-yl)-*N-*(4-hydroxyphenyl)-3-(6-{[(3*R*)-3-methyl-3,4-dihydroisochinolin-2(1*H*)-yl]-carbonyl}-1,3-benzodioxol-5-yl)-indolizin-1-carboxamid,
- *N-*(4-Hydroyphenyl)-3-(6-{[(3*R*)-3-methyl-3,4-dihydroisochinolin-2(1*H*-yl]-carbonyl}-1,3-benzodioxol-5-yl)-*N*-(pyridin-4-yl)-5,6,7,8-tetrahydroindolizin-1-carboxamid,
- 3-(5-Chlor-2-{[(3*R*)-3-methyl-3,4-dihydroisochinolin-2(1*H*)-yl]-carbonyl}-phenyl)-*N*-(4-hydroxyphenyl)-*N*-(1-methyl-1*H*-pyrrolo[2,3-*b*]pyridin-5-yl)-indolizin-1-carboxamid,
- *N*-(4-Hydroxyphenyl)-*N*-(2-methoxypyridin-4-yl)-3-(6-{[(3*R*)-3-methyl-3,4-dihydroisochinolin-2(1*H*)-yl]-carbonyl}-1,3-benzodioxol-5-yl)-indolizin-1-carboxamid,
deren Enantiomere und Diastereoisomere sowie deren Additionssalze mit einer pharmazeutisch annehmbaren Säure oder Base.

6. Verfahren zur Herstellung der Verbindungen der Formel (I) nach Anspruch 1, **dadurch gekennzeichnet, dass** man als Ausgangsprodukt die Verbindung der Formel (II) verwendet: 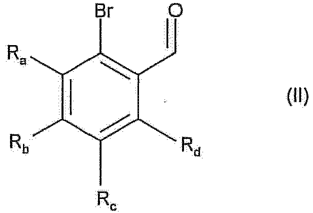 in der Rₐ, R_{b}, Rₑ und R_{d} die bezüglich der Formel (I) angegebenen Bedeutungen besitzen,
welche Verbindung der Formel (II) einer Heck-Reaktion in wässrig oder organischem Medium in Gegenwart eines Palladiumkatalysators, einer Base, von Phosphin und der Verbindung der Formel (III): 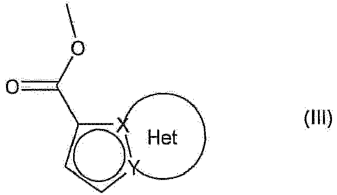 in der die Gruppen X, Y und Het die bezüglich der Formel (I) angegebenen Bedeutungen besitzen, unterworfen wird
zur Bildung der Verbindung der Formel (IV): 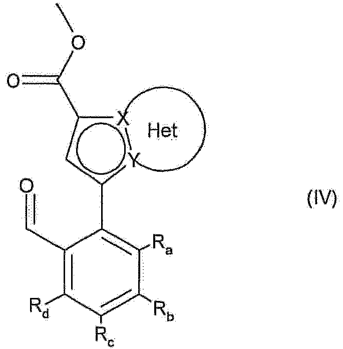 in der Rₐ, R_{b}, R_{c}, R_{d}, X, Y und Het die bezüglich der Formel (I) angegebenen Bedeutungen besitzen,
die Aldehydfunktion der Verbindung der Formel (IV) zu der Carbonsäure oxidiert wird zur Bildung der Verbindung der Formel (V): 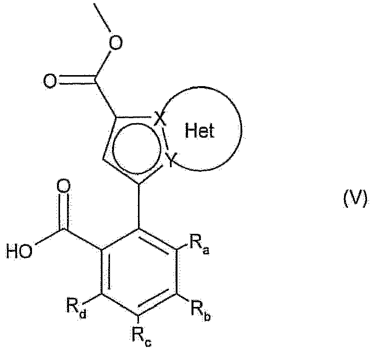 in der Rₐ, R_{b}, Rₑ, R_{d}, X, Y und Het die bezüglich der Formel (I) angegebenen Bedeutungen besitzen,
welche Verbindung der Formel (V) anschließend einer Peptidkupplung mit einer Verbindung der Formel (VI): 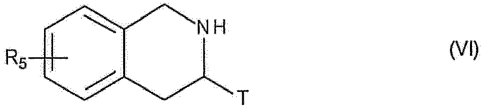 in der T und R₅ die bezüglich der Formel (I) angegebenen Bedeutungen besitzen, unterworfen wird zur Bildung der Verbindung der Formel (VII): 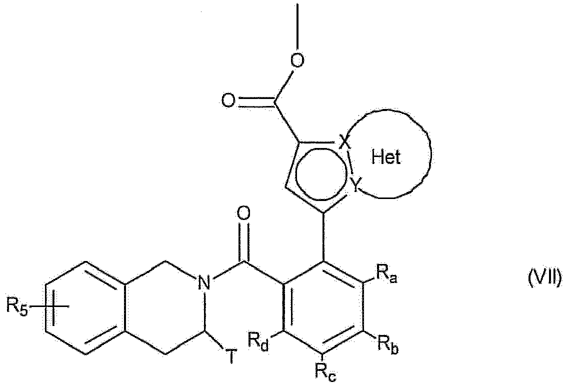 in der Rₐ, R_{b}, R_{c}, R_{d}, T, R₅, X, Y und Het die bezüglich der Formel (I) angegebenen Bedeutungen besitzen,
die Esterfunktion der Verbindung der Formel (VII) zu der entsprechenden Carbonsäure oder dem entsprechenden Carboxylat hydrolysiert wird, welche(s) vor der Kupplung mit einem Amin NHR₃R₄, worin R₃ und R₄ die gleiche Bedeutung wie bezüglich der Formel (I) besitzen, in ein Säurederivat, wie das entsprechende Acylchlorid oder Anhydrid umgewandet werden kann,
welche Verbindung der Formel (I) mit Hilfe einer klassischen Trennmethode gereinigt werden kann, welche gewünschtenfalls in ihre Additionssalze mit einer phannazeutisch annehmbaren Säure oder Base umgewandelt werden kann und die gegebenenfalls mit Hilfe einer klassischen Trennmethode in ihre Isomeren getrennt werden kann, wobei es sich versteht, dass zu jedem geeigneten Zeitpunkt im Verlaufe des oben beschriebenen Verfahrens bestimmte Gruppen (Hydroxy, Amino ...) der Reaktionsteilnehmer oder Zwischenprodukte der Synthese für die Zwecke der Synthese geschützt und dann wieder von den Schutzgruppen befreit werden können.

7. Verfahren zur Herstellung gemäß Anspruch 6 einer Verbindung der Formel (I), in der eine der Gruppen R₃ oder R₄ durch eine Hydroxygruppe substituiert ist, **dadurch gekennzeichnet, dass** das Amin NHR₃R₄ vor jeder Kupplung mit der Carbonsäure der Verbindung der Formel (VII) oder mit einem der entsprechenden Säurederivat einer Reaktion zum Schutz der Hydroxyfunktion unterworfen wird, wonach die erhaltene geschützte Verbindung der Formel (I) einer Reaktion zur Abspaltung der Schutzgruppe unterworfen wird und dann gegebenenfalls in eines ihrer Additionssalze mit einer pharmazeutisch annehmbaren Säure oder Base umgewandelt wird.

8. Pharmazeutische Zubereitung enthaltend eine Verbindung der Formel (I) nach einem der Ansprüche 1 bis 5 oder eines ihrer Additionssalze mit einer pharmazeutisch annehmbaren Säure oder Base in Kombination mit einem oder mehreren phannazeutisch annehmbaren Hilfsstoffen.

9. Phannazeutische Zubereitung nach Anspruch 8 zur Verwendung als proapoptotisches Mittel.

10. Pharmazeutische Zubereitung nach Anspruch 8 zur Verwendung bei der Behandlung von Krebs, Autoimmunerkrankungen und Erkrankungen des Immunsystems.

11. Pharmazeutische Zubereitung nach Anspruch 8 zur Verwendung bei der Behandlung von Blasenkrebs, Gehirnkrebs, Brustkrebs, Uteruskrebs, chronischlymphatischen Leukämien, Dannkrebs, Kolorektalkrebs, Speiseröhrenkrebs, Leberkrebs, lymphoblastischen Leukämien, Non-Hodgkin-Lymphomen, Melanomen, malignen Hämopathien, Myelomen, Ovarialkrebs, nicht-kleinzelligem Lungenkrebs, Prostatakrebs und kleinzelligem Lungenkrebs.

12. Verbindung der Formel (I) nach einem der Ansprüche 1 bis 5 oder eines ihrer Additionssalze mit einer pharmazeutisch annehmbaren Säure oder Base zur Verwendung bei der Behandlung von Blasenkrebs, Gehirnkrebs, Brustkrebs, Uteruskrebs, chronisch-lynphatischen Leukämien, Darmkrebs, Kolorektalkrebs, Speiseröhrenkrebs, Leberkrebs, lymphoblastischen Leukämien, Non-Hodgkin-Lymphomen, Melanomen, malignen Hämopathien, Myelomen, Ovarialkrebs, nicht-kleinzelligem Lungenkrebs, Prostatakrebs und kleinzelligem Lungenkrebs.

13. Kombination einer Verbindung der Formel (I) nach einem der Ansprüche 1 bis 5 mit einem Antikrebsmittel ausgewählt aus genotoxischen Mitteln, mitotischen Gif ten, Antimetaboliten, Proteasominhibitoren, Kineaseinhibitoren oder Antikörpern.

14. Pharmazeutische Zubereitung enthaltend eine Kombination nach Anspruch 13 in Kombination mit einem oder mehreren pharmazeutisch annehmbaren Hilfsstoffen.

15. Kombination nach Anspruch 13 zur Verwendung bei der Behandlung von Krebs.

16. Verbindung der Formel (I) nach einem der Ansprüche 1 bis 5 zur Verwendung in Kombination mit einer Strahlentherapie bei der Behandlung von Krebs.

## Claims

1. Compound of formula (I): 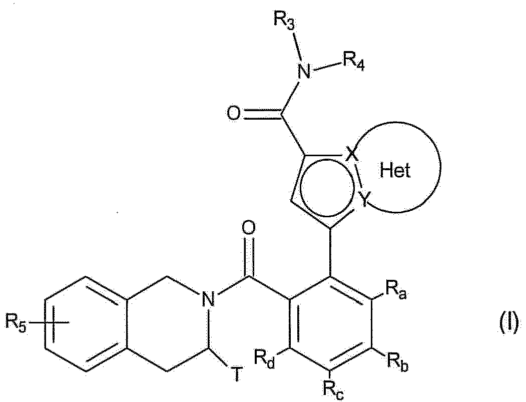 wherein:
◆ X and Y represent a carbon atom or a nitrogen atom, it being understood that they may not simultaneously represent two carbons atoms or two nitrogen atoms,
◆ the Het moiety of the group 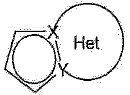 represents an optionally substituted, aromatic or non-aromatic ring composed of 5, 6 or 7 ring members, which may contain, in addition to the nitrogen represented by X or by Y, from one to 3 hetero atoms selected independently from oxygen, sulphur and nitrogen, it being understood that the nitrogen in question may be substituted by a group representing a hydrogen atom, a linear or branched (C₁-C₆)alkyl group or a group -C(0)-0-Alk wherein Alk is a linear or branched (C₁-C₆)alkyl group,
◆ T represents a hydrogen atom, a linear or branched (C₁-C₆)alkyl group optionally substituted by from one to three halogen atoms, a group (C₂-C₄)alkyl-NR₁R₂, or a group (C₁-C₄)alkyl-OR₆,
◆ R₁ and R₂ independently of one another represent a hydrogen atom or a linear or branched (C₁-C₆)alkyl group, or R₁ and R₂ form with the nitrogen atom carrying them a heterocycloalkyl,
◆ R₃ represents a linear (C₁-C₆)alkyl group, an aryl group or a heteroaryl group, it being possible for the last two groups to be substituted by from one to three groups selected from halogen, linear or branched (C₁-C₆)alkyl, linear or branched (C₁-C₆)alkoxy and cyano, it being understood that one or more of the carbon atoms of the preceding groups, or of their possible substituents, may be deuterated,
◆ R₄ represents a 4-hydroxyphenyl group, it being understood that one or more of the carbon atoms of the preceding group, or of its possible substituents, may be deuterated,
◆ R₅ represents a hydrogen or halogen atom, a linear or branched (C₁-C₆)alkyl group, or a linear or branched (C₁-C₆)alkoxy group,
◆ R₆ represents a hydrogen atom or a linear or branched (C₁-C₆)alkyl group,
◆ Rₐ and R_{d} each represent a hydrogen atom and (R_{b},R_{c}) form together with the carbon atoms carrying them a 1,3-dioxolane group or a 1,4-dioxane group, or Rₐ, R_{c} and R_{d} each represent a hydrogen atom and R_{b} represents a hydrogen, a halogen, a methyl or a methoxy, or Rₐ, R_{b} and R_{d} each represent a hydrogen atom and R_{c} represents a hydroxy group or a methoxy group,
it being understood that:
- "aryl" means a phenyl, naphthyl, biphenyl or indenyl group,
- "heteroaryl" means any mono- or bi-cyclic group composed of from 5 to 10 ring members, having at least one aromatic moiety and containing from 1 to 4 hetero atoms selected from oxygen, sulphur and nitrogen (including quaternary nitrogens),
- "cycloalkyl" means any mono- or bi-cyclic, non-aromatic, carbocyclic group containing from 3 to 10 ring members,
- "heterocycloalkyl" means any mono- or bi-cyclic, non-aromatic, condensed or spiro group composed of 3 to 10 ring members and containing from 1 to 3 hetero atoms selected from oxygen, sulphur, SO, SO₂ and nitrogen,
it being possible for the aryl, heteroaryl, cycloalkyl and heterocycloalkyl groups so defined and the groups alkyl, alkenyl, alkynyl and alkoxy to be substituted by from 1 to 3 groups selected from linear or branched (C₁-C₆)alkyl, (C₃-C₆)spiro, linear or branched (C₁-C₆)alkoxy, (C₁-C₆)alkyl-S-, hydroxy, oxo (or *N*-oxide where appropriate), nitro, cyano, -COOR', -OCOR', NR'R", linear or branched (C₁-C₆)polyhaloalkyl, trifluorornethoxy, (C₁-C₆)alkylsulphonyl, halogen, aryl, heteroaryl, aryloxy, arylthio, cycloalkyl, heterocycloalkyl optionally substituted by one or more halogen atoms or alkyl groups, it being understood that R' and R", each independently of the other, represent a hydrogen atom or a linear or branched (C₁-C₆)alkyl group, it being possible for the Het moiety of the group 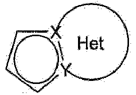 defined in formula (I) to be substituted by from one to three groups selected from linear or branched (C₁-C₆)alkyl, hydroxy, linear or branched (C₁-C₆)alkoxy, NR₁'R₁" and halogen, it being understood that R₁' and R₁" are as defined for the groups R' and R" mentioned hereinbefore,
their enantiomers and diastereoisomers, and addition salts thereof with a pharmaceutically acceptable acid or base.

2. Compound of formula (I) according to claim 1, wherein the group 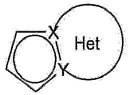 represents one of the following groups: 5,6,7,8-tetrahydroindolizine optionally substituted by an amino group; indolizine; 1,2,3,4-tetrahydropyrrolo[1,2-*a*]pyrazine optionally substituted by a methyl; pyrrolo[1,2-*a*]pyrimidine.

3. Compound of formula (I) according to claim 1 or 2, wherein T represents a hydrogen atom, a methyl group, a group 2-(morpholin-4-yl)ethyl, 3-(morpholin-4-yl)propyl, -CH₂-OH, 2-aminoethyl, 2-(3,3-difluoropiperidin-1-yl)ethyl, 2-[(2,2-difluoroethyl)amino]ethyl or 2-(3-methoxyazetidin-1-yl)ethyl.

4. Compound of formula (I) according to one of claims 1 to 3, wherein R₃ represents a heteroaryl group selected from the following group: 1*H-*indole, 2,3-dihydro-1*H-*indole, 1*H-*indazole, pyridine, 1*H-*pyrrolo[2,3-*b*]pyridine, 1*H-*pyrazole, imidazo[1,2-*a*]pyridine, pyrazolo[1,5-*a*]pyrimidine, [1,2,4]triazolo[1,5-*a*]pyrimidine, and 1*H*-pyrazolo-[3,4-*b*]pyridine, all of which may be substituted by a linear or branched (C₁-C₆)alkyl group.

5. Compounds of formula (I) according to claim 1, selected from the following group:
- *N*-(4-hydroxyphenyl)-3-(6-{[(3*R*)-3-methyl-3,4-dihydroisoquinolin-2(1*H*)-yl]-carbonyl}-1,3-benzodioxol-5-yl)-*N-*{1-[2-(morpholin-4-yl)ethyl]-1*H-*indol-5-yl}-5,6,7,8-tetrahydroindolizine-1-carboxamide,
- *N*-(4-hydroxyphenyl)-3-(6-{[(3*S*)-3-[2-(moipholin-4-yl)ethyl]-3,4-dihydroiso-quinolin-2(1*H*)-yl]carbonyl}-1,3-benzodioxol-5-yl)-*N*-phenyl-5,6,7,8-tetrahydroindolizine-1-carboxamide,
- *N-*{3-fluoro-4-[2-(morpholin-4-yl)ethoxy]phenyl}-*N-*(4-hydroxyphenyl)-3-(6-{[(3*R*)-3-methyl-3,4-dihydroisoquinolin-2(1*H*)-yl]carbonyl}-1,3-benzodioxol-5-yl)indolizine-1-carboxamide,
- *N*-(4-hydroxyphenyl)-3-(6-{[(3*R*)-3-methyl-3,4-dihydroisoquinolin-2(1*H*)-yl]carbonyl}-1,3-benzodioxol-5-yl)-*N*-(pyridin-4-yl)indolizine-1-carboxamide,
- *N*-(4-hydroxyphenyl)-3-(6-{[(3*R*)-3-methyl-3,4-dihydroisoquinolin-2(1*H*)-yl]-carbonyl}-1,3 benzodioxol-5-yl)-*N-*(2-methylpyridin-4-yl)indolizine-1-carboxamide,
- *N*-(4-hydroxyphenyl)-3-(6-{[(3*R*)-3-methyl-3,4-dihydroisoquinolin-2(1*H*)-yl]-carbonyl}-1,3-benzodioxol-5-yl)-*N-*(1-methyl-1*H-*pynolo[2,3-*b*]pyridin-5-yl)-indolizine-1-carboxamide,
- *N-*(4-hydroxyphenyl)-3-(6-{[(3*R*)-3-[3-(morpholin-4-yl)propyl]-3,4-dihydro-isoquinolin-2(1*H*)-yl]carbonyl}-1,3-benzodioxol-5-yl)-*N-*phenyl-5,6,7,8-tetrahydroindolizine-1-carboxamide,
- *N-*(2,6-dimethylpyridin-4-yl)-*N-*(4-hydroxyphenyl)-3-(6-{[(3*R*)-3-methyl-3,4-dihydroisoquinolin-2(1*H*)-yl]carbonyl}-1,3-benzodioxol-5-yl)indolizine-1-carboxamide,
- *N*-(4-hydroxyphenyl)-3-(6-{[(3*R*)-3-methyl-3,4-dihydroisoquinolin-2(1*H*)-yl]-carbonyl}-1,3-benzodioxol-5-yl)-*N-*(pyridin-4-yl)-5,6,7,8-tetrahydroindolizine-1-carboxamide,
- 3-(5-chloro-2-{[(3*R*}-3-methyl-3,4-dihydroisoquinolin-2(1*H)*-yl]carbonyl}phenyl)-*N*-(4-hydroxyphenyl)-*N*-(1-methyl-1*H-*pyrrolo[2,3-*b*]pyridin-5-yl)indolizine-1-carboxamide,
- *N*-(4-hydroxyphenyl)-*N*-(2-methoxypyridin-4-yl)-3-(6-{[(3*R*)-3-methyl-3,4-dihydroisoquinolin-2(1*H*)-yl]carbonyl}-1,3-benzodioxol-5-yl)indolizine-1-carboxamide,
their enantiomers and diastereoisomers, and addition salts thereof with a pharmaceutically acceptable acid or base.

6. Process for the preparation of compounds of formula (I) according to claim 1, **characterised in that** there is used as starting material the compound of formula (II): 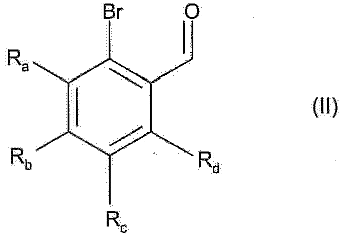 wherein Rₐ, R_{b}, R_{c} and R_{d} are as defined for formula (I),
which compound of formula (II) is subjected to a Heck reaction, in an aqueous or organic medium, in the presence of a palladium catalyst, of a base, of a phosphine and of the compound of formula (III): 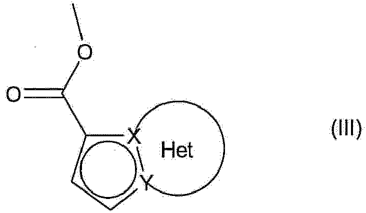 wherein the groups X, Y and Het are as defined for formula (I), to obtain the compound of formula (IV): 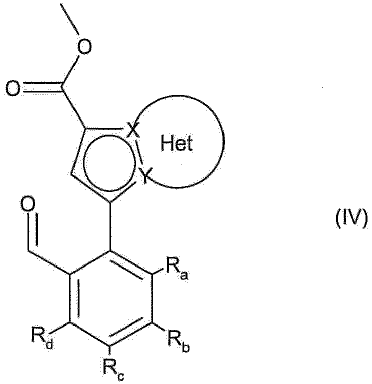 wherein Rₐ, R_{b}, R_{c}, R_{d}, X, Y and Het are as defined for formula (I),
the aldehyde function of which compound of formula (IV) is oxidised to a carboxylic acid to form the compound of formula (V): 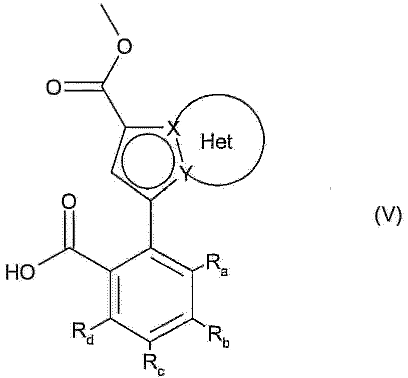 wherein Rₐ, R_{b}, R_{c}, R_{d}, X, Y and Het are as defined for formula (I),
which compound of formula (V) is then subjected to peptide coupling with a compound of formula (VI): 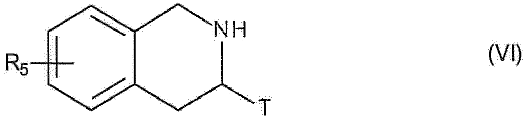 wherein T and R₅ are as defined for formula (I),
to yield the compound of formula (VII): 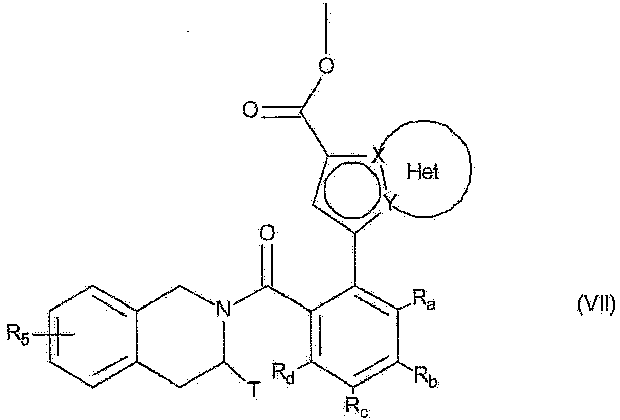 wherein Rₐ, R_{b}, R_{c}, R_{d}, T, R₅, X, Y and Het are as defined for formula (I),
the ester function of which compound of formula (VII) is hydrolysed to yield the corresponding carboxylic acid or carboxylate, which may be converted into an acid derivative such as the corresponding acyl chloride or anhydride before being coupled with an amine NHR₃R₄ wherein R₃ and R₄ have the same meanings as for formula (I), to yield the compound of formula (I),
which compound of formula (I) may be purified according to a conventional separation technique, which is converted, if desired, into its addition salts with a pharmaceutically acceptable acid or base and which is optionally separated into its isomers according to a conventional separation technique,
it being understood that, at any time considered appropriate in the course of the above-described process, certain groups (hydroxy, amino...) of the reagents or intermediates of synthesis may be protected and then deprotected according to the requirements of synthesis.

7. Process according to claim 6 for the preparation of a compound of formula (I) wherein one of the groups R₃ or R₄ is substituted by a hydroxy function, **characterised in that** the amine NHR₃R₄ is subjected beforehand to a reaction protecting the hydroxy function prior to any coupling with the carboxylic acid formed from the compound of formula (VII), or with a corresponding acid derivative thereof, the resulting protected compound of formula (I) subsequently undergoes a deprotection reaction and is then optionally converted into one of its addition salts with a pharmaceutically acceptable acid or base.

8. Pharmaceutical composition comprising a compound of formula (I) according to any one of claims 1 to 5 or an addition salt thereof with a pharmaceutically acceptable acid or base in combination with one or more pharmaceutically acceptable excipients.

9. Pharmaceutical composition according to claim 8 for use as a pro-apoptotic agent.

10. Pharmaceutical composition according to claim 8 for use in the treatment of cancers, auto-immune diseases and diseases of the immune system.

11. Pharmaceutical composition according to claim 8 for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

12. Compound of formula (I) according to one of claims 1 to 5, or an addition salt thereof with a pharmaceutically acceptable acid or base, for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

13. Association of a compound of formula (I) according to any one of claims 1 to 5 with an anti-cancer agent selected from genotoxic agents, mitotic poisons, antimetabolites, proteasome inhibitors, kinase inhibitors and antibodies.

14. Pharmaceutical composition comprising an association according to claim 13 in combination with one or more pharmaceutically acceptable excipients.

15. Association according to claim 13 for use in the treatment of cancers.

16. Compound of formula (I) according to any one of claims 1 to 5 for use in association with radiotherapy in the treatment of cancers.
